# EUROPEAN PATENT SPECIFICATION

(11) **EP 1 351 565 B1**
(45) Date of publication and mention of the grant of the patent: **16.03.2011**
(21) Application number: 01901992.6
(22) Date of filing: 15.01.2001
(51) Int. Cl.: A01H 1/06, A61K 31/7076, A61K 31/7088, C12N 1/00, C12N 5/00, C12Q 1/68

(54) **CHEMICAL INHIBITORS OF MISMATCH REPAIR**
CHEMISCHE HEMMSTOFFE DES MISMATCH-REPAIR
INHIBITEURS CHIMIQUES DE REPARATION DE DESAPPARIEMENTS

(43) Date of publication of application: 15.10.2003
(73) Proprietor: Morphotek, Inc., Exton, PA 19341 (US)
(72) Inventor: NICHOLAIDES, Nicholas, C., Glen Mills Pennsylvania 19342 (US); GRASSO, Luigi, Bryn Mawr Pennsylvania 19010 (US); SASS, Philip, M., Audubon, PA 19403 (US)
(74) Representative: W.P. Thompson & Co.
(86) International application number: PCT/US2001/000934
(87) International publication number: WO 2002/054856

(56) References cited:
- WO-A2-99/19492
- US-A- 5 907 079
- US-A- 6 146 894
- US-B1- 6 191 268
- MYERS S R ET AL: "BIOALKYLATION AND BIOOXIDATION OF ANTHRACENE IN-VITRO AND IN-VIVO" BIOCHEMICAL AND BIOPHYSICAL RESEARCH COMMUNICATIONS, vol. 151, no. 3, 1988, pages 1441-1445, XP008041016 ISSN: 0006-291X
- TRACZEWSKA T M: "Changes of toxicological properties of biodegradation products of anthracene and phenanthrene" WATER SCIENCE AND TECHNOLOGY, vol. 41, no. 12, 2000, pages 31-38, XP008041035 ISSN: 0273-1223
- MIRONOV N ET AL: "Induction of mutations in mismatch repair-proficient and -deficient human colon cancer cell lines by carcinogens producing different types of DNA damage" PROCEEDINGS OF THE AMERICAN ASSOCIATION FOR CANCER RESEARCH ANNUAL MEETING, vol. 40, March 1999 (1999-03), page 625, XP001182969 & 90TH ANNUAL MEETING OF THE AMERICAN ASSOCIATION FOR CANCER RESEARCH; PHILADELPHIA, PENNSYLVANIA, USA; APRIL 10-14, 1999 ISSN: 0197-016X
- YU ET AL.: 'Adriamycin induces large deletions in a major type of mutation in CHO cells' MUTATION RESEARCH vol. 325, no. 2-3, November 1994, pages 91 - 98, XP002939394
- CHAKRAVARTI ET AL.: 'Relating aromatic hydrocarbon-induced DNA adducts and c-H-ras mutations in mouse skin papillomas: the role of apurinic sites' PROC. NATL. ACAD. SCI. USA vol. 92, October 1995, pages 10422 - 10426, XP002939395
- DRUMMOND ET AL.: 'Cisplatin and adriamycin resistance are associated with MutLa and mismatch repair deficiency in an ovarian cell line' THE JOURNAL OF BIOLOGICAL CHEMISTRY vol. 271, no. 33, 16 August 1996, pages 19645 - 19648, XP002939396
- Y. QIAN ET AL.: 'Molecular events after antisense inhibition of hMSH2 in a HeLa cell line' MUTATION RESEARCH vol. 418, no. 2-3, 12 October 1998, pages 61 - 71, XP002939397
- BALOGH GABRIELA A. ET AL: 'Mutations in mismatch repair genes are involved in the neoplastic transformation of human breast epithelial cells.' INTERNATIONAL JOURNAL OF ONCOLOGY vol. 23, no. 2, August 2003, pages 411 - 419 ISSN: 1019-6439

## Description

### TECHNICAL FIELD OF THE INVENTION

The invention is related to the area of mutagenesis. In particular it is related to the field of blocking specific DNA repair processes.

### BACKGROUND OF THE INVENTION

Mismatch repair (MMR) is a conserved DNA repair process that is involved in post-replicative repair of mutated DNA sequences that occurs after genome replication. The process involves a group of gene products, including the *mutS* homologs *GTBP, hMSH2,* and *hMSH3* and the *mutL* homologs *hMLH1, hPMS1,* and *hPMS2* (Bronner, C.E. et al. (1994) Nature 368:258-261; Papadopoulos, N. et al. (1994) Science 263:1625-1629; Leach, F.S. et al. (1993) Cell 75:1215-1225 ; Nicolaides, N.C. et al. (1994) Nature 371:75-80) that work in concert to correct mispaired mono-, di-, and tri-nucleotides, point mutations, and to monitor for correct homologous recombination. Germline mutations in any of the genes involved in this process results in global point mutations, and instability of mono, di and tri-nucleotide repeats (a feature referred to as microsatellite instability (MI)), throughout the genome of the host cell. In man, genetic defects in MMR results in the predisposition to hereditary nonpolyposis colon cancer, a disease in which tumors retain a diploid genome but have widespread MI (Bronner, C.E. et al. (1994) Nature 368:258-261; Papadopoulos, N. et al. (1994) Science 263:1625-1629; Leach, F.S. et al. (1993) Cell 75:1215-1225; Nicolaides, N.C. et al. (1994) Nature 371:75-80; Harfe B.D., and S. Jinks-Robertson (2000) An. Rev. Genet. 34:359-399; Modrich, P. (1994) Science 266:1959-1960). Though the mutator defect that arises from MMR deficiency can affect any DNA sequence, microsatellite sequences are particularly sensitive to MMR. abnormalities (Peinado, M.A. et al.(1992) Proc. Natl. Acad. Sci. USA 89:10065-10069). Microsatellite instability is therefore a useful indicator of defective MMR. In addition to its occurrence in virtually all tumors arising in HNPCC patients, MI is found in a small fraction of sporadic tumors with distinctive molecular and phenotypic properties that is due to defective MMR (Perucho, M. (1996) Biol. Chem. 377:675-684).

MMR deficiency leads to a wide spectrum of mutations (point mutations, insertions, deletions, recombination, etc.) that can occur throughout the genome of a host cell. This effect has been found to occur across a diverse array of organisms ranging from but not limited to unicellular microbes, such as bacteria and yeast, to more complex organisms such as *Drosophila* and mammals, including mice and humans (Harfe B.D., and S. Jinks-Robertson (2000) An. Rev. Genet. 34:359-399; Modrich, P. (1994) Science 266:1959-1960). The ability to block MMR in a normal host cell or organism can result in the generation of genetically altered offspring or sibling cells that have desirable output traits for applications such as but not limited to agriculture, pharmaceutical, chemical manufacturing and specialty goods. A chemical method that can block the MMR process is beneficial for generating genetically altered hosts with commercially valuable output traits. A chemical strategy for blocking MMR *in vivo* offers a great advantage over a recombinant approach for producing genetically altered host organisms. One advantage is that a chemical approach bypasses the need for introducing foreign DNA into a host, resulting in a rapid approach for inactivating MMR and generating genetically diverse offspring or sib cells. Moreover, a chemical process is highly regulated in that once a host organism with a desired output trait is generated, the chemical is removed from the host and its MMR process would be restored, thus fixing the genetic alteration in subsequent generations. The invention described herein is directed to the discovery of small molecules that are capable of blocking MMR, thus resulting in host organisms with MI, a hallmark of MMR deficiency (Peinado, M.A. et al. (1992) Proc. Natl. Acad. Sci. USA 89:10065-10069; Perucho, M. (1996) Biol. Chem. 377:675-684; Wheeler, J.M. et al. (2000) J. Med. Genet. 37:588-592; Hoang, J.M. et al. (1997) Cancer Res. 57:300-303). Moreover, host organisms exhibiting MI are then selected for to identify subtypes with new output traits, such as but not limited to mutant nucleic acid molecules, polypeptides, biochemicals, physical appearance at the microscopic and/or macroscopic level, or phenotypic alterations in a whole organism. In addition, the ability to develop MMR defective host cells by a chemical agent provides a valuable method for creating genetically altered cell hosts for product development. The invention described herein is directed to the creation of genetically altered cell hosts via the blockade of MMR using chemical agents *in vivo.*

The advantages of the present invention are further described in the examples and figures described within this document.

### SUMMARY OF THE INVENTION

The invention provides methods for rendering cells hypermutable by blocking MMR activity with chemical agents.

The invention also provides genetically altered cell lines which have mutations introduced through interruption of mismatch repair.

The invention further provides methods to produce an enhanced rate of genetic hypermutation in a cell.

The invention encompasses methods of mutating a gene of interest in a cell, methods of creating cells with new phenotypes, and methods of creating cells with new phenotypes and a stable genome.

The invention also provides methods of creating genetically altered whole organisms and methods of creating whole organisms with new phenotypes.

These and other objects of the invention are provided by one or more of the embodiments described below.

A method for screening chemical compounds that block mismatch repair (MMR) is provided but not part of the invention. An MMR-sensitive reporter gene containing an out-of-frame polynucleotide repeat in its coding region is introduced into an MMR proficient cell. The cell is grown in the presence of chemicals. Chemicals that alter the genetic structure of the polynucleotide repeat yield a biologically active reporter gene product. Chemicals that disrupt the polynucleotide repeat are identified as MMR blocking agents.

Furthermore, an isolated MMR blocking chemical is described. The chemical can block MMR of a host cell, yielding a cell that exhibits an enhanced rate of hypermutation.

In another embodiment of the invention, a method is provided for introducing a mutation into a gene of interest. A chemical that blocks mismatch repair is added to the culture of a cell line. The cells become hypermutable as a result of the introduction of the chemical. The cell further comprises a gene of interest. The cell is cultured and tested to determine whether the gene of interest harbors a mutation.

In another embodiment of the invention, a method is provided for producing new phenotypes of a cell. A chemical that blocks mismatch repair is added to a cell culture. The cell becomes hypermutable as a result of the introduction of the chemical. The cell is cultured and tested for the expression of new phenotypes.

In another embodiment of the invention, a method is provided for restoring genetic stability in a cell in which mismatch repair is blocked via a chemical agent. The chemical is removed from the cell culture and the cell restores its genetic stability.

In another embodiment of the invention, a method is provided for restoring genetic stability in a cell with blocked mismatch repair and a newly selected phenotype. The chemical agent is removed from the cell culture and the cell restores its genetic stability and the new phenotype is stable.

In another embodiment of the invention, a chemical method for blocking MMR in plants is provided. The plant is grown in the presence of a chemical agent. The plant is grown and exhibits an enhanced rate of hypermutation

In another embodiment of the invention, a method for screening chemical inhibitors of MMR in plants *in vivo* is provided. MMR-sensitive plant expression vectors are engineered. The reporter vectors are introduced into plant hosts. The plant is grown in the presence of a chemical agent. The plant is monitored for altered reporter gene function.

In another embodiment of the invention, a method is provided for introducing a mutation into a gene of interest in a plant. A chemical that blocks mismatch repair is added to a plant. The plant becomes hypermutable as a result of the introduction of the chemical. The plant further comprises a gene of interest. The plant is grown. The plant is tested to determine whether the gene of interest harbors a mutation.

In another embodiment of the invention, a method is provided for producing new phenotypes of a plant. A chemical that blocks mismatch repair is added to a plant. The plant becomes hypermutable as a result of the introduction of the chemical. The plant is grown and tested for the expression of new phenotypes.

In another embodiment of the invention, a method in provided for restoring genetic stability in a plant in which mismatch repair is blocked via a chemical agent. The chemical is removed from the plant culture and the plant restores its genetic stability.

In another embodiment of the invention, a method is provided for restoring genetic stability in a plant with blocked mismatch repair and a newly selected phenotype. The chemical agent is removed from the plant culture and the plant restores its genetic stability and the new phenotype is stable.

These and other embodiments of the invention provide the art with methods that can generate enhanced mutability in microbes, organisms of the protista class, insect cells, mammalian cells, plants, and animals as well as providing cells, plants and animals harboring potentially useful mutations.

### BRIEF DESCRIPTION OF THE DRAWINGS

**Figure 1** shows diagrams of mismatch repair (MMR) sensitive reporter genes.
**Figure 2** shows a screening method for identifying MMR blocking chemicals.
**Figure 3** shows identification of a small chemical that blocks MMR and genetically alters the pCAR-OF vector in *vivo.*
**Figure 4** shows shifting of endogenous microsatellites in human cells induced by a chemical inhibitor of MMR.
**Figure 5** shows sequence analysis of microsatellites from cells treated with chemical inhibitors of MMR with altered repeats.
**Figure 6** shows generation of host organisms with new phenotypes using a chemical blocker of MMR.
**Figure 7** shows a schematic diagram of MMR-sensitive reporter gene for plants.
**Figure 8** shows derivatives of lead compounds and thereof that are inhibitors of MMR *in vivo.*

### DETAILED DESCRIPTION OF THE INVENTION

Various definitions are provided herein. Most words and terms have the meaning that would be attributed to those words by one skilled in the art. Words or terms specifically defined herein have the meaning provided in the context of the present invention as a whole and as are typically understood by those skilled in the art. Any conflict between an art-understood definition of a word or term and a definition of the word or term as specifically taught herein shall be resolved in favor of the latter. Headings used herein are for convenience and are not to be construed as limiting.

As used herein the term "anthracene" refers to the compound anthracene. However, when referred to in the general sense, such as "anthracenes," "an anthracene" or "the anthracene," such terms denote any compound that contains the fused triphenyl core structure of anthracene, i.e., 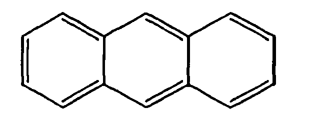 regardless of extent of substitution.

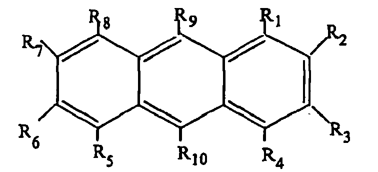

In certain preferred embodiments of the invention, the anthracene has the formula: wherein R₁-R₁₀ are independently hydrogen, hydroxyl, amino, alkyl, substituted alkyl, alkenyl, substituted alkenyl, alkynyl, substituted alkynyl, O-alkyl, S-alkyl, N-alkyl, O-alkenyl, S-alkenyl, N-alkenyl, O-alkynyl, S-alkynyl, N-alkynyl, aryl, substituted aryl, aryloxy, substituted aryloxy, heteroaryl, substituted heteroaryl, aralkyloxy, arylakyl, alkylaryl, alkylaryloxy, arylsulfonyl, alkylsulfonyl, alkoxycarbonyl, aryloxycarbonyl, guanidino, carboxy, an alcohol, an amino acid, sulfonate, alkyl sulfonate, CN, NO₂, an aldehyde group, an ester, an ether, a crown ether, a ketone, an organosulfur compound, an organometallic group, a carboxylic acid, an organosilicon or a carbohydrate that optionally contains one or more alkylated hydroxyl groups;
wherein said heteroalkyl, heteroaryl, and substituted heteroaryl contain at least one heteroatom that is oxygen, sulfur; a metal atom, phosphorus, silicon or nitrogen; and
wherein said substituents of said substituted alkyl, substituted alkenyl, substituted alkynyl, substituted aryl, and substituted heteroaryl are halogen, CN, NO₂, lower alkyl, aryl, heteroaryl, aralkyl, aralkyloxy, guanidino, alkoxycarbonyl, alkoxy, hydroxy, carboxy and amino;
and wherein said amino groups optionally substituted with an acyl group, or 1 to 3 aryl or lower alkyl groups;
or wherein any two of R₁-R₁₀ can together form a polyether;
or wherein any two of R₁-R₁₀ can, together with the intervening carbon atoms of the anthracene core, form a crown ether.

As used herein, "alkyl" refers to a hydrocarbon containing from 1 to about 20 carbon atoms. Alkyl groups may straight, branched, cyclic, or combinations thereof. Alkyl groups thus include, by way of illustration only, methyl, ethyl, propyl, isopropyl, butyl, isobutyl, cyclopentyl, cyclopentylmethyl, cyclohexyl, cyclohexylmethyl, and the like. Also included within the definition of "alkyl" are fused and/or polycyclic aliphatic cyclic ring systems such as, for example, adamantane. As used herein the term "alkenyl" denotes an alkyl group having at least one carbon-carbon double bond. As used herein the term "alkynyl" denotes an alkyl group having at least one carbon-carbon triple bond.

In some preferred embodiments, the alkyl, alkenyl, alkynyl, aryl, aryloxy, and heteroaryl substituent groups described above may bear one or more further substituent groups; that is, they may be "substituted". In some preferred embodiments these substituent groups can include halogens (for example fluorine, chlorine, bromine and iodine), CN, NO₂ lower alkyl groups, aryl groups, heteroaryl groups, aralkyl groups, aralkyloxy groups, guanidino, alkoxycarbonyl, alkoxy, hydroxy, carboxy and amino groups. In addition, the alkyl and aryl portions of aralkyloxy, arylalkyl, arylsulfonyl, alkylsulfonyl, alkoxycarbonyl, and aryloxycarbonyl groups also can bear such substituent groups. Thus, by way of example only, substituted alkyl groups include, for example, alkyl groups fluoro-, chloro-, bromo- and iodoalkyl groups, aminoalkyl groups, and hydroxyalkyl groups, such as hydroxymethyl, hydroxyethyl, hydroxypropyl, hydroxybutyl, and the like. In some preferred embodiments such hydroxyalkyl groups contain from 1 to about 20 carbons.

As used herein the term "aryl" means a group having 5 to about 20 carbon atoms and which contains at least one aromatic ring, such as phenyl, biphenyl and naphthyl. Preferred aryl groups include unsubstituted or substituted phenyl and naphthyl groups. The term "aryloxy" denotes an aryl group that is bound through an oxygen atom, for example a phenoxy group.

In general, the prefix "hetero" denotes the presence of at least one hetero (i.e., non-carbon) atom, which is in some preferred embodiments independently one to three O, N, S, P, Si or metal atoms. Thus, the term "heteroaryl" denotes an aryl group in which one or more ring carbon atom is replaced by such a heteroatom. Preferred heteroaryl groups include pyridyl, pyrimidyl, pyrrolyl, furyl, thienyl, and imidazolyl groups.

The term "aralkyl" (or "arylalkyl") is intended to denote a group having from 6 to 15 carbons, consisting of an alkyl group that bears an aryl group. Examples of aralkyl groups

In general, the prefix "hetero" denotes the presence of at least one hetero (i.e., non-carbon) atom, which is in some preferred embodiments independently one to three O, N, S, P, Si or metal atoms. Thus, the term "heteroaryl" denotes an aryl group in which one or more ring carbon atom is replaced by such a heteroatom. Preferred heteroaryl groups include pyridyl, pyrimidyl, pyrrolyl, furyl, thienyl, and imidazolyl groups.

The term "aralkyl" (or "arylalkyl") is intended to denote a group having from 6 to 15 carbons, consisting of an alkyl group that bears an aryl group. Examples of aralkyl groups include benzyl, phenethyl, benzhydryl and naphthylmethyl groups.

The term "alkylaryl" (or "alkaryl") is intended to denote a group having from 6 to 15 carbons, consisting of an aryl group that bears an alkyl group. Examples of aralkyl groups include methylphenyl, ethylphenyl and methylnaphthyl groups.

The term "arylsulfonyl" denotes an aryl group attached through a sulfonyl group, for example phenylsulfonyl. The term "alkylsulfonyl" denotes an alkyl group attached through a sulfonyl group, for example methylsulfonyl.

The term "alkoxycarbonyl" denotes a group of formula -C(=O)-O-R where R is alkyl, alkenyl, or alkynyl, where the alkyl, alkenyl, or alkynyl portions thereof can be optionally substituted as described herein.

The term "aryloxycarbonyl" denotes a group of formula -C(=O)-O-R where R is aryl, were the aryl portion thereof can be optionally substituted as described herein.

The terms "arylalkyloxy" or "aralkyloxy" are equivalent, and denote a group of formula -O-R'-R", where R' is R is alkyl, alkenyl, or alkynyl which can be optionally substituted as described herein, and wherein R" denotes a aryl or substituted aryl group.

The terms "alkylaryloxy" or "alkaryloxy" are equivalent, and denote a group of formula -O-R'-R", where R' is an aryl or substituted aryl group, and R" is alkyl, alkenyl, or alkynyl which can be optionally substituted as described herein.

As used herein, the term "aldehyde group" denotes a group that bears a moiety of formula -C(=O)-H. The term "ketone" denotes a moiety containing a group of formula -R-C(=O)-R=, where R and R= are independently alkyl, alkenyl, alkynyl, aryl, heteroaryl, aralkyl, or alkaryl, each of which may be substituted as described herein.

As used herein, the term "ester" denotes a moiety having a group of formula -R-C(=O)-O-R= or -R-O-C(=O)-R= where R and R= are independently alkyl, alkenyl, alkynyl, aryl, heteroaryl, aralkyl, or alkaryl, each of which may be substituted as described herein.

The term "ether" denotes a moiety having a group of formula -R-O-R= or where R and R= are independently alkyl, alkenyl, alkynyl, aryl, heteroaryl, aralkyl, or alkaryl, each of which may be substituted as described herein.

The term "crown ether" has its usual meaning of a cyclic ether containing several oxygen atoms. As used herein the term "organosulfur compound" denotes aliphatic or aromatic sulfur containing compounds, for example thiols and disulfides. The term "organometallic group" denotes an organic molecule containing at least one metal atom.

The term "organosilicon compound" denotes aliphatic or aromatic silicon containing compounds, for example alkyl and aryl silanes.

The term "carboxylic acid" denotes a moiety having a carboxyl group, other than an amino acid.

As used herein, the term "amino acid" denotes a molecule containing both an amino group and a carboxyl group. In some preferred embodiments, the amino acids are α-, β-, γ-or δ-amino acids, including their stereoisomers and racemates. As used herein the term "L-amino acid" denotes an α-amino acid having the L configuration around the α-carbon, that is, a carboxylic acid of general formula CH(COOH)(NH₂)-(side chain), having the L-configuration. The term "D-amino acid" similarly denotes a carboxylic acid of general formula CH(COOH)CNH₂)-(side chain), having the D-configuration around the α-carbon. Side chains of L-amino acids include naturally occurring and non-naturally occurring moieties. Non-naturally occurring (i.e., unnatural) amino acid side chains are moieties that are used in place of naturally occurring amino acid side chains in, for example, amino acid analogs. *See,* for example, Lehninger, Biochemistry, Second Edition, Worth Publishers, Inc, 1975, pages 72-77, incorporated herein by reference. Amino acid substituents may be attached through their carbonyl groups through the oxygen or carbonyl carbon thereof, or through their amino groups, or through functionalities residing on their side chain portions.

As used herein "polynucleotide" refers to a nucleic acid molecule and includes genomic DNA cDNA, RNA, mRNA and the like.

As used herein "antisense oligonucleotide" refers to a nucleic acid molecule that is complementary to at least a portion of a target nucleotide sequence of interest and specifically hybridizes to the target nucleotide sequence under physiological conditions.

As used herein "inhibitor of mismatch repair" refers to an agent that interferes with at least one function of the mismatch repair system of a cell and thereby renders the cell more susceptible to mutation.

As used herein "hypermutable" refers to a state in which a cell *in vitro* or *in vivo* is made more susceptible to mutation through a loss or impairment of the mismatch repair system.

As used herein "agents," "chemicals," and "inhibitors" when used in connection with inhibition of MMR refers to chemicals, oligonucleotides, analogs of natural substrates, and the like that interfere with normal function of MMR.

Methods for developing hypermutable cells and whole organisms have been discovered by taking advantage of the conserved mismatch repair (MMR) process of a host. Dominant negative alleles of MMR genes, when introduced into cells or transgenic animals, increase the rate of spontaneous mutations by reducing the effectiveness of DNA repair and thereby render the cells or animals hypermutable. Hypermutable microbes, protozoans, insects, mammalian cells, plants or whole animals can then be utilized to develop new mutations in a gene of interest. It has been discovered that chemicals that block MMR, and thereby render cells hypermutable, is an efficient way to introduce mutations in cells and genes of interest. In addition to destabilizing the genome of cells exposed to chemicals that inhibit MMR activity may be done transiently, allowing cells to become hypermutable, and removing the chemical exposure after the desired effect (e.g., a mutation in a gene of interest) is achieved. The chemicals that inhibit MMR activity that are suitable for use in the invention include, but are not limited to, anthracene derivatives, nonhydrolyzable ATP analogs, ATPase inhibitors, antisense oligonucleotides that specifically anneal to polynucleotides encoding mismatch repair proteins, DNA polymerase inhibitors, and exonuclease inhibitors. These chemicals can enhance the rate of mutation due to inactivation of MMR yielding clones or subtypes with altered biochemical properties. Methods for identifying chemical compounds that inhibit MMR *in vivo* are also described herein.

The process of MMR, also called mismatch proofreading, is carried out by a group of protein complexes in cells ranging from bacteria to man (Harfe B.D., and S. Jinks-Robertson (2000) An. Rev. Genet. 34:359-399; Modrich, P. (1994) Science 266:1959-1960). An MMR gene is a gene that encodes for one of the proteins of such a mismatch repair complex. Although not wanting to be bound by any particular theory of mechanism of action, an MMR complex is believed to detect distortions of the DNA helix resulting from non-complementary pairing of nucleotide bases. The non-complementary base on the newer DNA strand is excised, and the excised base is replaced with the appropriate base, which is complementary to the older DNA strand. In this way, cells eliminate many mutations that occur as a result of mistakes in DNA replication.

Dominant negative alleles cause an MMR defective phenotype even in the presence of a wild-type allele in the same cell. An example of a dominant negative allele of an MMR gene is the human gene *hPMS2-134* (SEQ ID NO:25), which carries a truncating mutation at codon 134 (Nicolaides, N.C. et al. (1998) Mol. Cell. Biol. 18:1635-1641). The mutation causes the product of this gene to abnormally terminate at the position of the 134th amino acid, resulting in a shortened polypeptide containing the N-terminal 133 amino acids (SEQ ID NO:24). Such a mutation causes an increase in the rate of mutations, which accumulate in cells after DNA replication. Expression of a dominant negative allele of a mismatch repair gene results in impairment of mismatch repair activity, even in the presence of the wild-type allele.

The MMR process has been shown to be blocked by the use of nonhydrolyzable forms of ATP (Galio, L. et al. (1999) Nucl. Acids Res. 27:2325-2331; Allen, D.J. et al. (1997) EMBO J. 16:4467-4476; Bjornson, K.P. et al. (2000) Biochem. 39:3176-3183). It has been demonstrated that MMR activity might have been blocked by using benzo [a]pyrene or 7,12-dimethylbenz[a]anthracene in immortalized human breast epithelial cells (Huang, Y. et al. (1999): Mol. Carcinogen. 24:118-127). Such chemicals can be identified by screening cells for defective MMR activity. Cells from bacteria, yeast, fungi, insects, plants, animals, and humans can be screened for defective mismatch repair. Genomic DNA, cDNA, or mRNA from any cell can be analyzed for variations from the wild type sequences in cells or organisms grown in the presence of MMR blocking compounds. Various techniques of screening can be used. The suitability of such screening assays, whether natural or artificial, for use in identifying hypermutable cells, insects, fungi, plants or animals can be evaluated by testing the mismatch repair activity caused by a compound or a mixture of compounds, to determine if it is an MMR inhibitor.

A cell, a microbe, or a whole organism such as an insect, fungus, plant or animal in which a chemical inhibitor of mismatch repair has been treated will become hypermutable. This means that the spontaneous mutation rate of such cells or whole organism is elevated compared to cells or animals without such treatment. The degree of elevation of the spontaneous mutation rate can be at least 2-fold, 5-fold, 10-fold, 20-fold, 50-fold, 100-fold, 200-fold, 500-fold, or 1000-fold that of the normal cell or animal. The use of chemical mutagens such as, but limited to, N-methyl-N'-nitro-N-nitrosoguanidine (MNNG), methane sulfonate, dimethyl sulfonate, 06-methyl benzadine, ethyl methanesulfonate (EMS), methylnitrosourea (MNU), ethylnitrosourea (ENU), *etc.* can be used in MMR defective cells or whole organisms to increase the rates an additional 10 to 100 fold that of the MMR deficiency itself.

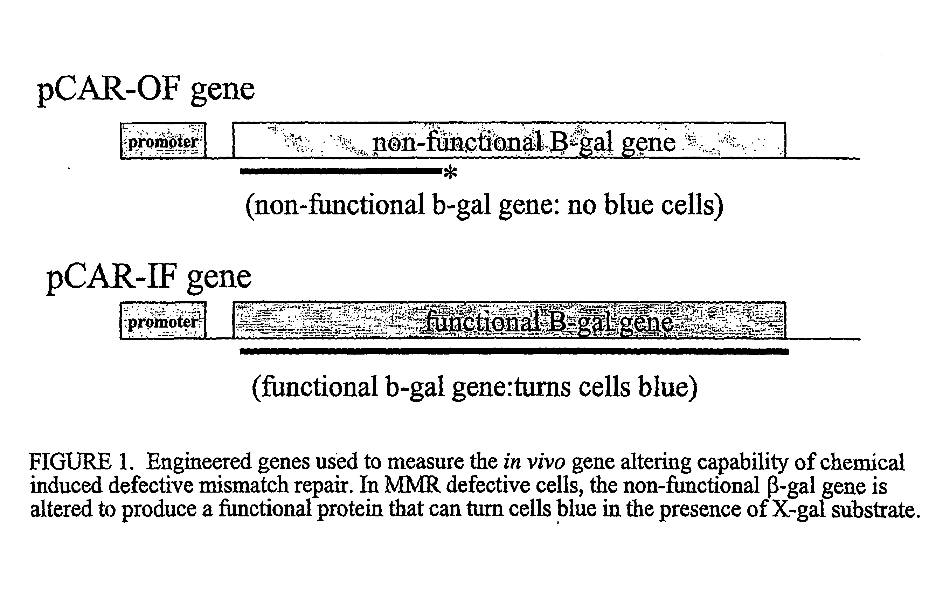

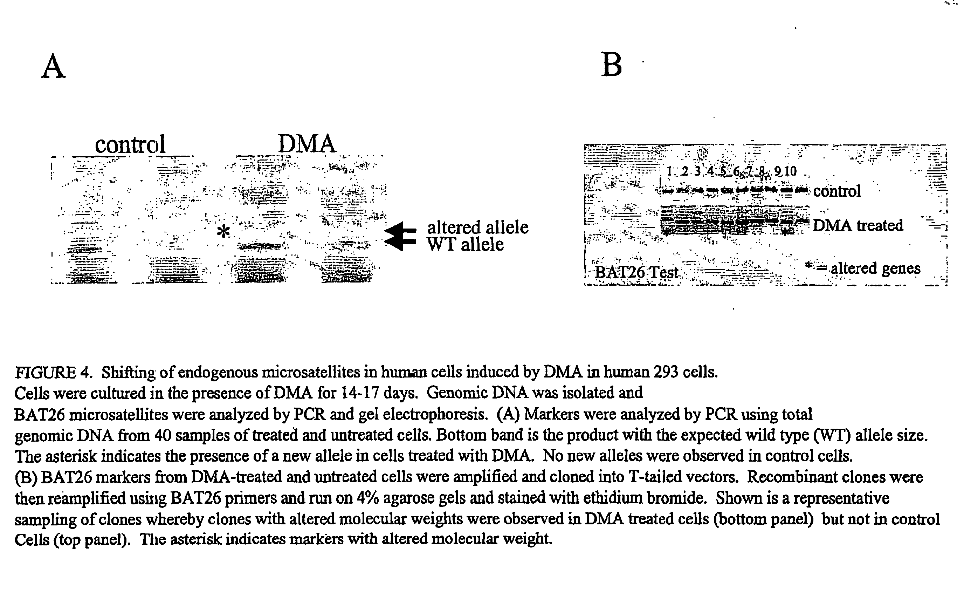

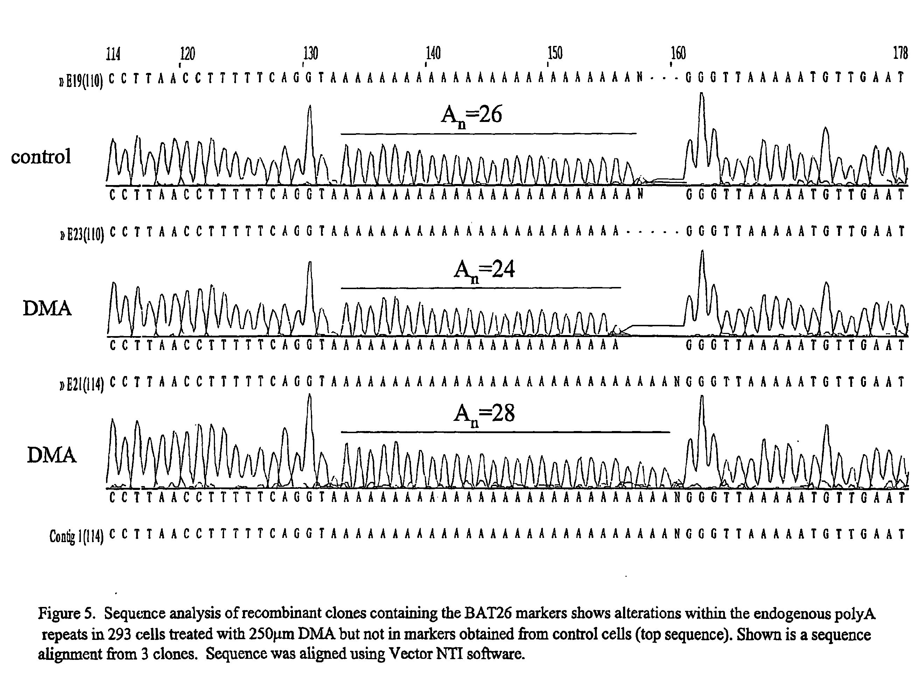

A screening assay for identifying chemical inhibitors of MMR is developed and employed. A chemical compound can be in any form or class ranging from but not limited to amino acid, steroidal, aromatic, or lipid precursors. The chemical compound can be naturally occurring or made in the laboratory. The screening assay can be natural such as looking for altered endogenous repeats within an host organism's genome (as demonstrated in Figs. 4 and 5), or made in the laboratory using an MMR-sensitive reporter gene as demonstrated in Figs. 1-3).

The chemical compound can be introduced into the cell by supplementing the growth medium, or by intracellular delivery such as but not limited to using microinjection or carrier compounds.

According to another aspect of the invention, a chemical compound from the anthracene class can be exposed to MMR proficient cells or whole organism hosts, the host is grown and screened for subtypes containing genetically altered genes with new biochemical features.

The anthracene compounds that are suitable for use in the invention include, but are not limited to anthracenes having the formula: 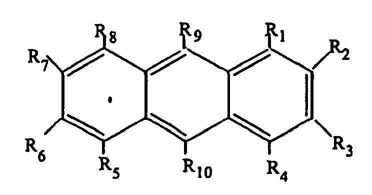 wherein R₁-R₁₀ are independently hydrogen, hydroxyl, amino, alkyl, substituted alkyl, alkenyl, substituted alkenyl, alkynyl, substituted alkynyl, O-alkyl, S-alkyl, N-alkyl, O-alkenyl, S-alkenyl, N-alkenyl,O-alkynyl, S-alkynyl, N-alkynyl, aryl, substituted aryl, aryloxy, substituted aryloxy, heteroaryl, substituted heteroaryl, aralkyloxy, arylalkyl, alkylaryl, alkylaryloxy, arylsulfonyl, alkylsulfonyl, alkoxycarbonyl, aryloxycarbonyl, guanidino, carboxy, an alcohol, an amino acid, sulfonate, alkyl sulfonate, CN, NO₂, an aldehyde group, an ester, an ether, a crown ether, a ketone, an organosulfur compound, an organometallic group, a carboxylic acid, an organosilicon or a carbohydrate that optionally contains one or more alkylated hydroxyl groups;
wherein said heteroalkyl, heteroaryl, and substituted heteroaryl contain at least one heteroatom that is oxygen, sulfur, a metal atom, phosphorus, silicon or nitrogen; and
wherein said substituents of said substituted alkyl, substituted alkenyl, substituted alkynyl, substituted aryl, and substituted heteroaryl are halogen, CN, NO₂, lower alkyl, aryl, heteroaryl, aralkyl, aralkyloxy, guanidino, alkoxycarbonyl, alkoxy, hydroxy, carboxy and amino;
and wherein said amino groups optionally substituted with an acyl group, or 1 to 3 aryl or lower alkyl groups;
or wherein any two of R₁-R₁₀ can together form a polyether;
or wherein any two of R₁-R₁₀ can, together with the intervening carbon atoms of the anthracene core, form a crown ether.

The method of the invention also encompasses inhibiting MMR with an anthracene of the above formula wherein R₅ and R₆ are hydrogen, and the remaining substituents are as described above.

The some embodiments, in the anthracene compound R₁-R₁₀ are independently hydrogen, hydroxyl, alkyl, aryl, arylaklyl, or hydroxyalkyl. In other embodiments, R₁-R₁₀ are independently hydrogen, hydroxyl, methyl, ethyl, propyl, isopropyl, butyl, isobutyl, phenyl, tolyl, hydroxymethyl, hydroxypropyl, or hydroxybutyl.

In specific embodiments of the invention the anthracenes include, but are not limited to 1,2-dimethylanthracene, 9,10-dimethyl anthracene, 7,8-dimethylanthracene, 9,10-diphenylanthracene, 9,10-dihydroxymethylanthracene, 9-hydroxymethyl-10-methylanthracene, dimethylanthracene-1,2-diol, 9-hydroxymethyl-10-methylanthracene-1,2-diol, 9-hydroxymethyl-10-methylanthracene-3,4-diol, 9, 10-di-m-tolyanthracene, and the like.

The chiral position of the side chains of the anthracenes is not particularly limited and may be any chiral position and any chiral analog. The anthracenes may also comprise a stereoisomeric forms of the anthracenes and includes any isomeric analog.

Examples of hosts are but not limited to cells or whole organisms from human, primate, mammal, rodent, plant, fish, reptiles, amphibians, insects, fungi, yeast or microbes of prokaryotic origin.

Also described is the use of ATP analogs capable of blocking ATPase activity required for MMR. MMR reporter cells are screened with ATP compound libraries to identify those compounds capable of blocking MMR *in vivo.* Examples of ATP analogs that are useful in blocking MMR activity include, but are not limited to, nonhydrolyzable forms of ATP such as AMP-PNP and ATP[gamma]S block the MMR activity (Galio, L. et al. (1999) Nucl. Acids Res. 27:2325-2331; Allen, D.J. et al. (1997) EMBO J. 16:4467-4476; Bjornson K.P. et al. (2000) Biochem. 39:3176-3183).

Also described is the use of nuclease inhibitors that are able to block the exonuclease activity of the MMR biochemical pathway. MMR reporter cells are screened with nuclease inhibitor compound libraries to identify compounds capable of blocking MMR *in vivo.* Examples of nuclease inhibitors that are useful in blocking MMR activity include, but are not limited to analogs of N-Ethylmaleimide, an endonuclease inhibitor (Huang, Y.C., et.al. (1995) Arch. Biochem. Biophys. 316:485), heterodimeric adenine-chain-acridine compounds, exonulcease III inhibitors (Belmont P, et.al., Bioorg Med Chem Lett (2000) 10:293-295), as well as antibiotic compounds such as Heliquinomycin, which have helicase inhibitory activity (Chino, M, et.al. J. Antibiot. (Tokyo) (1998) 51:480-486).

Also described is the use of DNA polymerase inhibitors that are able to block the polymerization required for mismatch-mediated repair. MMR reporter cells are screened with DNA polymerase inhibitor compound libraries to identify those compounds capable of blocking MMR *in vivo*. Examples of DNA polymerase inhibitors that are useful in blocking MMR activity include, but are not limited to, analogs of actinomycin D (Martin, S.J., et.al. (1990) J. Immunol. 145:1859), Aphidicolin (Kuwakado, K. et.al. (1993) Biochem. Pharmacol. 46:1909) 1-(2'-Deoxy-2'-fluoro-beta-L-arabinofuranosyl)-5-methyluracil (L-FMAU) (Kukhanova M, et.al., Biochem Pharmacol (1998) 55:1181-1187), and 2',3'-dideoxyribonucleoside 5'-triphosphates (ddNTPs) (Ono, K., et.al., Biomed Pharmacother (1984) 38:382-389).

Also described the use of antisense oligonucleotides that are administered to cells to disrupt at least one function of the mismatch repair process. The antisense polynucleotides hybridize to MMR polynucleotides. Both full-length and antisense polynucleotide frgaments are suitable for use. "Antisense polynucleotide fragments" of the invention include, but are not limited to polynuclotides that specifically hybridize to an MMR encoding RNA (as determined by sequence comparison of nucleotides encoding the MMR to nucleotides encoding other known molecules). Identification of sequences that are substantially unique to MMR-encoding polynucleotides can be ascertained by analysis of any publicly available sequence database and/or with any commercially available sequence comparison programs. Antisense molecules may be generated by any means including, but not limited to chemical synthesis, expression in an *in vitro* transcription reaction, through expression in a transformed cell comprising a vector that may be transcribed to produce antisense molecules, through restriction digestion and isolation, through the polymerase chain reaction, and the like.

Antisense oligonucleotides, or fragments thereof may include the nucleotide sequences set forth in SEQ ID NOs:15, 17, 19, 21, 23, 25, 27, and 29 or sequences complementary or homologous thereto, for example. Those of skill in the art recognize that the invention may be predicted using any MMR gene. Specifically, antisense nucleic acid molecules comprise a sequence complementary to at least about 10, 15, 25, 50, 100, 250 or 500 nucleotides or an entire MMR encoding sequence. Preferably, the antisense oligonucleotides comprise a sequence complementary to about 15 consecutive nucleotides of the coding strand of the MMR encoding sequence.

An antisense nucleic acid molecule is antisense to a "coding region" of the coding strand of a nucleotide sequence encoding an MMR protein. The coding strand may also include regulatory regions of the MMR sequence. The term "coding region" refers to the region of the nucleotide sequence comprising codons which are translated into amino acid residues (e.g., the protein coding region of human PMS2 corresponds to the coding region SEQ ID NO:17). In another embodiment, the antisense nucleic acid molecule is antisense to a "noncoding region" of the coding strand of a nucleotide sequence encoding an MMR protein. The term "noncoding region" refers to 5' and 3' sequences which flank the coding region that are not translated into amino acids *(i.e.,* also referred to as 5' and 3' untranslated regions (UTR)).

Preferably, antisense oligonucleotides are directed to regulatory regions of a nucleotide sequence encoding an MMR protein, or mRNA corresponding thereto, including, but not limited to, the initiation codon, TATA box, enhancer sequences, and the like. Given the coding strand sequences provided herein, antisense nucleic acids of the invention can be designed according to the rules of Watson and Crick or Hoogsteen base pairing. The antisense nucleic acid molecule can be complementary to the entire coding region of an MMR mRNA, but more preferably is an oligonucleotide that is antisense to only a portion of the coding or noncoding region of an MMR mRNA. For example, the antisense oligonucleotide can be complementary to the region surrounding the translation start site of an MMR mRNA. An antisense oligonucleotide can be, for example, about 5, 10, 15, 20, 25, 30, 35, 40, 45 or 50 nucleotides in length.

Screening is any process whereby a chemical compound is exposed to a cell or whole organism. The process of screening can be carried out using but not limited to a whole animal, plant, insect, microbe, or by using a suspension of one or more isolated cells in culture. The cell can be any type of eukaryotic or prokaryotic cell, including, for example, cells isolated from humans or other primates, mammals or other vertebrates, invertebrates, and single celled organisms such as protozoa, yeast, or bacteria.

In general, screening will be carried out using a suspension of cells, or a single cell, but other methods can also be applied as long as a sufficient fraction of the treated cells or tissue is exposed so that isolated cells can be grown and utilized. Techniques for chemical screening are well known to those in the art. Available techniques for screening include cell-based assays, molecular assays, and whole organism-based assays. Compounds can be added to the screening assays of the invention in order to identify those agents that are capable of blocking MMR in cells.

The screening assays provide a system wherein a cell, cells or a whole organism is contacted with a candidate compound and then tested to determine whether mismatch repair has been adversely affected. The method in which MMR is analyzed may be any known method, including, but not limited to analysis of the molecular sequence of the MMR gene, and analyzing endogenous repeats in the subject's genome. Further, a convenient assay to analyze the effects of candidate agents on reporter genes transfected into cells is provided.

MMR-inhibitors identified by the methods can be used to generate new mutations in one or more gene(s) of interest. A gene of interest can be any gene naturally possessed by a cell line, microbe or whole organism. An advantage of using chemicals rather than recombinant technologies to block MMR are that the process is faster; there is no need to produce stable clones with a knocked out MMR gene or a clone expressing a dominant negative MMR gene allele. Another advantage is that host organisms need not be screened for integrated knock out targeting vectors or stable expression of a dominant negative MMR gene allele. Finally, once a cell, plant or animal has been exposed to the MMR-blocking compound and a new output trait is generated, the MMR process can be restored by removal of compound. Mutations can be detected by analyzing the genotype of the cell, or whole organism, for example, by examining the sequence of genomic DNA, cDNA, messenger RNA, or amino acids associated with the gene of interest. Mutations can also be detected by screening for new output traits such as hypoxanthine-guanine phosphoribosyltransferase (HPRT) revertants. A mutant polypeptide can be detected by identifying alterations in electrophoretic mobility, spectroscopic properties, or other physical or structural characteristics of a protein encoded by a mutant gene. One can also screen for altered function of the protein *in situ,* in isolated form, or in model systems. One can screen for alteration of any property of the cell, plant or animal associated with the function of the gene of interest.

Several advantages exist in generating genetic mutations by blocking MMR *in* vivo in contrast to general DNA damaging agents such as MNNG, MNU and EMS. Cells with MMR deficiency have a wide range of mutations dispersed throughout their entire genome in contrast to DNA damaging agents such as MNNG, MNU, EMS and ionizing radiation. Another advantage is that mutant cells that arise from MMR deficiency are diploid in nature and do not lose large segments of chromosomes as is the case of DNA damaging agents such as EMS, MNU, and ionizing radiation (Honma, M. et al. (1997) Mutat. Res. 374:89-98). This unique feature allows for subtle changes throughout a host's genome that leads to subtle genetic changes yielding genetically stable hosts with commercially important output traits.

The invention also encompasses blocking MMR in vivo and in vitro and further exposing the cells or organisms to a chemical mutagen in order to increase the incidence of genetic mutation.

The invention also encompasses withdrawing exposure to inhibitors of mismatch repair once a desired mutant genotype or phenotype is generated such that the mutations are thereafter maintained in a stable genome.

The above disclosure generally describes the present invention. A more complete understanding can be obtained by reference to the following specific examples, which are provided herein for purposes of illustration only, and are not intended to limit the scope of the invention.

### EXAMPLES

### EXAMPLE 1: Generation of a cell-based screening assay to identify chemicals capable of inactivating mismatch repair in vivo.

A hallmark of MMR deficiency is the generation of unstable microsatellite repeats in the genome of host cells (Peinado, M.A. et al. (1992) Proc. Natl. Acad Sci. USA 89:10065-10069; Strand, M. et al. (1993) Nature 365:274-276; Parsons, R. et al. (1993) Cell 75:1227-1236). This phenotype is preferred to as microsatellite instability (MI) (Harfe, B.D. and S. Jinks-Robertson (2000) Ann. Rev. Genet. 34:359-399; Modrich, P. (1994) Science 266:1959-1960; Peinado, M.A. et al. (1992) Proc. Natl. Acad. Sci. USA 89:10065-10069; Perucho, M. (1996) Biol. Chem. 377:675-684; Hoang, J.M. et al. (1997) Cancer Res. 57:300-303; Strand, M. et al. (1993) Nature 365:274-276). MI consists of deletions and/or insertions within repetitive mono-, di- and/or tri nucleotide repetitive sequences throughout the entire genome of a host cell. Extensive genetic analysis of eukaryotic cells have found that the only biochemical defect that is capable of producing MI is defective MMR (Harfe, B.D. and S. Jinks-Robertson (2000) Ann. Rev. Genet. 34:359-399; Modrich, P. (1994) Science 266:1959-1960; Peinado, M.A. et al. (1992) Proc. Natl. Acad. Sci. USA 89:10065-10069; Perucho, M. (1996) Biol. Chem. 377:675-684; Hoang, J.M. et al. (1997) Cancer Res. 57:300-303; Strand, M. et al.(1993) Nature 365:274-276). In light of this unique feature that defective MMR has on promoting microsatellite instability, endogenous MI is now used as a biochemical marker to survey for lack of MMR activity within host cells (Hoang, J.M. et al. (1997) Cancer Res. 57:300-303).

A method used to detect MMR deficiency in eukaryotic cells is to employ a reporter gene that has a polynucleotide repeat inserted within the coding region that disrupts its reading frame due to a frame shift. In the case where MMR is defective, the reporter gene will acquire random mutations (*i.e.*, insertions and/or deletions) within the polynucelotide repeat yielding clones that contain a reporter with an open reading frame. This reporter gene can be of any biochemical pathway such as but not limited to β-glucoronidase, β-galactosidase, neomycin resistant gene, hygromycin resistance gene, green fluorescent protein, and the like. A schematic diagram of MMR-sensitive reporters are shown in Fig. 1, where the polynucleotide repeat can consist of mono-, di-, tri- or tetranucleotides. We have employed the use of a β-galactosidase MMR-sensitive reporter gene to measure for MMR activity in H36 cells, which are a murine hybridoma cell line. The reporter construct used is called pCAR-OF, which contains a hygromycin resistance (HYG) gene plus a β-galactosidase gene with a 29 bp out-of-frame poly-CA tract inserted at the 5' end of its coding region. The pCAR-OF reporter cannot generate β-galactosidase activity unless a frame-restoring mutation (*i.e.*, insertion or deletion) arises following transfection. This line has been shown to be sensitive to inactivated MMR where using a dominant negative MMR gene allele has found this condition to result in the production of β-galactosidase (unpublished data). An example of these data using the dominant negative PMS134 allele is shown in Table 1. Briefly, H36 cells were each transfected with an expression vector containing the PMS134 allele (referred to as HB134) or empty vector and the pCAR-OF vector in duplicate reactions using the protocol below. The PMS134 gene is cloned into the pEF expression vector, which contains the elongation factor promoter upstream of the cloning site followed by a mammalian polyadenylation signal. This vector also contains the NEOr gene that allows for selection of cells in G418 to identify those retaining this plasmid. Briefly, cells were transfected with 1 µg of the PMS134 or empty vector using polyliposomes following the manufacturer's protocol (Life Technologies). Cells were then selected in 0.5 mg/ml of G418 for 10 days and G418 resistant cells were pooled together to analyze for gene expression. PMS134 positive cells, which were determined by RT-PCR and western blot (not shown) were expanded and transfected with the pCAR-OF reporter gene that contains a hygromycin (HYG) resistance gene as reporter using the protocol described above. Cells were selected in 0.5 mg/ml G418 and 0.5mg/ml HYG to select for cells retaining both the MMR effector and the pCAR-OF reporter plasmids. All cultures transfected with the pCAR vector resulted in a similar number of HYG/G418 resistant cells. Cultures were then expanded and tested for β-galactosidase activity *in situ* as well as by biochemical analysis of cell extracts. For *in situ* analysis, 100,000 cells were harvested and fixed in 1% gluteraldehyde, washed in phosphate buffered saline solution and incubated in 1 ml of X-gal substrate solution [0.15 M NaCl, 1 mM MgCl₂, 3.3 mM K₄Fe(CN)₆, 3.3 mM K₃Fe(CN)₆, 0.2% X-Gal] in 24 well plates for 2 hours at 37°C. Reactions were stopped in 500 mM sodium bicarbonate solution and transferred to microscope slides for analysis. Three fields of 200 cells each were counted for blue (β-galactosidase positive cells) or white (β-galactosidase negative cells) to assess for MMR inactivation. Table 1 shows the results from these studies. While no β-galactosidase positive cells were observed in H36 empty vector cells and 10% of the cells per field were β-galactosidase positive in HB134 cultures.
**Table 1**. β-galactosidase expression of H36 empty vector and HB134 cells transfected with pCAR-OF reporter vectors. Cells were transfected with the pCAR-OF reporter plasmid. Transfected cells were selected in HYG and G418, expanded and stained with X-gal solution to measure for β-galactosidase activity (blue colored cells). 3 fields of 200 cells each were analyzed by microscopy. The results below represent the mean +/standard deviation of these experiments.

**Table 1.**

| CELL LINE | # BLUE CELLS |
|---|---|
| H36 empty vector | 0 +/- 0 |
| HB134 | 20 +/- 3 |

Cultures can be further analyzed by biochemical assays using cell extracts to measure β-galactosidase activity as previously described (Nicolaides, N.C. et al. (1998) Mol. Cell. Biol. 18:1635-1641).

The data described in Table 1 show that by inhibiting the MMR activity of an MMR proficient cell host can result in MI and the altering of microsatellites in the pCAR-OF vector results in cells that produce functional β-galactosidase enzyme. The use of the H36pCAR-OF cell line can now be used to screen for chemicals that are able to block MMR of the H36 cell line.

### EXAMPLE 2: Screening assays for identifying chemical blockers of MMR.

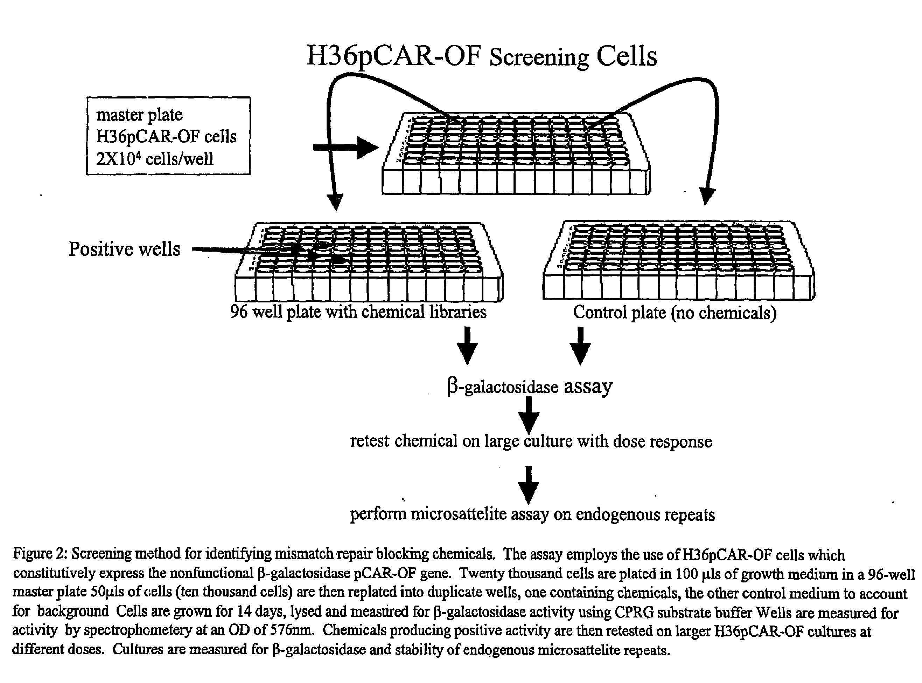

A method for screening chemical libraries is provided in this example using the H36pCAR-OF cell line described in Example 1. This cell line is a hardy, stable line that can be formatted into 96-well microtiter plates for automated screening for chemicals that specifically block MMR. An overview of the screening process is given in Figure 2, however, the process is not limited to the specifications within this example. Briefly, 10,000 cells in a total volume of 0.1ml of growth medium (RPMI1640 plus 10% fetal bovine serum) are added to 96-well microtiter plates containing any variety of chemical compounds. Cells are grown for 14-17 days at 37°C in 5%CO₂. Cells are then lysed in the growth medium with 50uls of lysis buffer containing 0.1 M Tris buffer (pH 8.0), 0.1% Triton X-100, 45 mM 2-mercaptoethanol, 1mM MgCl₂, 0.1 M NaPO₄ and 0.6 mg/ml Chlorophenol-red- β-D-galactopyranoside (CPRG, Roche). Reactions are incubated for 1 hour, terminated by the addition of 50 µls of 0.5 M Na₂CO₃, and analyzed by spectrophotometry at 576 nm.

Experimental wells are compared to untreated or vehicle treated wells to identify those with increased β-galactosidase activity. Compounds producing MMR blocking activity are then further analyzed using different cell lines containing the pCAR-OF plasmid to measure the ability to block MMR as determined by MI in MMR proficient hosts by analyzing endogenous microsatellites for instability using assays described below.

### EXAMPLE 3: Defining MMR blocking chemicals.

The identification of chemical inhibitors of MMR can be difficult in determining those that are standard mutagens from those that induce genomic instability via the blockade of MMR. This Example teaches of a method for determining blockers of MMR from more general mutagens. Once a compound has been identified in the assay described above, one can determine if the compound is a general mutagen or a speific MMR blocker by monitoring mutation rates in MMR proficient cells and a controlled subclone that is MMR defective. One feature of MMR deficiency is the increased resistance to toxicity of DNA alkylating agents that allows for enhanced rates of mutations upon mutagen exposure (Liu, L., et.al. Cancer Res (1996) 56:5375-5379). This unique feature allows for the use of a MMR proficient cell and a controlled line to measure for enhanced activity of a chemical compound to induce mutations in MMR proficient vs MMR deficient lines. If the compound is a true inhibitor of MMR then genetic mutations should occur in MMR proficient cells while no "enhanced " mutation rate will be found in already MMR defective cells. Using these criteria chemicals such as ICR191, which induces frameshift mutations in mammalian cells would not be considered a MMR blocking compound because of its ability to produce enhanced mutation rates in already MMR defective cell lines (Chen, W.D., et.al. J Natl Cancer Inst. (2000) 92:480-485). These screening lines include the but are not limited those in which a dominant negative MMR gene has been introduced such as that described in EXAMPLE 1 or those in which naturally MMR deficient cells such as HCT116 has been cured by introduction of a complementing MMR gene as described (Chen, W.D., et.al. J Natl Cancer Inst. (2000) 92:480-485).

### EXAMPLE 4: Identification of chemical inhibitors of MMR in vivo.

MMR is a conserved post replicative DNA repair mechanism that repairs point mutations and insertion/deletions in repetitive sequences after cell division. The MMR requires an ATPase activity for initiation complex recognition and DNA translocation. *In vitro* assays have shown that the use of nonhydrolyzable forms of ATP such as AMP-PNP and ATP[gamma]S block the MMR activity (Galio, L. et al. (1999) Nucl. Acids Res. 27:2325-2331; Allen, D.J. et al. (1997) EMBO J. 16:4467-4476; Bjornson K.P. et al. (2000) Biochem. 39:3176-3183).

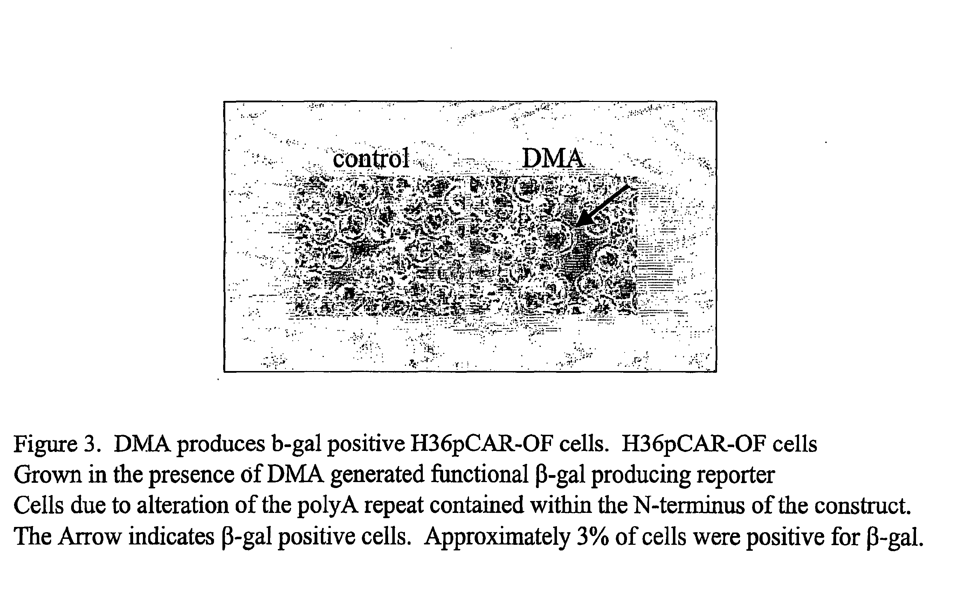

The use of chemicals to inhibit endogenous MMR *in vivo* has not been distinguished in the public domain. In an attempt to identify chemicals that can inhibit MMR *in vivo,* we used our H36pCAR-OF screening assay to screen for chemicals that are able to cause microsatellite instability and restoration of β-galactosidase activity from the pCAR-OF vector, an effect that can only be caused due to MMR deficiency. In our screening assays we used a variety of classes of compounds ranging from steroids such as pontasterone to potent alkylating agents such as EMS, to kinase and other enzyme inhibitors. Screens identified one class of chemicals that were capable of generating β-galactosidase positive cells. These molecules were derived from the anthracene class. An example of one such anthracene derivative for the purposes of this application is a molecule called 9,10-dimethylanthracene, referred to from here on as DMA. Fig. 3 shows the effect of DMA in shifting the pCAR-OF reporter plasmid. In contrast, general DNA alkylating agents such as EMS or MNNG did not result in MI and/or the shifting of the polynulceotide tract in the pCAR-OF reporter.

The most likely explanation for the differences in β-galactosidase activity was that the DMA compound disturbed MMR activity, resulting in a higher frequency of mutation within the pCAR-OF reporter and re-establishing the ORF. To directly test the hypothesis that MMR was altered, we employ a biochemical assay for MMR with the individual clones as described by Nicolaides *et al.,* 1997 (Nicolaides, N.C. et al. (1998) Mol. Cell. Biol. 18:1635-1641). Nuclear extracts are prepared from the clones and incubated with heteroduplex substrates containing either a /CA\ insertion-deletion or a G/T mismatch under conditions described previously. The /CA\ and G/T heteroduplexes are used to test repair from the 3' and 5' directions, respectively as described (Nicolaides, N.C. et al. (1998) Mol. Cell. Biol. 18:1635-1641).

### Biochemical assays for mismatch repair.

### Enzymatic Repair Assays:

MMR activity in nuclear extracts is performed as described, using 24 fmol of substrate (Nicolaides, N.C. et al. (1998) Mol. Cell. Biol. 18:1635-1641). Complementation assays are done by adding ∼ 100 ng of purified MutLa or MutSa components to 100 µg of nuclear extract, adjusting the final KCl concentration to 100 mM (Nicolaides, N.C. et al. (1998) Mol. Cell. Biol. 18:1635-1641). The substrates used in these experiments contain a strand break 181 nucleotides 5' or 125 nucleotides 3' to the mismatch.

### Biochemical Activity Assays:

To demonstrate the direct effect to small molecules on MMR proteins, molecular assays such as mismatch binding and MMR complex formation are performed in the presence or absence of drug. Briefly, MMR gene cDNAs are PCR amplified using primers encompassing the entire coding regions of the known MMR proteins MSH2 (SEQ ID NO:20), GTBP (SEQ ID NO:26), MLH1 (SEQ ID NO:22), human PMS2 (SEQ ID NO:16), mouse PMS2 (SEQ ID NO: 14), PMS1 (SEQ ID NO:18), and MHS3 (SEQ ID NO:28) from any species with a sense primer containing a T7 promoter and a Kozak translation signal as previously described (Nicolaides, N.C. et al. (1998) Mol. Cell. Biol. 18:1635-1641). The coding regions of known MMR proteins include the sequences shown in Table 3 for mouse *PMS2* (SEQ ID NOS:15), human *PMS2* (SEQ ID NO: 17), human *PMS1* (SEQ ID NO:19), human *MSH2* (SEQ ID NO:21), human *MLH1* (SEQ ID NO:23), and human *MSH3* (SEQ ID NO:29). Products are transcribed and translated using the TNT system (Promega). An example of PCR primers and *in vitro* transcription-translation reactions are listed below.

### In vitro transcription-translation:

Linear DNA fragments containing *hPMS2* (SEQ ID NO: 17) and *hMLH1* (SEQ ID NO:23) cDNA sequences were prepared by PCR, incorporating sequences for *in vitro* transcription and translation in the sense primer. A full-length *hMLH1* fragment was prepared using the sense primer
5'-ggatcctaatacgactcactatagggagaccaccatgtcgttcgtggcaggg-3' (SEQ ID NO:1)(codons 1-6) and the antisense primer 5'-taagtcttaagtgctaccaac-3' (SEQ ID NO:2)(located in the 3' untranslated region, nt 2411-2433), using a wild-type *hMLH1* cDNA clone as template. A full-length *hPMS2* fragment was prepared with the sense primer
5'-ggatcctaatacgactcactatagggagaccaccatggaacaattgcctgcgg-3' (SEQ ID NO:3)(codons 1-6) and the antisense primer 5'-aggttagtgaagactctgtc-3' (SEQ ID NO:4)(located in 3' untranslated region, nt 2670-2690) using a cloned *hPMS2* cDNA as template. These fragments were used to produce proteins via the coupled transcription-translation system (Promega). The reactions were supplemented with ³⁵S-labelled methionine or unlabelled methionine. Lower molecular weight bands are presumed to be degradation products and/or polypeptides translated from alternative internal methionines.

To study the effects of MMR inhibitors, assays are used to measure the formation of MLHl and PMS2 with or without compound using polypeptides produced in the TNT System (Promega) followed by immunoprecipitation (IP). To facilitate the IP, tags may be placed at the C-terminus of the PMS2 protein to use for antibody binding or antibodies directed to the MMR protein itself can be used for IP.

### Immunoprecipitations:

Immunoprecipitations are performed on *in vitro* translated proteins by mixing the translation reactions with 1 µg of the MLH1 specific monoclonal antibody (mAB) MLH14 (Oncogene Science, Inc.), a polyclonal antibody generated to codons 2-20 of hPMS2 described above, or a polyclonal antibody generated to codons 843-862 of hPMS2 (Santa Cruz Biotechnology, Inc.) in 400 µl of EBC buffer (50 mM Tris, pH 7.5, 0.1 M NaCl, 0.5% NP40). After incubation for 1 hr at 4°C, protein A sepharose (Sigma) is added to a final concentration of 10% and reactions are incubated at 4°C for 1 hour. Proteins bound to protein A are washed five times in EBC and separated by electrophoresis on 4-20% Tris-glycine gels, which are then dried and autoradiographed.

Compounds that block heterodimerization of mutS or mutL proteins can now be identified using this assay.

### EXAMPLE 5: Use of chemical MMR inhibitors yields microsatellite instability in human cells

In order to demonstrate the global ability of a chemical inhibitor of MMR in host cells and organisms, we treated human HEK293 cells (referred to as 293 cells) with DMA and measured for microsatellite instability of endogenous loci using the BAT26 diagnostic marker (Hoang J.M. et al. (1997) Cancer Res. 57:300-303). Briefly, 105 cells were grown in control medium or 250 µM DMA, a concentration that is found to be non-toxic, for 14 to 17 days. Cells are then harvested and genomic DNA isolated using the salting out method (Nicolaides, N.C. et al. (1991) Mol. Cell. Biol. 11 :6166-6176.).

Various amounts of test DNAs from HCT116 (a human colon epithelial cell line) and 293 were first used to determine the sensitivity of our microsatellite test. The BAT26 alleles are known to be heterogeneous between these two cell lines and the products migrate at different molecular weights (Nicolaides personal observation). DNAs were diluted by limiting dilution to determine the level of sensitivity of the assay. DNAs were PCR amplified using the BAT26F: 5'-tgactacttttgacttcagcc-3' (SEQ ID NO:43) and the BAT26R: 5'-aaccattcaacatttttaaccc-3' (SEQ ID NO:44) primers in buffers as described (Nicolaides, N.C. et al. (1995) Genomics 30:195-206). Briefly 1 pg to 100 ngs of DNA were amplified using the following conditions: 94°C for 30 sec, 58°C for 30 sec, 72°C for 30 sec for 30 cycles. PCR reactions were electrophoresed on 12% polyacrylamide TBE gels (Novex) or 4% agarose gels and stained with ethidium bromide. These studies found that 0.1 ng of genomic DNA was the limit of detection using our conditions.

To measure for microsatellite stability in 293 cells grown with or without DMA, 0.1 ngs of DNA from DMA-treated or control 293 cells were amplified using the reaction conditions above. Forty individual reactions were carried out for each sample to measure for minor alleles. Fig. 4A shows a typical result from these studies whereby BAT26 alleles were amplified from DMA-treated and untreated cells and analyzed on 12% PAGE gels (Novex). Alleles from DMA-treated cells showed the presence of an altered allele (asterisk) that migrated differently from the wild type allele. No altered alleles were found in the MMR-proficient control cells as expected since MI only occurs in MMR defective cell hosts. To confirm these data, PCRs were repeated using isolated BAT26 products. Primers and conditions were the same as described above except that reactions were amplified for 20 cycles. PCR products were gel-purified and cloned into T-tailed vectors (InVitrogen) as suggested by the manufacturer. Recombinant clones from DMA-treated and control cells were screened by PCR again using the BAT26 primers. Fifty bacterial colonies were analyzed for BAT26 structure by directly adding an aliquot of live bacteria to the PCR mix. PCR reactions were carried out as described above, and products were electrophoresed on 4% agarose gels and stained with ethidium bromide. As shown in Figure 4B, microsatellites from DMA-treated cells had alterations (asterisks) that made the marker length larger or smaller than the wild type allele found in control cells.

To confirm that these differences in molecular weight were due to shifts within the polynucleotide repeat, a hallmark of defective MMR, five clones from each sample were sequenced using an ABI automated sequencer with an M13-R primer located in the T-tail vector backbone. Sequence analysis revealed that the control cell clone used in our studies was homozygous for the BAT26 allele with a 26nt polyA repeat. Cells treated with DMA found multiple alleles ranging from the wild-type with 26 polyA repeat to shorter alleles (24 polyA repeat) and larger alleles (28 polyA repeat) (Fig. 5).

These data corroborate the H36pCAR data in Example 1 and Fig. 3 and demonstrates the ability to block MMR with a chemical in a range of hosts.

### Example 6: Chemical inhibitors of MMR generate DNA hypermutability in Plants and new phenotypes.

To determine if chemical inhibitors of MMR work across a diverse array of organisms, we explored the activity of DMA *on Arabidopsis thaliana* (AT), a member of the mustard plant family, as a plant model system to study the effects of DMA on generating MMR deficiency, genome alterations, and new output traits.

Briefly, AT seeds were sterilized with straight commercial bleach and 100 seeds were plated in 100mm Murashige and Skoog (MS) phytagar (Life Technology) plates with increasing amounts of DMA (ranging from 100µm to 50mM). A similar amount of seeds were plated on MS phytagar only or in MS phytagar with increasing amounts of EMS (100µM to 50mM), a mutagen commonly used to mutate AT seeds (McCallum, C.M.et al. (2000) Nat. Biotechnol.18:455-457)*.* Plates were grown in a temperature-controlled, fluorescent-lighted humidifier (Percival Growth Chamber) for 10 days. After 10 days, seeds were counted to determine toxicity levels for each compound. Table 2 shows the number of healthy cells/treatment as determined by root formation and shoot formation. Plantlets that were identical to untreated seeds were scored healthy. Seeds with stunted root or shoot formation were scored intermediate (inter). Non-germinated seeds were scored dead.

**Table 2: Toxicity curve of DMA and EMS on Arabidopsis (per 100 cells)**

| | 0 | 0.1 | 0.5 | 1.0 | 2.5 | 5.0 | 10 | 12.5 | 25 | 50 |
|---|---|---|---|---|---|---|---|---|---|---|
| **DMA** | | | | | | | | | | |
| Healthy | 100 | 94 | 99 | 99 | 80 | 85 | 65 | 0 | 0 | 0 |
| Inter | 0 | 0 | 0 | 0 | 20 | 15 | 32 | 85 | 100 | 0 |
| Dead | 0 | 0 | 0 | 0 | 0 | 0 | 0 | 0 | 0 | 100 |
| **EMS** | | | | | | | | | | |
| Healthy | 99 | 100 | 45 | 25 | 0 | 0 | 0 | 0 | 0 | 0 |
| Inter | 0 | 0 | 54 | 75 | 0 | 0 | 0 | 0 | 0 | 0 |
| Dead | 0 | 0 | 0 | 0 | 100 | 100 | 100 | 100 | 100 | 87 |

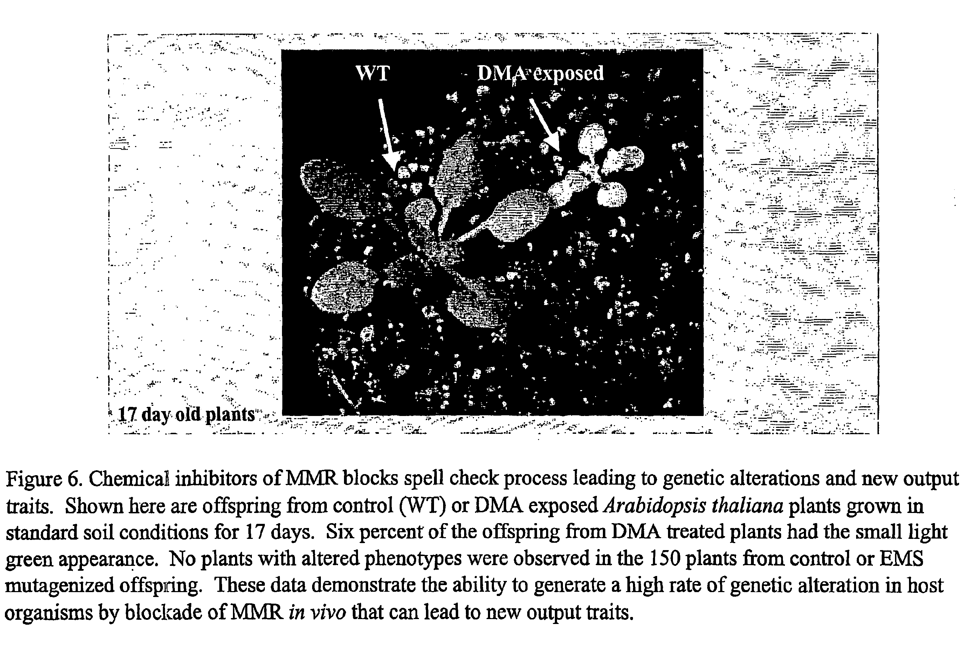

The data in Table 2 show that DMA toxicity occurs at 10mM of continuous culture, while toxicity occurs at 250 µm for EMS. Next, 50 seeds were plated in two 150mm dishes containing 2mM DMA, 250 µM EMS or no drug. Seeds were grown for 10 days and then 10 plants from each plate were transferred to soil. All plants appeared to be similar in color and height. Plants were grown at room temperature with daily cycles of 18 hr light and 6 hr dark. After 45 days seeds are harvested from siliques and stored in a desiccator at 4°C for 72 hours. Seeds are then sterilized and 100 seeds from each plant is sown directly into water-saturated soil and grown at room temperature with daily cycles of 18 hr light and 6 hr dark. At day 10 phenotypically distinct plants were found in 7 out of 118 DMA treated while no phenotypic difference was observed in 150 EMS-treated or 150 control plants. These 7 altered plants were light green in color and appeared to grow slower. Fig. 6 shows a typical difference between the DMA altered plant and controls. DMA-exposed plants produced offspring that were yellow in appearance in contrast to dark green, which is always found in wild-type plants. In addition, the yellow plants were also shorter. After 30 days, most wild-type plants produced flowers and siliques, while the 7 mutants just began flowering. After 45 days, control plants were harvested while mutant plants were harvested 10 to 15 days later. No such effects were observed in 150 plantlets from EMS treated plants.

The effect of DMA on MMR was confirmed by monitoring the structure of endogenous polynucleotide repeat markers within the plant genome. DNA was extracted using the DNAzol method following the manufacturer's protocol (Life Technology). Briefly, two leaves were harvested from DNM EMS or untreated plants and DNA was extracted. DNAs were quantified by optical density using a BioRad Spectrophotometer. In *Arabidopsis,* a series of poly-A (A)ₙ, (CA)ₙ and (GA)ₙ markers were found as a result of EMBL and GenBank database searches of DNA sequence data generated as a result of the *Arabidopsis* genome-sequencing project. Two markers that are naturally occurring, ATHACS and Nga128 are used to monitor microsatellite stability using primers described (Bell, C.J. and J.R. Ecker (1994) Genomics 19:137-144). ATHACS has a stretch of thirty-six adenine repeats (A)₃₆ whereas Nga128 is characterized by a di-nucleotide AG repeat that is repeated nineteen times (AG)₁₉ while the Nga280 marker contains a polyAG repeat marker with 15 dinucleotides. DMA-mediated alterations of these markers are measured by a PCR assay. Briefly, the genomic DNA is amplified with specific primers in PCR reaction buffers described above using 1-10ng plant genomic DNA. Primers for each marker are listed below:
nga280:
   nga280-F: 5'-CTGATCTCACGGACAATAGTGC-3' (SEQ ID NO:5)
   nga280-R: 5'-GGCTCCATAAAAAGTGCACC-3' (SEQ ID NO:6)
nga128:
   nga128-F: 5'-GGTCTGTTGATGTCGTAAGTCG-3' (SEQ ID NO:7)
   nga128-R: 5'-ATCTTGAAACCTTTAGGGAGGG-3' (SEQ ID NO:8)
ATHACS:
   ATHACS-F: 5'-AGAAGTTTAGACAGGTAC-3' (SEQ ID NO:9)
   ATHACS-R: 5'-AAATGTGCAATTGCCTTC-3' (SEQ ID NO:10)

Cycling conditions are 94°C for 15 seconds, 55°C for 15 seconds and 72°C for 30 seconds, conditions that have been demonstrated to efficiently amplify these two markers (personal observation, Morphotek). PCR products are analyzed on 3.5% metaphor agarose gel in Tris-Acetate-EDTA buffer following staining with ethidium bromide.

Another method used to demonstrate that biochemical activity of a plant host's MMR is through the use of reporter genes disrupted by a polynucleotide repeat, similar to that described in Example 1 and Fig. 1. Due to the high endogenous β-galactosidase background, we engineered a plant compatible MMR-sensitive reporter gene consisting of the β-glucoronidase (GUS) gene with a mononucleotide repeat that was inserted just downstream of the initiation codon. Two reporter constructs were generated. pGUS-OF, contained a 20 base adenine repeat inserted just downstream of the initiating methionine that resulted in a frameshift, therefore producing a nonfunctional enzyme. The second, pGUS-IF, contained a 19 base adenine repeat that retained an open reading frame and served as a control for β-glucoronidase activity. Both constructs were generated by PCR using the pBI-121 vector (Life Technologies) as template. The antisense primer was directed to the 3' end of the Nopaline Synthase (NOS) polytermination sequence contained within the pBI-121 plasmid and contained a unique EcoRl restriction site to facilitate cloning of the vector into the pBI-121 binary vector backbone. The sense primers contained a unique *Bam*HI restriction site to facilitate cloning of the chimeric GUS reporter gene into the pBI-121 binary vector backbone. The primers used to generate each reporter are:
1. sense primer for pGUS-IF (uidA-ATG-polyA-IF):
   5'- CCC GGA TCC ATG TTA AAA AAA AAA AAA AAA AAA CGT CCT GTA GAA ACC-3' (SEQ ID NO:11)
2. sense primer for pGUS-OF (uidA-ATG-polyA-OF):
   5'- CCC GGA TCC ATG TTA AAA AAA AAA AAA AAA AAA ACG TCC TGT AGA AAC C-3' (SEQ ID N0:12)
3. antisense primer (Nos-term):
   5'- CCC GAA TTC CCC GAT CTA GTA ACA TAG ATG-3' (SEQ ID NO:13)

PCR amplifications were carried out using reaction buffers described above. Reactions were performed using 1 ng of pBI-121 vector as template (Life Technologies) and the appropriate corresponding primers. Amplifications were carried at 94°C for 30 seconds, 54°C for 60 seconds and 72°C for 60 seconds for 25 cycles. PCR products of the expected molecular weight was gel purified, cloned into T-tailed vectors (InVitrogen), and sequenced to ensure authentic sequence using the following primers: CaMV-FORW. [= 5'-gat atc tcc act gac gta ag-3'] (SEQ ID NO:30) for sequencing from the CaMV promoter into the 5' end of GUS cDNAs; NOSpA-42F [= 5'-tgt tgc cgg tct tgc gat g-3'] (SEQ ID NO:31) for sequencing of the NOS terminator; NOSpA-Cend-R [= 5'-ccc gat cta gta aca tag atg-3'] (SEQ ID NO:32) for sequencing from the NOS terminator into the 3' end of the GUS cDNAs; GUS-63F [= 5'-cag tct gga tcg cga aaa ctg-3'] (SEQ ID NO:33), GUS-441F [= 5'-ggt gat tac cga cga aaa cga-3'] (SEQ ID NO:34), GUS-825F [= 5'-agt gaa ggg cga aca gtt cc-3'] (SEQ ID NO:35), GUS-1224F [= 5'-gag tat tgc caa cga acc-3'] (SEQ ID NO:36), GUS-1596F [= 5'-gta tca ccg cgt ctt tga tc-3'] (SEQ ID NO:37), GUS-265R [= 5'-cga aac gca gca cga tac g-3'] (SEQ ID NO:38), GUS-646R [= 5'-gtt caa cgc tga cat cac c-3'] (SEQ ID NO:39), GUS-1033R [= 5'-cat gtt cat ctg ccc agt cg-3'] (SEQ ID NO:40), GUS-1425R [= 5'-gct ttg gac ata cca tcc-3'] (SEQ ID NO:41), and GUS-1783R [= 5'-cac cga agt tca tgc cag-3'] (SEQ ID NO:42) for the sequence of the full length GUS cDNAs. No mutation were found in either the OF or IF version of the GUS cDNA, and the expected frames for both cDNAs were also confirmed. pCR-IF-GUS and pCR-OF-GUS plasmids were subsequently digested with the BamHI and EcoRI restriction endonucleases, to generate DNA fragments containing the GUS cDNA along with the NOS terminator. These fragments were ligated into the BamHI and the EcoRI sites of the pBI-121 plasmid, which was prepared for cloning by cutting it with the same enzymes to release the wild type GUS cDNA. The resulting constructs (pBI-IF-GUS and pBI-OF-GUS) were subsequently digested with Hind III and EcoR I to release the DNA fragments encompassing the CaMV promoter, the IF or OF GUS cDNA, and the NOS terminator. Finally, these fragments were ligated into the correspondent restriction sites present in the pGPTV-HPT binary vector (ATCC) to obtain the pCMV-IF-GUS-HPT and pCMV-OF-GUS-HPT binary vectors.

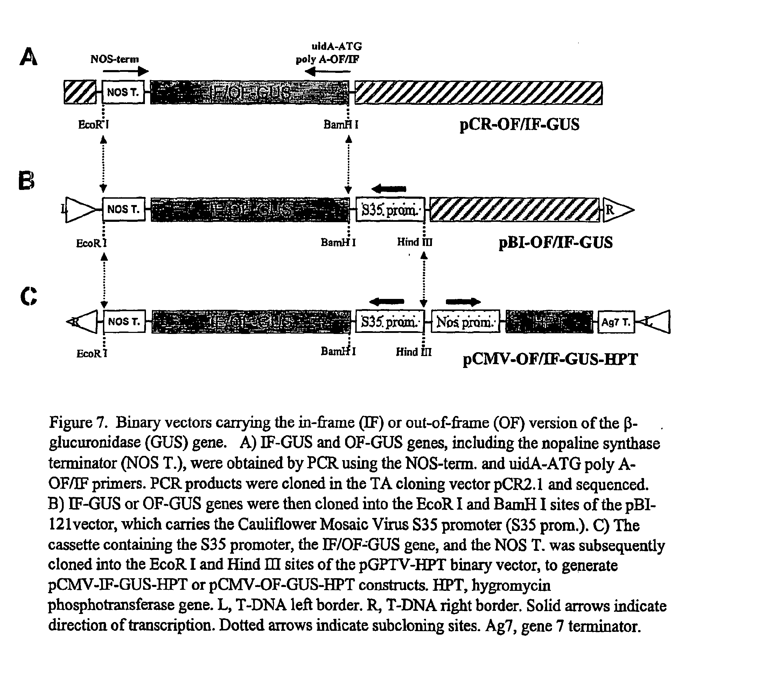

The resulting vectors, CMV-OF-GUS-HPT and CMV-IF-GUS-HPT now contain the CaMV35S promoter from the Cauliflower Mosaic 35 S Virus driving the GUS gene followed by a NOS terminator and polyadenylation signal (Fig. 7). In addition, this vector also contains a hygromycin resistance gene as a selectable marker that is used to select for plants containing this reporter.

### Generation of GUS reporter-expressing Arabidopsis thaliana transgenic plants.

*Agrobacterium tumefaciens* bacteria are used to shuttle binary expression vectors into plants. To generate β-glucoronidase-expressing *Arabidopsis thaliana (A. thaliana)* plants, *Agrobacterium tumefaciens* cells (strain GV3101) were electroporated with the CMV-OF-GUS-HPT or the CMV-IF-GUS-HPT binary vector using methods known by those skilled in the art. Briefly, one-month old *A. thaliana* (ecotype Columbia) plants were infected by immersion in a solution containing 5% sucrose, 0.05% silwet and binary vector-transformed *Agrobacteria* cells for 10 seconds. These plants were then grown at 25°C under a 16 hour day and 8 hour dark photoperiod. After 4 weeks, seeds (referred to as T1) were harvested and dried for 5 days. Thirty thousands seeds from ten CMV-OF-GUS-HPT or CMV-IF-GUS-HPT-transformed plants were sown in solid Murashige and Skoog (MS) media plates in the presence of 20 µg/ml of hygromycin (HYG). Three hundred plants were found to be HYG resistant and represented GUS expressing plants. These plants along with 300 control plants were grown in MS media for two weeks and then transferred to soil. Plants were grown for an additional four weeks under standard conditions at which time T2 seeds were harvested.

To confirm the integration and stability of the GUS vector in the plant genome, gene segregation and PCR analyses were conducted. Commonly, three out of four T1 plants transformed by *Agrobacteria* technology are expected to carry the vector inserted within a single locus and are therefore considered heterozygous for the integrated gene. Approximately 75% of the seeds (T2) generated from most of the T1 plants were found HYG-resistant and this in accordance with the expected 1:2:1 ratio of null (no GUS containing plants), heterozygous, and homozygous plants, respectively, in self-pollinating conditions. To confirm that these plants contained the GUS expression vector, genomic DNA was isolated from leaves of T1 plants using the DNAzol-mediated technique as described above. One ng of genomic DNA was analyzed by polymerase chain reaction (PCR) to confirm the presence of the GUS vector. PCR was carried out for 25 cycles with the following parameters: 95°C for 30 seconds; 54°C for 1 minute; and 72°C for 2 minutes using primers listed above. Positive reactions were observed in DNA from CMV-OF-GUS-HPT and CMV-IF-GUS-HPT-transformed plants and not from control (uninfected) plants.

In order to assess the expression of the GUS in T1 plants, leaf tissue was collected from T1 plants, homogenized in liquid nitrogen using glass pestles, and suspended in RLT lysing buffer (Qiagen, RNeasy plant RNA extraction kit). Five micrograms of total RNA was purified according to the manufacturer's suggested protocol and then loaded onto a 1.2% agarose gel (1x MOPS buffer, 3% formaldehyde), size-fractionated by electrophoresis, and transferred onto N-Hybond+ membrane (Amersham). Each membrane was incubated at 55°C in 10 ml of hybridization solution (North2South labeling kit, Pierce) containing 100 ng of GUS, tubulin, or HYG probes, which were generated by PCR amplification, according to the manufacturer's directions. Membranes were washed three times in 2x SSC, 0.1% SDS at 55°C, and three times in 2x SSC at ambient temperature. Detection was carried out using enhanced chemiluminescence (ECL). GUS message was detected in three out of ten analyzed transgenic lines, while no signal was found in the control plants. Collectively these studies demonstrated the generation of GUS expressing transgenic *A. thaliana* plants.

To determine the status of MR activity in host plants, one can measure for the production of functional β-glucoronidase by staining plant leaves or roots in *situ* for β-glu activity. Briefly, plant tissue is washed twice with water and fixed in 4 mls of 0.02% glutaraldehyde for 15 minutes. Next, tissue is rinsed with water and incubated in X-glu solution [0.1M NaPO₄, 2.5 mM K₃Fe(CN)₆, 2.5mM K₄Fe(CN)₆, 1.5 mM MgCl₂, and 1 mg/ml X-GLU (5 bromo-4-chloro-3-indoyl- β-D-glucuronide sodium salt) (Gold Biotechnology)] for 6 hours at 37°C. Tissues are then washed twice in phosphate buffered saline (PBS) solution, once in 70% ethanol and incubated for 4 hours in methanol:acetone (3:1) for 8 hours to remove chlorophyll. Tissues are then washed twice in PBS and stored in PBS with 50% glycerol. Plant tissue with functional GUS activity will stain blue.

The presence of GUS activity in CMV-IF-GUS-HPT plants indicates that the in-frame N-terminus insertion of the poly A repeat does not disrupt the GUS protein function. The CMV-OF-GUS-HPT plants treated with DMA, EMS or untreated are tested to determine if these plants produce GUS activity. The presence of GUS activity in DMA treated plants indicates that the polyA repeat was altered, therefore, resulting in a frame-restoring mutation. Agents such as EMS, which are known to damage DNA by alkylation cannot affect the stability of a polynucleotide repeat. This data indicates that plants are defective for MMR, the only process known to be responsible for MI.

These data demonstrate the utility and power of using a chemical inhibitor of MMR to generate a high degree of genetic alteration that is not capable by means of standard DNA damaging drugs. Moreover, this application teaches of the use of reporter genes such as GUS-OF in plants to monitor for the MMR activity of a plant host.

### EXAMPLE 7: Use of chemical MMR inhibitors yields microsatellite instability in microbes.

To demonstrate the ability of chemical inhibitors to block MMR in a wide range of hosts, we employed the use of *Pichia* yeast containing a pGUS-OF reporter system similar to that described in Example 5. Briefly, the GUS-OF and GUS-IF gene, which contains a polyA repeat at the N-terminus of the protein was subcloned from the pCR=IF-GUS and pCR-OF-GUS plasmids into the EcoRI site of the pGP vector, which is a consitutively expressed yeast vector containing a zeocin resistance gene as selectable marker. pGP-GUS-IF and pGP-GUS-OF vectors were electroporated into competent *Pichia* cells using standard methods known by those skilled in the art. Cells were plated on YPD agar (10g/L yeast extract; 20 g/L peptone; 2% glucose; 1.5% bactoagar) plates containing 100 µg/ml zeocin. Recombinant yeast are then analyzed for GUS expression/function by replica plating on YPD agar plates containing 100 µg/ml zeocin plus 1 mg/ml X-glu (5-bromo-4-chloro-3-indoyl-beta-D-glucuronide sodium salt) and grown at 30°C for 16 hours. On hundred percent of yeast expressing GUS-IF were found to turn blue in the presence of the X-glu substrate while none of the control yeast turned blue. None of the yeast containing the GUS-OF turned blue in the presence of the X-glu substrate under normal growth conditions.

To demonstrate the ability of chemicals to block MMR in yeast, GUS-OF and control cells were incubated with 300 µM DMA, EMS, or no chemical for 48 hours. After incubation, yeast were plated on YPD-ZEO-X-GLU plates and grown at 30°C for 16 hours. After incubation, a subset of yeast expressing GUS-OF contain blue subclones, while none are seen in EMS or control cells. These data demonstrate the ability of chemicals to block MMR of microbes *in vivo* to produce subclones with new output traits.

### EXAMPLE 8: Classes of other chemicals capable of blocking MMR in vivo

The discovery of anthracene compounds presents a new method for blocking MMR activity of host organisms *in vivo.* While 9,10-dimethylanthracene (DMA) was found to block MMR in cell hosts, other analogs with a similar chemical composition from this class are also claimed in this invention. These include anthracene and related analogs such as 9,10-diphenylanthracene and 9,10-di-M-tolylanthracene. Myers et al. ((1988) Biochem. Biophys. Res. Commun. 151:1441-1445) disclosed that at high concentrations, DMA acts as a potent weak mutagen, while metabolized forms of DMA are the "active" ingredients in promoting mutation. This finding suggests that metabolites of anthracene-based compounds may also act as active inhibitors of *MMR in vivo.* For instance, metabolism of anthracene and 9,10-dimethylanthracene by *Micrococcus sp., Pseudomonas sp.* and *Bacillus macerans* microbes have found a number of anthracene and 9,10-dimethylanthracene metabolites are formed. These include anthracene and 9,10-dimethylanthracene cis-dihydrodiols, hydroxy-methyl-derivatives and various phenolic compounds. Bacteria metabolize hydrocarbons using the dioxygenase enzyme system, which differs from the mammalian cytochrome P-450 monoxygenase. These findings suggest the use of bacteria for biotransforming anthracene and DMA for additional MMR blocking compounds (Traczewska, T.M. et al. (1991) Acta. Microbiol. Pol. 40:235-241). Metabolism studies of DMA by rat-liver microsomal preparations has found that this molecule is converted to 9-Hydroxymethyl-10-methylanthracene (9-OHMeMA) and 9,10-dihydroxymethyl-anthracene (9,10-DiOHMeA) (Lamparczyk, H.S. et al. (1984) Carcinogenesis 5:1405-1410). In addition, the trans-1,2-dihydro-1,2-dihydroxy derivative of DMA (DMA 1,2-diol) was found to be a major metabolite as determined by chromatographic, ultraviolet (UV), nuclear magnetic resonance (MMR), and mass spectral properties. DMA 1,2-diol was also created through the oxidation of DMA in an ascorbic acid-ferrous sulfate-EDTA system. Other dihydrodiols that are formed from DMA by metabolic are the trans-1,2- and 3,4-dihydrodiols of 9-OHMeMA (9-OHMeMA 1,2-diol and 9-OHMeMA 3,4-diol) while the further metabolism of DMA 1,2-diol can yield both of these dihydrodiols. Finally, when 9-OHMeMA is further metabolized, two main metabolites are formed; one was identified as 9,10-DiOHMeA and the other appeared to be 9-OHMeMA 3,4-diol.

The metabolism of 9-methylanthracene (9-MA), 9-hydroxymethylanthracene (9-OHMS), and 9,10-dimethylanthracene (9,10-DMA) by fungus also has been reported (Cerniglia, C.E. et al. (1990) Appl. Environ. Microbiol. 56:661-668). These compounds are also useful for generating DMA derivatives capable of blocking MMR. Compounds 9-MA and 9,10-DMA are metabolized by two pathways, one involving initial hydroxylation of the methyl group(s) and the other involving epoxidation of the 1,2- and 3,4- aromatic double bond positions, followed by enzymatic hydration to form hydroxymethyl transdihydrodiols. For 9-MA metabolism, the major metabolites identified are trans-1,2-dihydro-1,2-dihydroxy and trans-3,4-dihydro-3,4-dihydroxy derivatives of 9-MA and 9-OHMA, whereby 9-OHMA can be further metabolized to trans-1,2- and 3,4-dihydrodiol derivatives. Circular dichroism spectral analysis revealed that the major enantiomer for each dihydrodiol was predominantly in the S,S configuration, in contrast to the predominantly R,R configuration of the trans-dihydrodiol formed by mammalian enzyme systems. These results indicate that *Caenorhabditis elegans* metabolizes methylated anthracenes in a highly stereoselective manner that is different from that reported for rat liver microsomes.

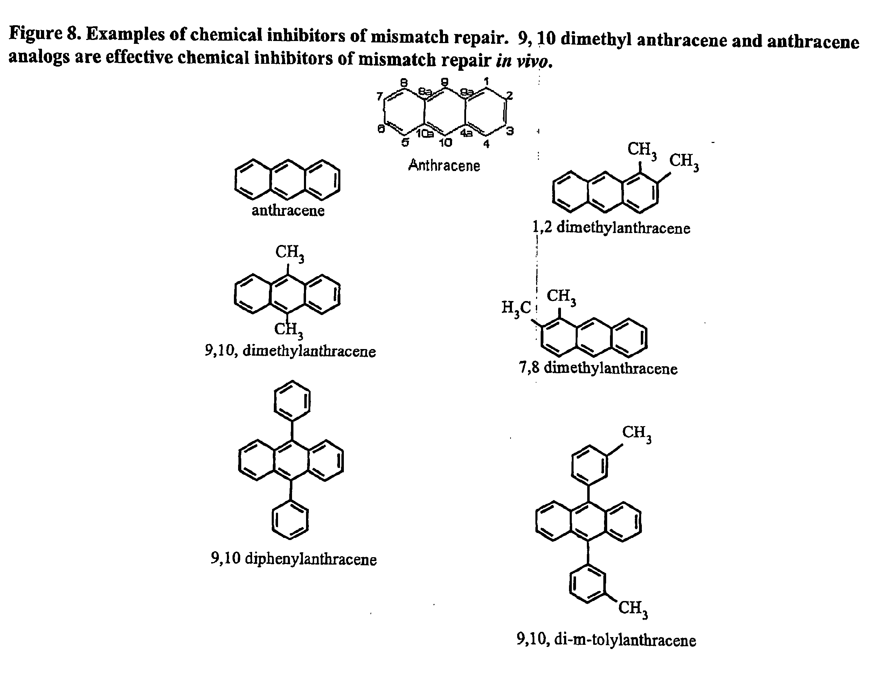

The analogs as listed above provide an example but are not limited to anthracene-derived compounds capable of eliciting MMR blockade. Additional analogs that are of potential use for blocking OMIT are shown in Fig.8.

### Other classes of small molecular weight compounds that are capable of blocking MMR in vivo.

MMR is a multi-step process that involves the formation of protein complexes that detect mismatched bases or altered repetitive sequences and interface these mutations with enzymes that degrade the mutant base and repair the DNA with correct nucleotides. First, mismatched DNA is recognized by the mutS heterodimeric complex consisting of MSH2 and GTBP proteins. The DNA bound mutS complex is then recognized by the mutL heterdimeric complex that consists of PMS2 and MLH1 proteins. The mutL complex is thought to interface exonucleases with the mismatched DNA site, thus initiating this specialized DNA repair process. After the mismatched bases are removed, the DNA is repaired with a polymerase.

There are several steps in the normal process that can be targeted by small molecular weight compounds to block MMR. This application teaches of these steps and the types of compounds that may be used to block this process.

### ATPase inhibitors:

The finding that nonhydrolyzable forms of ATP are able to suppress MMR *in vitro* also suggest that the use for this type of compound can lead to blockade of MMR *in vivo* and mutation a host organism's genome (Galio, L. et al. (1999) Nucl. Acids Res. 27:2325-2331; Allen, D.J. et al. (1997) EMBO J. 16:4467-4476; Bjornson, K.P. et al. (2000) Biochem. 39:3176-3183*).* One can use a variety of screening methods described within this application to identify ATP analogs that block the ATP-dependent steps of mismatch repair *in vivo.*

### Nuclease inhibitors:

The removal of mismatched bases is a required step for effective MMR (Harfe, B.D. and S. Jinks-Robertson (2000) Ann. Rev. Genet. 34:359-399). This suggests that compounds capable of blocking this step can lead to blockade of MMR *in vivo* and mutation a host organism's genome. One can use a variety of screening methods described within this application to identify nuclease inhibitors analogs that block the nuclease steps of mismatch repair *in vivo*. An example of the types of nuclease inhibitors are but not limited to analogs of N-Ethylmaleinude, an endonuclease inhibitor (Huang, Y.C., et.al. (1995) Arch. Biochem. Biophys. 316:485), heterodimeric adenine-chain-acridine compounds, exonulcease III inhibitors (Belmont P, et.al., Bioorg Med Chem Lett (2000) 10:293-295), as well as antibiotic compounds such as Heliquinomycin, which have helicase inhibitory activity (Chino, M, et.al. J. Antibiot. (Tokyo) (1998) 51:480-486).

### Polymerase inhibitors :

Short and long patch repair is a required step for effective MMR (Modrich, P. (1994) Science 266:1959-1960). This suggests that compounds capable ofblocking MMR-associated polymerization can lead to blockade of MMR *in vivo* and mutation a host organism's genome. One can use a variety of screening methods described within this application to identify polymerase inhibitors analogs that block the polymerization steps of mismatch repair *in vivo.* An example of DNA polymerase inhibitors that are useful in blocking MMR activity include, but are not limited to, analogs of actinomycin D (Martin, S.J., et.al. (1990) J. Immunol. 145:1859), Aphidicolin (Kuwakado, K. et.al. (1993) Biochem. Pharmacol. 46:1909) 1-(2'-Deoxy-2'-fluoro-beta-L-arabinofuranosyl)-5-methyluracil (L-FMAU) (Kukhanova M, et.al., Biochem Pharmacol (1998) 55:1181-1187), and 2',3'-dideoxyribonucleoside 5'-triphosphates (ddNTPs) (Ono, K., et.al., Biomed Pharmacother (1984) 38:382-389).

### Chemical Inhibitors of Mismatch Repair Gene Expression

MMR is a multi-protein process that requires the cooperation of several proteins such as but not limited to mutS homologs, MSH2, MSH3, MSH6, GTBP; mutL homologs PMS1, PMS2, MLH1; and exonucleases and helicases such as MutH and MutY (Harfe, B.D. and S. Jinks-Robertson (2000) Ann. Rev. Genet. 34:359-399; Modrich, P. (1994) Science 266:1959-1960). Chemicals capable of blocking the expression of these genes can lead to the blockade of MMR. An example of a chemical that is capable of blocking MMR gene expression is an oligodeoxynucleotide that can specifically bind and degrade an MMR gene message and protein production as described by Chauhan DP, et.al. (Clin Cancer Res (2000) 6:3827-3831). One can use a variety of screening methods described within this application to identify inhibitors that block the expression and/or function of MMR genes *in vivo.*

### DISCUSSION

The results described herein demonstrate the use of chemicals that can block mismatch repair of host organisms *in vivo* to produce genetic mutations. The results also demonstrate the use of reporter systems in host cells and organisms that are useful for screening chemicals capable of blocking MMR of the host organism. Moreover, the results demonstrate the use of chemical inhibitors to block MMR in mammalian cells, microbes, and plants to produce organisms with new output traits. The data presented herein provide novel approaches for producing genetically altered plants, microbes, and mammalian cells with output traits for commercial applications by inhibiting MMR with chemicals. This approach gives advantages over others that require the use of recombinant techniques to block MMR or to produce new output traits by expression of a foreign gene. This method will be useful in producing genetically altered host organisms for agricultural, chemical manufacturing, pharmaceutical, and environmental applications.
PMS2 (mouse) (SEQ ID NO:14)
PMS2 (mouse cDNA) (SEQ ID NO:15)
PMS2 (human) (SEQ ID NO:16)
PMS2 (human cDNA) (SEQ ID NO:17)
PMS 1 (human) (SEQ ID NO:18)
PMS 1 (human) (SEQ ID NO:19)
MSH2 (human) (SEQ ID NO:20)
MSH2 (human cDNA) (SEQ ID NO:21)
MLHI (human) (SEQ ID NO:22)
MLH1 (human) (SEQ ID NO:23)
hPMS2-134 (human) (SEQ ID NO:24)
hPMS2-134 (human cDNA) (SEQ ID NO:25)
GTBP (human) (SEQ ID NO:26)
GTBP (human cDNA) (SEQ ID NO:27)
MSH3 (human) (SEQ ID NO:28)
MSH3 (human DNA) (SEQ ID NO:29)

## Claims

1. An *in vitro* method for making a mammalian or plant cell exhibiting microsatellite instability comprising exposing a mammalian or plant cell to an inhibitor of mismatch repair, wherein said inhibitor is an-anthracene, wherein said anthracene has the formula: 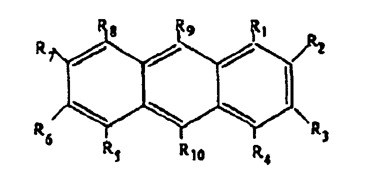 wherein R₁-R₁₀ are independently hydrogen, hydroxyl, amino, alkyl, substituted alkyl, alkenyl, substituted alkenyl, alkynyl, substituted alkynyl, O-alkyl, S-alkyl, N-alkyl, O-alkenyl, S-alkenyl, N-alkenyl, O-alkynyl, S-alkynyl, N-alkynyl, aryl, substituted aryl, aryloxy, substituted aryloxy, heteroaryl, substituted heteroaryl, aralkyloxy, arylalkyl, alkylaryl, alkylaryloxy, arylsulfonyl, alkylsulfonyl, alkoxycarbonyl, aryloxycarbonyl, guanidino, carboxy, an alcohol, an amino acid, sulfonate, alkyl sulfonate, CN, NO₂, an aldehyde group, an ester, an ether, a crown ether, a ketone,
an organosulfur compound, an organometallic group, a carboxylic acid, an organosilicon or a carbohydrate that optionally contains one or more alkylated hydroxyl groups;
wherein said heteroalkyl, heteroaryl, and substituted heteroaryl contain at least one heteroatom that is oxygen, sulfur, a metal atom, phosphorus, silicon or nitrogen; and wherein said substituents of said substituted alkyl, substituted alkenyl, substituted alkynyl, substituted aryl, and substituted heteroaryl are halogen, CN, NO₂ lower alkyl, aryl, heteroaryl, aralkyl, aralkyloxy, guanidino, alkoxycarbonyl, alkoxy, hydroxy, carboxy and amino;
wherein said amino groups are optionally substituted with an acyl group, or 1 to 3 aryl or lower alkyl groups;
or wherein any two of R₁-R₁₀ can together form a polyether;
or wherein any two of R₁-R₁₀ can together with the intervening carbon atoms of the anthracene core form a crown ether;
culturing said mammalian or plant cell; and
selecting cells exhibiting microsatellite instability.

2. A method for making a plant comprising cells exhibiting microsatellite instability, said method comprising exposing at least one cell of said plant to an inhibitor of mismatch repair, wherein said inhibitor is an anthracene, wherein said anthracene has the formula: 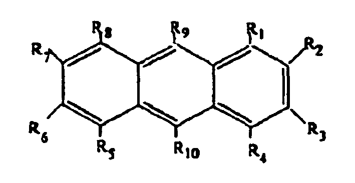 wherein R₁-R₁₀ are independently hydrogen, hydroxyl, amino, alkyl, substituted alkyl, alkenyl, substituted alkenyl, alkynyl, substituted alkynyl, O-alkyl, S-alkyl, N-alkyl, O-alkenyl, S-alkenyl, N-alkenyl, O-alkynyl, S-alkynyl, N-alkynyl, aryl, substituted aryl, aryloxy, substituted aryloxy, heteroaryl, substituted heteroaryl, aralkyloxy, arylalkyl, alkylaryl, alkylaryloxy, arylsulfonyl, alkylsulfonyl, alkoxycarbonyl, aryloxycarbonyl, guanidino, carboxy, an alcohol, an amino acid, sulfonate, alkyl sulfonate, CN, NO₂, an aldehyde group, an ester, an ether, a crown ether, a ketone, an organosulfur compound, an organometallic group, a carboxylic acid, an organosilicon or a carbohydrate that optionally contains one or more alkylated hydroxyl groups;
wherein said heteroalkyl, heteroaryl, and substituted heteroaryl contain at least one heteroatom that is oxygen, sulfur, a metal atom, phosphorus, silicon or nitrogen ; and
wherein said substituents of said substituted alkyl, substituted alkenyl, substituted alkynyl, substituted aryl, and substituted heteroaryl are halogen; CN, NO₂, lower alkyl, aryl, heteroaryl, aralkyl, aralkyloxy, guanidino, alkoxycarbonyl, alkoxy, hydroxy, carboxy and amino; and wherein said amino groups are optionally substituted with an acyl group, or I to 3 aryl or lower alkyl groups;
or wherein any two of R₁-R₁₀ can together form a polyether;
or wherein any two of R₁-R₁₀ can together with the intervening carbon atoms of the anthracene core form a crown ether; and
selecting plants comprising cells exhibiting microsatellite instability.

3. A method according to claim 2 wherein said inhibitor is added to the growth medium of said plant.

4. A method according to any preceding claim, wherein R₁-R₁₀ are independently hydrogen, hydroxyl, alkyl, aryl, arylalkyl, hydroxyalkyl, methyl, ethyl, propyl, isopropyl, butyl, isobutyl, phenyl, tolyl, hydroxymethyl, hydroxypropyl or hydroxybutyl.

5. A method according to any of claims I to 3, wherein said anthracene is selected from 1,2-dimethylanthracene, 9, 10-dimethylanthracene, 7,8-dimethylanthracene, 9,10-diphenylanthracene, 9, 10-dihydroxymethylanthracene, 9-hydroxymethyl-10-methylanthracene, dimethylanthracene-1,2-diol, 9-hydroxymethyl-10-methylanthracene-1,2-diol, 9-hydroxymethyl-10-methylanthracene-3,4-diol and 9,10-di-m-tolylanthracene.

6. A method according to any of claims 1 to 3, wherein R₅ and R₆ are hydrogen.

7. A method according to any of claims 1 to 3, wherein R₃, R₄ R₅, R₆, R₇, R₈, R₉, and R₁₀ are hydrogen.

8. A method according to any of claims 1 to 3, wherein R₁ R₂, R₃, R₄, R₅, R₆, R₇, and R₈ are hydrogen.

9. A method according to any of claims 1 to 3, wherein R₃, R₄, R₅, R₆, R₇, and Rg are hydrogen.

10. A method according to any of claims 1 to 3, wherein R₁, R₂, R₃, R₄, R₅, R₆, R₉, and R₁₀ are hydrogen.

11. A method according to any of claims 1 to 3, wherein, R₁, R₂, R₅, R₆, R₇, and R₈ are hydrogen.

12. A method according to any of claims 1 to 3, wherein R₁, R₂, R₃, R₄, R₅, R₆, R₇, R₈ and R₁₀ are hydrogen.

13. An *in vitro* method for generating a mutation in a gene of interest comprising exposing a cell comprising said gene of interest to an inhibitor of mismatch repair, wherein said inhibitor is an anthracene having the formula: 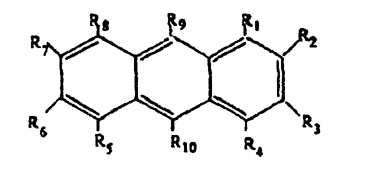 wherein R₁-R₁₀ are independently hydrogen, hydroxyl, amino, alkyl, substituted alkyl, alkenyl, substituted alkenyl, alkynyl, substituted alkynyl, O-alkyl, S-alkyl, N-alkyl, O-alkenyl, S-alkenyl, N-alkenyl, O-alkynyl, S-alkynyl, N-alkynyl, aryl, substituted aryl, aryloxy, substituted aryloxy, heteroaryl, substituted heteroaryl, aralkyloxy, arylalkyl, alkylaryl, alkylaryloxy, arylsulfonyl, alkylsulfonyl, alkoxycarbonyl, aryloxycarbonyl, guanidino, carboxy, an alcohol, an amino acid, sulfonate, alkyl sulfonate, CN, NOz, an aldehyde group, an ester, an ether, a crown ether, a ketone, an organosulfur compound, an organometallic group, a carboxylic acid, an organosilicon or a carbohydrate that optionally contains one or more alkylated hydroxyl groups;
wherein said heteroalkyl, heteroaryl, and substituted heteroaryl contain at least one heteroatom that is oxygen, sulfur, a metal atom, phosphorus, silicon or nitrogen; and
wherein said substituents of said substituted alkyl, substituted alkenyl, substituted alkynyl, substituted aryl, and substituted heteroaryl are halogen, CN, NO₂, lower alkyl, aryl, heteroaryl, aralkyl, aralkyloxy, guanidino, alkoxycarbonyl, alkoxy, hydroxy, carboxy and amino;
wherein said amino groups are optionally substituted with an acyl group, or 1 to 3 aryl or lower aikyi groups; or wherein any two of R₁-R₁₀ can together form a polyether;
or wherein any two of R₁-R₁₀ can together with the intervening carbon atoms of the anthracene core form a crown ether;
culturing said cell comprising said gene of interest;
selecting cells comprising said gene of interest and exhibiting microsatellite instability; and testing said cells to determine whether said gene of interest comprises a mutation.

14. A method according to claim 13, wherein said testing comprises analyzing a polynucleotide sequence of said gene of interest.

15. A method according to claim 13 or 14, wherein said cell is a mammalian cell.

16. A method according to any of claims 13 to 15, including exposing said cell to a mutagen.

17. A method according to claim 16, wherein said mutagen is selected from N-methyl-N'-nitro-N-nitrosoguanidine, methane sulfonate, dimethyl sulfonate, 0-6-methylbenzadine, ethyl methanesulfonate, methylnitrosourea and ethylnitrosourea.

18. A method according to any of claims 13 to 17, including the step of removing said inhibitor of mismatch repair after testing said cell to determine whether said gene of interest comprises a mutation.

19. A method according to any of claims 1 to 3 or 13 to 18, wherein said anthracene is selected from 1,2-dimethylanthracene, 9,10-dimethylanthracene, 7,8-dimethylanthracene, 9,10-diphenylanthracene, 9,10-dihydroxymethylanthracene, 9-hydroxymethyl-10-methylanthracene, dimethylanthracene-1,2-diol, 9-hydroxymethyl-10-methylanthracene-1,2-diol, 9-hydroxymethyl-10-methylanthracene-3,4-diol and 9,10-di-m-tolylanthracene.

20. A method according to any preceding claim further comprising removing said inhibitor of mismatch repair.

## Patentansprüche

1. In vitro-Verfahren zur Herstellung einer Säuger- oder Pflanzenzelle, die Mikrosatelliten-Instabilität aufweist, umfassend das Aussetzen einer Säuger- oder Pflanzenzelle einem Inhibitor einer Fehlpaarungsreparatur, wobei der Inhibitor ein Anthracen ist, wobei das Anthracen die Formel 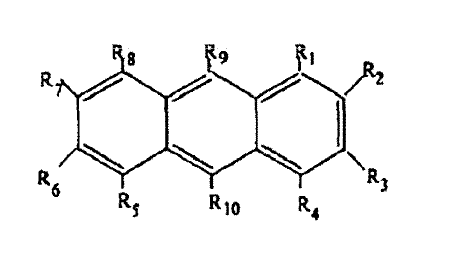 aufweist, wobei R₁-R₁₀ unabhängig Wasserstoff, Hydroxyl, Amino, Alkyl, substituiertes Alkyl, Alkenyl, substituiertes Alkenyl, Alkinyl, substituiertes Alkinyl, O-Alkyl, S-Alkyl, N-Alkyl, O-Alkenyl, S-Alkenyl, N-Alkenyl, O-Alkinyl, S-Alkinyl, N-Alkinyl, Aryl, substituiertes Aryl, Aryloxy, substituiertes Aryloxy, Heteroaryl, substituiertes Heteroaryl, Aralkyloxy, Arylalkyl, Alkylaryl, Alkylaryloxy, Arylsulfonyl, Alkylsulfonyl, Alkoxycarbonyl; Aryloxycarbonyl, Guanidino, Carboxyl, einen Alkohol, eine Aminosäure, Sulfonat, Alkylsulfonat, CN, NO₂, einen Aldehydrest, einen Ester, einen Ether, einen Kronenether, ein Keton, eine Organoschwefelverbindung, Organometallgruppe, eine Carbonsäure, ein Organosilicium oder ein Kohlenhydrat, welches gegebenenfalls eine oder mehrere alkylierte Hydroxylgruppen enthält, darstellen;
wobei das Heteroalkyl, Heteroaryl und substituierte Heteroaryl mindestens ein Heteroatom enthalten, das Sauerstoff, Schwefel, ein Metallatom, Phosphor, Silicium oder Stickstoff darstellt; und
wobei die Substituenten des substituierten Alkyls, substituierten Alkenyls,
substituierten Alkinyls, substituierten Aryls und substituierten Heteroaryls Halogen, CN, NO₂, Niederalkyl, Aryl, Heteroaryl, Aralkyl, Aralkyloxy, Guanidino, Alkoxycarbonyl, Alkoxy, Hydroxyl, Carboxyl und Amino darstellen;
wobei die Aminogruppen gegebenenfalls mit einem Acylrest oder 1 bis 3 Aryl oder Niederalkylresten substituiert sind;
oder wobei beliebige zwei von R₁-R₁₀ zusammen einen Polyether bilden können;
oder wobei beliebige zwei von R₁-R₁₀ zusammen mit den dazwischen liegenden Kohlenstoffatomen des Anthracenkerns einen Kronenether bilden können;
Züchten der Säuger- oder Pflanzenzelle; und
Auswählen von Zellen, die Mikrosatelliten-Instabilität aufweisen.

2. Verfahren zur Herstellung einer Pflanze, die Zellen umfasst, welche Mikrosatelliten-Instabilität aufweisen, wobei das Verfahren das Aussetzen mindestens einer Zelle der Pflanze einem Inhibitor einer Fehlpaarungsreparatur umfasst, wobei der Inhibitor ein Anthracen ist, wobei das Anthracen die Formel 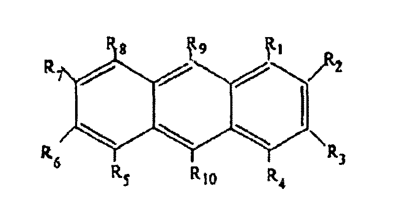 aufweist, wobei R₁-R₁₀ unabhängig Wasserstoff, Hydroxyl, Amino, Alkyl, substituiertes Alkyl, Alkenyl, substituiertes Alkenyl, Alkinyl, substituiertes Alkinyl, O-Alkyl, S-Alkyl, N-Alkyl, O-Alkenyl, S-Alkenyl, N-Alkenyl, O-Alkinyl, S-Alkinyl, N-Alkinyl, Aryl, substituiertes Aryl, Aryloxy, substituiertes Aryloxy, Heteroaryl, substituiertes Heteroaryl, Aralkyloxy, Arylalkyl, Alkylaryl, Alkylaryloxy, Arylsulfonyl, Alkylsulfonyl, Alkoxycarbonyl, Aryloxycarbonyl, Guanidino, Carboxyl, einen Alkohol, eine Aminosäure, Sulfonat, Alkylsulfonat, CN, NO₂, einen Aldehydrest, einen Ester, einen Ether, einen Kronenether, ein Keton, eine Organoschwefelverbindung, eine Organometallgruppe, eine Carbonsäure, ein Organosilicium oder ein Kohlenhydrat, welches gegebenenfalls eine oder mehrere alkylierte Hydroxylgruppen enthält, darstellen;
wobei das Heteroalkyl, Heteroaryl und substituierte Heteroaryl mindestens ein Heteroatom enthalten, das Sauerstoff, Schwefel, ein Metallatom, Phosphor, Silicium oder Stickstoff darstellt; und
wobei die Substituenten des substituierten Alkyls, substituierten Alkenyls, substituierten Alkinyls, substituierten Aryls und substituierten Heteroaryls Halogen, CN, NO₂, Niederalkyl, Aryl, Heteroaryl, Aralkyl, Aralkyloxy, Guanidino, Alkoxycarbonyl, Alkoxy, Hydroxyl, Carboxyl und Amino darstellen; und
wobei die Aminogruppen gegebenenfalls mit einem Acylrest oder 1 bis 3 Aryl oder Niederalkylresten substituiert sind;
oder wobei beliebige zwei von R₁-R₁₀ zusammen einen Polyether bilden können;
oder wobei beliebige zwei von R₁-R₁₀ zusammen mit den dazwischenliegenden Kohlenstoffatomen des Anthracenkerns einen Kronenether bilden können; und Auswählen von Pflanzen, die Zellen umfassen, welche Mikrosatelliten-Instabilität aufweisen.

3. Verfahren gemäß Anspruch 2, wobei der Inhibitor dem Wachstumsmedium der Pflanze zugegeben wird.

4. Verfahren gemäß einem der vorstehenden Ansprüche, wobei R₁-R₁₀ unabhängig Wasserstoff, Hydroxyl, Alkyl, Aryl, Arylalkyl, Hydroxyalkyl, Methyl, Ethyl, Propyl, Isopropyl, Butyl, Isobutyl, Phenyl, Tolyl, Hydroxymethyl, Hydroxypropyl oder Hydroxybutyl darstellen.

5. Verfahren gemäß einem der Ansprüche 1 bis 3, wobei das Anthracen ausgewählt ist aus 1,2-Dimethylanthracen, 9,10-Dimethylanthracen, 7,8-Dimethylanthracen, 9,10-Diphenylanthracen, 9,10-Dihydroxymethylanthracen, 9-Hydroxymethyl-10-methylanthracen, Dimethylanthracen-1,2-diol, 9-Hydroxymethyl-10-methylanthracen-1,2-diol, 9-Hydroxymethyl-10-methylanthracen-3,4-diol und 9,10-Di-m-tolylanthracen.

6. Verfahren gemäß einem der Ansprüche 1 bis 3, wobei R₅ und R₆ Wasserstoff sind.

7. Verfahren gemäß einem der Ansprüche 1 bis 3, wobei R₃, R₄, R₅, R₆, R₇, R₈, R₉ und R₁₀ Wasserstoff sind.

8. Verfahren gemäß einem der Ansprüche 1 bis 3, wobei R₁, R₂, R₃, R₄, R₅, R₆, R₇ und R₈ Wasserstoff sind.

9. Verfahren gemäß einem der Ansprüche 1 bis 3, wobei R₃, R₄, R₅, R₆, R₇ und R₈ Wasserstoff sind.

10. Verfahren gemäß einem der Ansprüche 1 bis 3, wobei R₁, R₂, R₃, R₄, R₅, R₆, R₉ und R₁₀ Wasserstoff sind.

11. Verfahren gemäß einem der Ansprüche 1 bis 3, wobei R₁, R₂, R₅, R₆, R₇ und R₈ Wasserstoff sind.

12. Verfahren gemäß einem der Ansprüche 1 bis 3, wobei R₁, R₂, R₃, R₄, R₅, R₆, R₇, R₈ und R₁₀ Wasserstoff sind.

13. In vitro-Verfahren zum Generieren einer Mutation in einem Gen von Interesse, umfassend das Aussetzen einer Zelle, die das Gen von Interesse umfasst, einem Inhibitor einer Fehlpaarungsreparatur, wobei der Inhibitor ein Anthracen mit der Formel 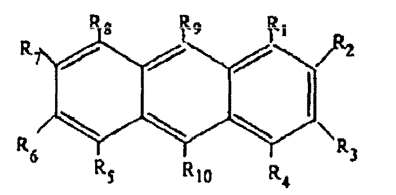 darstellt, wobei R₁-R₁₀ unabhängig Wasserstoff, Hydroxyl, Amino, Alkyl, substituiertes Alkyl, Alkenyl, substituiertes Alkenyl, Alkinyl, substituiertes Alkinyl, O-Alkyl, S-Alkyl, N-Alkyl, O-Alkenyl, S-Alkenyl, N-Alkenyl, O-Alkinyl, S-Alkinyl, N-Alkinyl, Aryl, substituiertes Aryl, Aryloxy, substituiertes Aryloxy, Heteroaryl, substituiertes Heteroaryl, Aralkyloxy, Arylalkyl, Alkylaryl, Alkylaryloxy, Arylsulfonyl, Alkylsulfonyl, Alkoxycarbonyl, Aryloxycarbonyl, Guanidino, Carboxyl, einen Alkohol, eine Aminosäure, Sulfonat, Alkylsulfonat, CN, NO₂, einen Aldehydrest, einen Ester, einen Ether, einen Kronenether, ein Keton, eine Organoschwefelverbindung, eine Organometallgruppe, eine Carbonsäure, ein Organosilicium oder ein Kohlenhydrat, welches gegebenenfalls eine oder mehrere alkylierte Hydroxylgruppen enthält, darstellen;
wobei das Heteroalkyl, Heteroaryl und substituierte Heteroaryl mindestens ein Heteroatom enthalten, das Sauerstoff, Schwefel, ein Metallatom, Phosphor, Silicium oder Stickstoff darstellt; und
wobei die Substituenten des substituierten Alkyls, substituierten Alkenyls, substituierten Alkinyls, substituierten Aryls und substituierten Heteroaryls Halogen, CN, NO₂, Niederalkyl, Aryl, Heteroaryl, Aralkyl, Aralkyloxy, Guanidino, Alkoxycarbonyl, Alkoxy, Hydroxyl, Carboxyl und Amino darstellen;
wobei die Aminogruppen gegebenenfalls mit einem Acylrest oder 1 bis 3 Aryl oder Niederalkylresten substituiert sind;
oder wobei beliebige zwei von R₁-R₁₀ zusammen einen Polyether bilden können;
oder wobei beliebige zwei von R₁-R₁₀ zusammen mit den dazwischenliegenden Kohlenstoffatomen des Anthracenkerns einen Kronenether bilden können;
Züchten der Zelle, die das Gen von Interesse umfasst;
Auswählen von Zellen, die das Gen von Interesse umfassen und Mikrosatelliten-Instabilität aufweisen; und
Testen der Zellen, um festzustellen, ob das Gen von Interesse eine Mutation umfasst.

14. Verfahren gemäß Anspruch 13, wobei das Testen das Analysieren einer Polynucleotidsequenz des Gens von Interesse umfasst.

15. Verfahren gemäß Anspruch 13 oder 14, wobei die Zelle eine Säugerzelle ist.

16. Verfahren gemäß einem der Ansprüche 13 bis 15, das das Aussetzen der Zelle einem Mutagen einschließt.

17. Verfahren gemäß Anspruch 16, wobei das Mutagen ausgewählt ist aus N-Methyl-N'-nitro-N-nitrosoguanidin, Methansulfonat, Dimethylsulfonat, O-6-Methylbenzadin, Ethylmethansulfonat, Methylnitrosoharnstoff und Ethylnitrosoharnstoff.

18. Verfahren gemäß einem der Ansprüche 13 bis 17, das den Schritt der Entfernung des Inhibitors einer Fehlpaarungsreparatur nach dem Testen der Zelle einschließt, um festzustellen, ob das Gen von Interesse eine Mutation umfasst.

19. Verfahren gemäß einem der Ansprüche 1 bis 3 oder 13 bis 18, wobei das Anthracen ausgewählt ist aus 1,2-Dimethylanthracen, 9,10-Dimethylanthracen, 7,8-Dimethylanthracen, 9,10-Diphenylanthracen, 9,10-Dihydroxymethylanthracen, 9-Hydroxymethyl-10-methylanthracen, Dimethylanthracen-1,2-diol, 9-Hydroxymethyl-10-methylanthracen-1,2-diol, 9-Hydroxymethyl-10-methylanthracen-3,4-diol und 9,10-Di-m-tolylanthracen.

20. Verfahren gemäß einem der vorstehenden Ansprüche, ferner umfassend das Entfernen des Inhibitors einer Fehlpaarungsreparatur.

## Revendications

1. Procédé *in vitro* pour produire une cellule de mammifère ou végétale présentant une instabilité de microsatellites comprenant l'exposition d'une cellule de mammifère ou végétale à un inhibiteur de réparation des mésappariements, où ledit inhibiteur est un anthracène, où ledit anthracène a la formule : 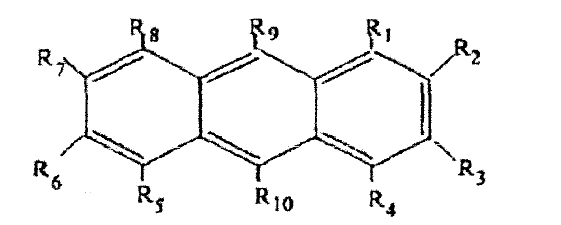 où R₁-R₁₀ sont indépendamment hydrogène, hydroxyle, amino, alkyle, alkyle substitué, alcényle, alcényle substitué, alcynyle, alcynyle substitué, O-alkyle, S-alkyle, N-alkyle, O-alcényle, S-alcényle, N-alcényle, O-alcynyle, S-alcynyle, N-alcynyle, aryle, aryle substitué, aryloxy, aryloxy substitué, hétéroaryle, hétéroaryle substitué, aralkyloxy, arylalkyle, alkylaryle, alkylaryloxy, arylsulfonyle, alkylsulfonyle, alcoxycarbonyle ; aryloxycarbonyle, guanidino, carboxy, un alcool, un aminoacide, sulfonate, alkylsulfonate, CN, NO₂, un groupe aldéhyde, un ester, un éther, un éther couronne, une cétone, un composé organosoufré, un groupe organométallique, un acide carboxylique, un organosilicium ou un glucide qui contient éventuellement un ou plusieurs groupes hydroxyle alkylés ; où lesdits hétéroalkyle, hétéroaryle et hétéroaryle substitué contiennent au moins un hétéroatome qui est l'oxygène, le soufre, un atome métallique, le phosphore, le silicium ou l'azote; et où lesdits substituants desdits alkyle substitué, alcényle substitué, alcynyle substitué, aryle substitué et hétéroaryle substitué sont halogène, CN, NO₂, alkyle inférieur, aryle, hétéroaryle, aralkyle, aralkyloxy, guanidino, alcoxycarbonyle, alcoxy, hydroxy, carboxy et amino ;
où lesdits groupes amino sont éventuellement substitués avec un groupe acyle, ou 1 à 3 groupes aryle ou alkyle inférieur ;
ou bien où deux quelconques de R₁-R₁₀ peuvent former ensemble un polyéther ;
ou bien où deux quelconques de R₁-R₁₀ peuvent former un éther couronne avec les atomes de carbone intermédiaires du noyau anthracène,
la culture de ladite cellule de mammifère ou végétale ; et
la sélection de cellules présentant une instabilité de microsatellites.

2. Procédé pour produire une plante comprenant des cellules présentant une instabilité de microsatellites, ledit procédé comprenant l'exposition d'au moins une cellule de ladite plante à un inhibiteur de réparation des mésappariements, où ledit inhibiteur est un anthracène, où ledit anthracène a la formule : 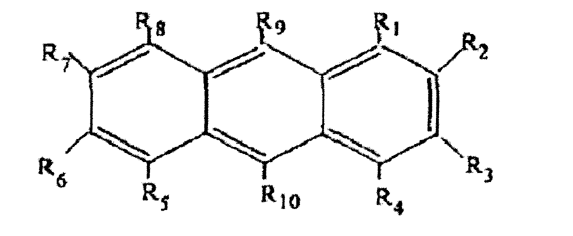 où R₁-R₁₀ sont indépendamment hydrogène, hydroxyle, amino, alkyle, alkyle substitué, alcényle, alcényle substitué, alcynyle, alcynyle substitué, O-alkyl, S-alkyle, N-alkyle, O-alcényle, S-alcényle, N-alcényle, O-alcynyle, S-alcynyle, N-alcynyle, aryle, aryle substitué, aryloxy, aryloxy substitué, hétéroaryle, hétéroaryle substitué, aralkyloxy, arylalkyle, alkylaryle, alkylaryloxy, arylsulfonyle, alkylsulfonyle, alcoxycarbonyle, aryloxycarbonyle, guanidino, carboxy, un alcool, un aminoacide, sulfonate, alkylsulfonate, CN, NO₂, un groupe aldéhyde, un ester, un éther, un éther couronne, une cétoine, un composé organosoufré, un groupe organométallique, un acide carboxylique, un organosilicium ou un glucide qui contient éventuellement un ou plusieurs groupes hydroxyle alkylés ; où lesdits hétéroalkyle, hétéroaryle et hétéroaryle substitué contiennent au moins un hétéroatome qui est l'oxygène, le soufre, un atome métallique, le phosphore, le silicium ou l'azote ; et ou lesdits substituants desdits alkyle substitué, alcényle substitué, alcynyle substitué, aryle substitué et hétéroaryle substitué sont halogène, CN, NO₂, alkyle inférieur, aryle, hétéroaryle, aralkyle, aralkyloxy, guanidino, alcoxycarbonyle, alcoxy, hydroxy, carboxy et amino ; et
où lesdits groupes amino sont éventuellement substitués avec un groupe acyle, ou 1 à 3 groupes aryle ou alkyle inférieur ;
ou bien où deux quelconques de R₁-R₁₀ peuvent former ensemble un polyéther ;
ou bien où deux quelconques de R₁-R₁₀ peuvent former un éther couronne avec les atomes de carbone intermédiaires du noyau anthracène, et
la sélection de plantes comprenant des cellules présentant une instabilité de microsatellites.

3. Procédé selon la revendication 2 où ledit inhibiteur est ajouté au milieu de croissance de ladite plante.

4. Procédé selon l'une quelconque des revendications précédentes où R₁-R₁₀ sont indépendamment hydrogène, hydroxyle, alkyle, aryle, arylalkyle, hydroxyalkyle, méthyle, éthyle, propyle, isopropyle, butyle, isobutyle, phényle, tolyle, hydroxyméthyle, hydroxypropyle ou hydroxybutyle.

5. Procédé selon l'une quelconque des revendications 1 à 3 où ledit anthracène est choisi parmi le 1,2-diméthylanthracène, le 9,10-diméthylanthracène, le 7,8-diméthylanthracène, le 9,10-diphénylanthracène, le 9,10-dihydroxyméthylanthracène, le 9-hydroxyméthyl-10-méthylanthracène, le diméthylanthracène-1,2-diol, Le 9-hydroxyméthyl-10-méthylanthracène-1,2-diol, le 9-hydroxyméthyl-10-méthylanthracène-3,4-diol et le 9,10-di-m-tolylanthracéne.

6. Procédé selon l'une quelconque des revendications 1 à 3 où R₅ et R₆ sont hydrogène.

7. Procédé selon l'une quelconque des revendications 1 à 3 où R₃, R₄, R₅, R₆, R₇, R₈, R₉ et R₁₀ sont hydrogène.

8. Procédé selon l'une quelconque des revendications 1 à 3 où R₁, R₂, R₃, R₄, R₅, R₆, R₇ et R₈ sont hydrogène.

9. Procédé selon l'une quelconque des revendications 1 à 3 où R₃, R₄, R₅, R₆, R₇ et R₈ sont hydrogène.

10. Procédé selon l'une quelconque des revendications 1 à 3 où R₁, R₂, R₃, Or R₅, R_{6,} R₉ et R₁₀ sont hydrogène.

11. Procédé selon l'une quelconque des revendications 1 à 3 où R₁, R₂, R₅, R₆, R₇ et R₈ sont hydrogène.

12. Procédé selon l'une quelconque des revendications 1 à 3 où R_{1,} R₂, R ₃, R₄, R₅, R_{6,} R₇, R₈ et R₁₀ sont hydrogène.

13. Procédé *in vitro* pour produire une mutation dans un gène d'intérêt comprenant l'exposition d'une cellule comprenant ledit gène d'intérêt à un inhibiteur de réparation des mésappariements, où ledit inhibiteur est un anthracène ayant la formule : 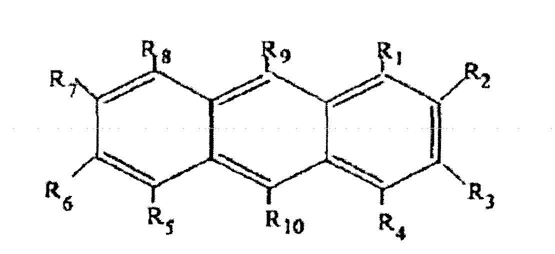 où R₁-R₁₀ sont indépendamment hydrogène, hydroxyle, amino, alkyle, alkyle substitué, alcényle, alcényle substitué, alcynyle, alcynyle substitué, O-alkyle, S-alkyle, N-alkyle, O-alcényle, S-alcényle, N-alcényle, O-alcynyle, S-alcynyle, N-alcynyle, aryle, aryle substitué, aryloxy, aryloxy substitué, hétéroaryle, hétéroaryle, substitué, aralkyloxy, arylalkyle, alkylaryle, alkylaryloxy, arylsulfonyle, alkylsulfonyle, alcoxycarbonyle ; aryloxycarbonyle, guanidino, carboxy, un alcool, un aminoacide, sulfonate, alkylsulfonate, CN, NO₂, un groupe aldéhyde, un ester, un éther, un éther couronne, une cétone, un composé organosoufré, un groupe organométallique, un acide carboxylique, un organosilicium ou un glucide qui contient éventuellement un ou plusieurs groupes hydroxyle alkylés ; où lesdits hétéroalkyle, hétéroaryle et hétéroaryle substitué contiennent au moins un hétéroatome qui est l'oxygène, le soufre, un atome métallique, le phosphore, le silicium ou l'azote ; et où lesdits substituants desdits alkyle substitué, alcényle substitué, alcynyle substitué, aryle substitué et hétéroaryle substitué sont halogène, CN, NO₂, alkyle inférieur, aryle, hétéroaryle, aralkyle, aralkyloxy, guanidino, alcoxycarbonyle, alcoxy, hydroxy, carboxy et amino ;
où lesdits groupes amino sont éventuellement substitués avec un groupe acyle, ou 1 à 3 groupes aryle ou alkyle intérieur
ou bien où deux quelconques de R₁-R₁₀ peuvent former ensemble un polyéther ;
ou bien où deux quelconques de R₁-R₁₀ peuvent former un éther couronne avec les atomes de carbone intermédiaires du noyau anthracène ;
la culture de ladite cellule comprenant ledit gène d'intérêt ;
la sélection de cellules comprenant ledit gène d'intérêt et présentant une instabilité de microsatellites et le test desdites cellules pour déterminer si ledit gène d'intérêt comprend une mutation.

14. Procédé selon la revendication 13 où ledit test comprend l'analyse d'une séquence polynucléotidique dudit gène d'intérêt.

15. Procédé selon la revendication 13 ou 14 où ladite cellule est une cellule de mammifère.

16. Procédé selon l'une quelconque des revendications 13 à 15 incluant l'exposition de ladite cellule à un mutagène.

17. Procédé selon la revendication 16 où ledit mutagène est choisi parmi la N-méthyl-N'-nitro-N-nitrosoguanïdine, le méthanesulfonate, le diméthylsulfonate, la O-6-méthylbenzadine, le méthanesulfonate d'éthyle, la méthylnitrosourée et l'éthylnitrosourée.

18. Procédé selon l'une quelconque des revendications 13 à 17 incluant l'étape de retrait dudit inhibiteur de réparation des mésappariements après le test de ladite cellule pour déterminer si ledit gène d'intérêt comprend une mutation.

19. Procédé selon l'une quelconque des revendications 1 à 3 ou 13 à 18, où ledit anthracène est choisi parmi le 1,2-diméthylanthracène, le 9,10-diméthylanthracène, le 7,8-diméthylanthracène, le 9,10-diphénylanthracène, le 9,10-dihydroxyméthylanthracène, le 9-hydroxyméthyl-10-méthylanthracène, le diméthytanthracène-1,2-diol, le 9-hydroxyméthyl-10-méthylanthracène-1,2-diol, le 9-hydroxyméthyl-10-méthylanthracène-3,4-diol et le 9,10-di-m-tolylanthracène.

20. Procédé selon l'une quelconque des revendications précédentes comprenant en outre le retrait dudit inhibiteur de réparation des mésappariements.


### SEQUENCE LISTING

<110> Nicolaides, Nicholas C
   Grasso, Luigi
   Sass, Philip M
<120> CHEMICAL INHIBITORS OF MISMATCH REPAIR
<130> MOR-0018
<140> 00/000,000
   <141> 2001-01-15
<160> 44
<170> PatentIn Ver. 2.1
<210> 1
   <211> 52
   <212> DNA
   <213> Artificial Sequence
<220>
   <223> Description of Artificial Sequence: oligonucleotide primer
<400> 1
   ggatcctaat acgactcact atagggagac caccatgtcg ttcgtggcag gg 52
<210> 2
   <211> 21
   <212> DNA
   <213> Artificial Sequence
<220>
   <223> Description of Artificial Sequence:oligonucleotide primer
<400> 2
   taagtcttaa gtgctaccaa c 21
<210> 3
   <211> 53
   <212> DNA
   <213> Artificial Sequence
<220>
   <223> Description of Artificial Sequence:oligonucleotide primer
<400> 3
   ggatcctaat acgactcact atagggagac caccatggaa caattgcctg cgg 53
<210> 4
   <211> 20
   <212> DNA
   <213> Artificial Sequence
<220>
   <223> Description of Artificial Sequence:oligonucleotide primer
<400> 4
   aggttagtga agactctgtc 20
<210> 5
   <211> 22
   <212> DNA
   <213> Artificial Sequence
<220>
   <223> Description of Artificial Sequence:oligonucleotide primer
<400> 5
   ctgatctcac ggacaatagt gc 22
<210> 6
   <211> 20
   <212> DNA
   <213> Artificial Sequence
<220>
   <223> Description of Artificial Sequence:oligonucleotide primer
<400> 6
   ggctccataa aaagtgcacc 20
<210> 7
   <211> 22
   <212> DNA
   <213> Artificial Sequence
<220>
   <223> Description of Artificial Sequence:oligonucleotide primer
<400> 7
   ggtctgttga tgtcgtaagt cg 22
<210> 8
   <211> 22
   <212> DNA
   <213> Artificial Sequence
<220>
   <223> Description of Artificial Sequence:oligonucleotide primer
<400> 8
   atcttgaaac ctttagggag gg 22
<210> 9
   <211> 18
   <212> DNA
   <213> Artificial Sequence
<220>
   <223> Description of Artificial Sequence:oligonucleotide primer
<400> 9
   agaagtttag acaggtac 18
<210> 10
   <211> 18
   <212> DNA
   <213> Artificial Sequence
<220>
   <223> Description of Artificial Sequence:oligonucleotide primer
<400> 10
   aaatgtgcaa ttgccttc 18
<210> 11
   <211> 51
   <212> DNA
   <213> Artificial Sequence
<220>
   <223> Description of Artificial Sequence:oligonucleotide primer
<400> 11
   cccggatcca tgttaaaaaa aaaaaaaaaa aaaaaacgtc ctgtagaaac c 51
<210> 12
   <211> 48
   <212> DNA
   <213> Artificial Sequence
<220>
   <223> Description of Artificial Sequence:oligonucleotide primer
<400> 12
   cccggatcca tgttaaaaaa aaaaaaaaaa aaaacgtcct gtagaaac 48
<210> 13
   <211> 30
   <212> DNA
   <213> Artificial Sequence
<220>
   <223> Description of Artificial Sequence:oligonucleotide primer
<400> 13
   cccgaattcc ccgatctagt aacatagatg 30
<210> 14
   <211> 859
   <212> PRT
   <213> Mus musculus
<400> 14
   Met Glu Gln Thr Glu Gly Val Ser Thr Glu Cys Ala Lys Ala Ile Lys
    1               5                  10                  15
   Pro Ile Asp Gly Lys Ser Val His Gln Ile Cys Ser Gly Gln Val Ile
               20                  25                  30
   Leu Ser Leu Ser Thr Ala Val Lys Glu Leu Ile Glu Asn Ser Val Asp
           35                  40                  45
   Ala Gly Ala Thr Thr Ile Asp Leu Arg Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Ala Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Gly Ser
           115                 120                 125
   Ala Ser Val Gly Thr Arg Leu Val Phe Asp His Asn Gly Lys Ile Thr
       130                 135                 140
   Gln Lys Thr Pro Tyr Pro Arg Pro Lys Gly Thr Thr Val Ser Val Gln
   145                 150                 155                 160
   His Leu Phe Tyr Thr Leu Pro Val Arg Tyr Lys Glu Phe Gln Arg Asn
                   165                 170                 175
   Ile Lys Lys Glu Tyr Ser Lys Met Val Gln Val Leu Gln Ala Tyr Cys
               180                 185                 190
   Ile Ile Ser Ala Gly Val Arg Val Ser Cys Thr Asn Gln Leu Gly Gln
           195                 200                 205
   Gly Lys Arg His Ala Val Val Cys Thr Ser Gly Thr Ser Gly Met Lys
       210                 215                 220
   Glu Asn Ile Gly Ser Val Phe Gly Gln Lys Gln Leu Gln Ser Leu Ile
   225                 230                 235                 240
   Pro Phe Val Gln Leu Pro Pro Ser Asp Ala Val Cys Glu Glu Tyr Gly
                   245                 250                 255
   Leu Ser Thr Ser Gly Arg His Lys Thr Phe Ser Thr Phe Arg Ala Ser
               260                 265                 270
   Phe His Ser Ala Arg Thr Ala Pro Gly Gly Val Gln Gln Thr Gly Ser
           275                 280                 285
   Phe Ser Ser Ser Ile Arg Gly Pro Val Thr Gln Gln Arg Ser Leu Ser
       290                 295                 300
   Leu Ser Met Arg Phe Tyr His Met Tyr Asn Arg His Gln Tyr Pro Phe
   305                 310                 315                 320
   Val Val Leu Asn Val Ser Val Asp Ser Glu Cys Val Asp Ile Asn Val
                   325                 330                 335
   Thr Pro Asp Lys Arg Gln Ile Leu Leu Gln Glu Glu Lys Leu Leu Leu
               340                 345                 350
   Ala Val Leu Lys Thr Ser Leu Ile Gly Met Phe Asp Ser Asp Ala Asn
           355                 360                 365
   Lys Leu Asn Val Asn Gln Gln Pro Leu Leu Asp Val Glu Gly Asn Leu
       370                 375                 380
   Val Lys Leu His Thr Ala Glu Leu Glu Lys Pro Val Pro Gly Lys Gln
   385                 390                 395                 400
   Asp Asn Ser Pro Ser Leu Lys Ser Thr Ala Asp Glu Lys Arg Val Ala
                   405                 410                 415
   Ser Ile Ser Arg Leu Arg Glu Ala Phe Ser Leu His Pro Thr Lys Glu
               420                 425                 430
   Ile Lys Ser Arg Gly Pro Glu Thr Ala Glu Leu Thr Arg Ser Phe Pro
           435                 440                 445
   Ser Glu Lys Arg Gly Val Leu Ser Ser Tyr Pro Ser Asp Val Ile Ser
       450                 455                 460
   Tyr Arg Gly Leu Arg Gly Ser Gln Asp Lys Leu Val Ser Pro Thr Asp
   465                 470                 475                 480
   Ser Pro Gly Asp Cys Met Asp Arg Glu Lys Ile Glu Lys Asp Ser Gly
                   485                 490                 495
   Leu Ser Ser Thr Ser Ala Gly Ser Glu Glu Glu Phe Ser Thr Pro Glu
               500                 505                 510
   Val Ala Ser Ser Phe Ser Ser Asp Tyr Asn Val Ser Ser Leu Glu Asp
           515                     520                     525
   Arg Pro Ser Gln Glu Thr Ile Asn Cys Gly Asp Leu Asp Cys Arg Pro
       530                     535                     540
   Pro Gly Thr Gly Gln Ser Leu Lys Pro Glu Asp His Gly Tyr Gln Cys
   545                     550                     555                     560
   Lys Ala Leu Pro Leu Ala Arg Leu Ser Pro Thr Asn Ala Lys Arg Phe
                   565                     570                     575
   Lys Thr Glu Glu Arg Pro Ser Asn Val Asn Ile Ser Gln Arg Leu Pro
               580                     585                     590
   Gly Pro Gln Ser Thr Ser Ala Ala Glu Val Asp Val Ala Ile Lys Met
           595                     600                     605
   Asn Lys Arg Ile Val Leu Leu Glu Phe Ser Leu Ser Ser Leu Ala Lys
       610                     615                     620
   Arg Met Lys Gln Leu Gln His Leu Lys Ala Gln Asn Lys His Glu Leu
   625                     630                     635                     640
   Ser Tyr Arg Lys Phe Arg Ala Lys Ile Cys Pro Gly Glu Asn Gln Ala
                   645                     650                     655
   Ala Glu Asp Glu Leu Arg Lys Glu Ile Ser Lys Ser Met Phe Ala Glu
               660                     665                     670
   Met Glu Ile Leu Gly Gln Phe Asn Leu Gly Phe Ile Val Thr Lys Leu
           675                     680                     685
   Lys Glu Asp Leu Phe Leu Val Asp Gln His Ala Ala Asp Glu Lys Tyr
       690                     695                     700
   Asn Phe Glu Met Leu Gln Gln His Thr Val Leu Gln Ala Gln Arg Leu
   705                     710                     715                     720
   Ile Thr Pro Gln Thr Leu Asn Leu Thr Ala Val Asn Glu Ala Val Leu
                   725                     730                     735
   Ile Glu Asn Leu Glu Ile Phe Arg Lys Asn Gly Phe Asp Phe Val Ile
               740                     745                     750
   Asp Glu Asp Ala Pro Val Thr Glu Arg Ala Lys Leu Ile Ser Leu Pro
           755                     760                     765
   Thr Ser Lys Asn Trp Thr Phe Gly Pro Gln Asp Ile Asp Glu Leu Ile
       770                 775                 780
   Phe Met Leu Ser Asp Ser Pro Gly Val Met Cys Arg Pro Ser Arg Val
   785                 790                 795                 800
   Arg Gln Met Phe Ala Ser Arg Ala Cys Arg Lys Ser Val Met Ile Gly
                   805                 810                 815
   Thr Ala Leu Asn Ala Ser Glu Met Lys Lys Leu Ile Thr His Met Gly
               820                 825                 830
   Glu Met Asp His Pro Trp Asn Cys Pro His Gly Arg Pro Thr Met Arg
           835                 840                 845
   His Val Ala Asn Leu Asp Val Ile Ser Gln Asn
       850                 855
<210> 15
   <211> 3056
   <212> DNA
   <213> Mus musculus
<400> 15
   gaattccggt gaaggtcctg aagaatttcc agattcctga gtatcattgg aggagacaga 60
   taacctgtcg tcaggtaacg atggtgtata tgcaacagaa atgggtgttc ctggagacgc 120
   gtcttttccc gagagcggca ccgcaactct cccgcggtga ctgtgactgg aggagtcctg 180
   catccatgga gcaaaccgaa ggcgtgagta cagaatgtgc taaggccatc aagcctattg 240
   atgggaagtc agtccatcaa atttgttctg ggcaggtgat actcagttta agcaccgctg 300
   tgaaggagtt gatagaaaat agtgtagatg ctggtgctac tactattgat ctaaggctta 360
   aagactatgg ggtggacctc attgaagttt cagacaatgg atgtggggta gaagaagaaa 420
   actttgaagg tctagctctg aaacatcaca catctaagat tcaagagttt gccgacctca 480
   cgcaggttga aactttcggc tttcgggggg aagctctgag ctctctgtgt gcactaagtg 540
   atgtcactat atctacctgc cacgggtctg caagcgttgg gactcgactg gtgtttgacc 600
   ataatgggaa aatcacccag aaaactccct acccccgacc taaaggaacc acagtcagtg 660
   tgcagcactt attttataca ctacccgtgc gttacaaaga gtttcagagg aacattaaaa 720
   aggagtattc caaaatggtg caggtcttac aggcgtactg tatcatctca gcaggcgtcc 780
   gtgtaagctg cactaatcag ctcggacagg ggaagcggca cgctgtggtg tgcacaagcg 840
   gcacgtctgg catgaaggaa aatatcgggt ctgtgtttgg ccagaagcag ttgcaaagcc 900
   tcattccttt tgttcagctg ccccctagtg acgctgtgtg tgaagagtac ggcctgagca 960
   cttcaggacg ccacaaaacc ttttctacgt ttcgggcttc atttcacagt gcacgcacgg 1020
   cgccgggagg agtgcaacag acaggcagtt tttcttcatc aatcagaggc cctgtgaccc 1080
   agcaaaggtc tctaagcttg tcaatgaggt tttatcacat gtataaccgg catcagtacc 1140
   catttgtcgt ccttaacgtt tccgttgact cagaatgtgt ggatattaat gtaactccag 1200
   ataaaaggca aattctacta caagaagaga agctattgct ggccgtttta aagacctcct 1260
   tgataggaat gtttgacagt gatgcaaaca agcttaatgt caaccagcag ccactgctag 1320
   atgttgaagg taacttagta aagctgcata ctgcagaact agaaaagcct gtgccaggaa 1380
   agcaagataa ctctccttca ctgaagagca cagcagacga gaaaagggta gcatccatct 1440
   ccaggctgag agaggccttt tctcttcatc ctactaaaga gatcaagtct aggggtccag 1500
   agactgctga actgacacgg agttttccaa gtgagaaaag gggcgtgtta tcctcttatc 1560
   cttcagacgt catctcttac agaggcctcc gtggctcgca ggacaaattg gtgagtccca 1620
   cggacagccc tggtgactgt atggacagag agaaaataga aaaagactca gggctcagca 1680
   gcacctcagc tggctctgag gaagagttca gcaccccaga agtggccagt agctttagca 1740
   gtgactataa cgtgagctcc ctagaagaca gaccttctca ggaaaccata aactgtggtg 1800
   acctggactg ccgtcctcca ggtacaggac agtccttgaa gccagaagac catggatatc 1860
   aatgcaaagc tctacctcta gctcgtctgt cacccacaaa tgccaagcgc ttcaagacag 1920
   aggaaagacc ctcaaatgtc aacatttctc aaagattgcc tggtcctcag agcacctcag 1980
   cagctgaggt cgatgtagcc ataaaaatga ataagagaat cgtgctcctc gagttctctc 2040
   tgagttctct agctaagcga atgaagcagt tacagcacct aaaggcgcag aacaaacatg 2100
   aactgagtta cagaaaattt agggccaaga tttgccctgg agaaaaccaa gcagcagaag 2160
   atgaactcag aaaagagatt agtaaatcga tgtttgcaga gatggagatc ttgggtcagt 2220
   ttaacctggg atttatagta accaaactga aagaggacct cttcctggtg gaccagcatg 2280
   ctgcggatga gaagtacaac tttgagatgc tgcagcagca cacggtgctc caggcgcaga 2340
   ggctcatcac accccagact ctgaacttaa ctgctgtcaa tgaagctgta ctgatagaaa 2400
   atctggaaat attcagaaag aatggctttg actttgtcat tgatgaggat gctccagtca 2460
   ctgaaagggc taaattgatt tccttaccaa ctagtaaaaa ctggaccttt ggaccccaag 2520
   atatagatga actgatcttt atgttaagtg acagccctgg ggtcatgtgc cggccctcac 2580
   gagtcagaca gatgtttgct tccagagcct gtcggaagtc agtgatgatt ggaacggcgc 2640
   tcaatgcgag cgagatgaag aagctcatca cccacatggg tgagatggac cacccctgga 2700
   actgccccca cggcaggcca accatgaggc acgttgccaa tctggatgtc atctctcaga 2760
   actgacacac cccttgtagc atagagttta ttacagattg ttcggtttgc aaagagaagg 2820
   ttttaagtaa tctgattatc gttgtacaaa aattagcatg ctgctttaat gtactggatc 2880
   catttaaaag cagtgttaag gcaggcatga tggagtgttc ctctagctca gctacttggg 2940
   tgatccggtg ggagctcatg tgagcccagg actttgagac cactccgagc cacattcatg 3000
   agactcaatt caaggacaaa aaaaaaaaga tatttttgaa gccttttaaa aaaaaa     3056
<210> 16
   <211> 862
   <212> PRT
   <213> Homo sapiens
<400> 16
   Met Glu Arg Ala Glu Ser Ser Ser Thr Glu Pro Ala Lys Ala Ile Lys
   1               5                   10                  15
   Pro Ile Asp Arg Lys Ser Val His Gln Ile Cys Ser Gly Gln Val Val
               20                  25                  30
   Leu Ser Leu Ser Thr Ala Val Lys Glu Leu Val Glu Asn Ser Leu Asp
           35                  40                  45
   Ala Gly Ala Thr Asn Ile Asp Leu Lys Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Thr Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Ala Ser
           115                 120                 125
   Ala Lys Val Gly Thr Arg Leu Met Phe Asp His Asn Gly Lys Ile Ile
       130                 135                 140
   Gln Lys Thr Pro Tyr Pro Arg Pro Arg Gly Thr Thr Val Ser Val Gln
   145                 150                 155                 160
   Gln Leu Phe Ser Thr Leu Pro Val Arg His Lys Glu Phe Gln Arg Asn
                   165                 170                 175
   Ile Lys Lys Glu Tyr Ala Lys Met Val Gln Val Leu His Ala Tyr Cys
               180                 185                 190
   Ile Ile Ser Ala Gly Ile Arg Val Ser Cys Thr Asn Gln Leu Gly Gln
           195                 200                 205
   Gly Lys Arg Gln Pro Val Val Cys Thr Gly Gly Ser Pro Ser Ile Lys
       210                 215                 220
   Glu Asn Ile Gly Ser Val Phe Gly Gln Lys Gln Leu Gln Ser Leu Ile
   225                 230                 235                 240
   Pro Phe Val Gln Leu Pro Pro Ser Asp Ser Val Cys Glu Glu Tyr Gly
                   245                 250                 255
   Leu Ser Cys Ser Asp Ala Leu His Asn Leu Phe Tyr Ile Ser Gly Phe
               260                 265                 270
   Ile Ser Gln Cys Thr His Gly Val Gly Arg Ser Ser Thr Asp Arg Gln
           275                 280                 285
   Phe Phe Phe Ile Asn Arg Arg Pro Cys Asp Pro Ala Lys Val Cys Arg
       290                 295                 300
   Leu Val Asn Glu Val Tyr His Met Tyr Asn Arg His Gln Tyr Pro Phe
   305                 310                 315                 320
   Val Val Leu Asn Ile Ser Val Asp Ser Glu Cys Val Asp Ile Asn Val
                   325                 330                 335
   Thr Pro Asp Lys Arg Gln Ile Leu Leu Gln Glu Glu Lys Leu Leu Leu
               340                 345                 350
   Ala Val Leu Lys Thr Ser Leu Ile Gly Met Phe Asp Ser Asp Val Asn
           355                 360                 365
   Lys Leu Asn Val Ser Gln Gln Pro Leu Leu Asp Val Glu Gly Asn Leu
       370                 375                 380
   Ile Lys Met His Ala Ala Asp Leu Glu Lys Pro Met Val Glu Lys Gln
   385                 390                 395                 400
   Asp Gln Ser Pro Ser Leu Arg Thr Gly Glu Glu Lys Lys Asp Val Ser
                   405                 410                 415
   Ile Ser Arg Leu Arg Glu Ala Phe Ser Leu Arg His Thr Thr Glu Asn
               420                 425                 430
   Lys Pro His Ser Pro Lys Thr Pro Glu Pro Arg Arg Ser Pro Leu Gly
           435                 440                 445
   Gln Lys Arg Gly Met Leu Ser Ser Ser Thr Ser Gly Ala Ile Ser Asp
       450                 455                 460
   Lys Gly Val Leu Arg Pro Gln Lys Glu Ala Val Ser Ser Ser His Gly
   465                 470                 475                 480
   Pro Ser Asp Pro Thr Asp Arg Ala Glu Val Glu Lys Asp Ser Gly His
                   485                 490                 495
   Gly Ser Thr Ser Val Asp Ser Glu Gly Phe Ser Ile Pro Asp Thr Gly
               500                 505                 510
   Ser His Cys Ser Ser Glu Tyr Ala Ala Ser Ser Pro Gly Asp Arg Gly
           515                 520                 525
   Ser Gln Glu His Val Asp Ser Gln Glu Lys Ala Pro Glu Thr Asp Asp
       530                 535                 540
   Ser Phe Ser Asp Val Asp Cys His Ser Asn Gln Glu Asp Thr Gly Cys
   545                 550                 555                 560
   Lys Phe Arg Val Leu Pro Gln Pro Thr Asn Leu Ala Thr Pro Asn Thr
                   565                 570                 575
   Lys Arg Phe Lys Lys Glu Glu Ile Leu Ser Ser Ser Asp Ile Cys Gln
                   580                     585                 590
   Lys Leu Val Asn Thr Gln Asp Met Ser Ala Ser Gln Val Asp Val Ala
               595                     600                 605
   Val Lys Ile Asn Lys Lys Val Val Pro Leu Asp Phe Ser Met Ser Ser
       610                     615                 620
   Leu Ala Lys Arg Ile Lys Gln Leu His His Glu Ala Gln Gln Ser Glu
   625                     630                 635                     640
   Gly Glu Gln Asn Tyr Arg Lys Phe Arg Ala Lys Ile Cys Pro Gly Glu
                   645                     650                     655
   Asn Gln Ala Ala Glu Asp Glu Leu Arg Lys Glu Ile Ser Lys Thr Met
                   660                     665                 670
   Phe Ala Glu Met Glu Ile Ile Gly Gln Phe Asn Leu Gly Phe Ile Ile
               675                     680                 685
   Thr Lys Leu Asn Glu Asp Ile Phe Ile Val Asp Gln His Ala Thr Asp
       690                     695                 700
   Glu Lys Tyr Asn Phe Glu Met Leu Gln Gln His Thr Val Leu Gln Gly
   705                     710                 715                     720
   Gln Arg Leu Ile Ala Pro Gln Thr Leu Asn Leu Thr Ala Val Asn Glu
                   725                     730                     735
   Ala Val Leu Ile Glu Asn Leu Glu Ile Phe Arg Lys Asn Gly Phe Asp
                   740                     745                 750
   Phe Val Ile Asp Glu Asn Ala Pro Val Thr Glu Arg Ala Lys Leu Ile
               755                     760                 765
   Ser Leu Pro Thr Ser Lys Asn Trp Thr Phe Gly Pro Gln Asp Val Asp
       770                     775                 780
   Glu Leu Ile Phe Met Leu Ser Asp Ser Pro Gly Val Met Cys Arg Pro
   785                     790                 795                     800
   Ser Arg Val Lys Gln Met Phe Ala Ser Arg Ala Cys Arg Lys Ser Val
                   805                     810                 815
   Met Ile Gly Thr Ala Leu Asn Thr Ser Glu Met Lys Lys Leu Ile Thr
                   820                     825                 830
   His Met Gly Glu Met Asp His Pro Trp Asn Cys Pro His Gly Arg Pro
           835                 840                 845
   Thr Met Arg His Ile Ala Asn Leu Gly Val Ile Ser Gln Asn
       850                 855                 860
<210> 17
   <211> 2771
   <212> DNA
   <213> Homo sapiens
<400> 17
   cgaggcggat cgggtgttgc atccatggag cgagctgaga gctcgagtac agaacctgct 60
   aaggccatca aacctattga tcggaagtca gtccatcaga tttgctctgg gcaggtggta 120
   ctgagtctaa gcactgcggt aaaggagtta gtagaaaaca gtctggatgc tggtgccact 180
   aatattgatc taaagcttaa ggactatgga gtggatctta ttgaagtttc agacaatgga 240
   tgtggggtag aagaagaaaa cttcgaaggc ttaactctga aacatcacac atctaagatt 300
   caagagtttg ccgacctaac tcaggttgaa acttttggct ttcgggggga agctctgagc 360
   tcactttgtg cactgagcga tgtcaccatt tctacctgcc acgcatcggc gaaggttgga 420
   actcgactga tgtttgatca caatgggaaa attatccaga aaacccccta cccccgcccc 480
   agagggacca cagtcagcgt gcagcagtta ttttccacac tacctgtgcg ccataaggaa 540
   tttcaaagga atattaagaa ggagtatgcc aaaatggtcc aggtcttaca tgcatactgt 600
   atcatttcag caggcatccg tgtaagttgc accaatcagc ttggacaagg aaaacgacag 660
   cctgtggtat gcacaggtgg aagccccagc ataaaggaaa atatcggctc tgtgtttggg 720
   cagaagcagt tgcaaagcct cattcctttt gttcagctgc cccctagtga ctccgtgtgt 780
   gaagagtacg gtttgagctg ttcggatgct ctgcataatc ttttttacat ctcaggtttc 840
   atttcacaat gcacgcatgg agttggaagg agttcaacag acagacagtt tttctttatc 900
   aaccggcggc cttgtgaccc agcaaaggtc tgcagactcg tgaatgaggt ctaccacatg 960
   tataatcgac accagtatcc atttgttgtt cttaacattt ctgttgattc agaatgcgtt 1020
   gatatcaatg ttactccaga taaaaggcaa attttgctac aagaggaaaa gcttttgttg 1080
   gcagttttaa agacctcttt gataggaatg tttgatagtg atgtcaacaa gctaaatgtc 1140
   agtcagcagc cactgctgga tgttgaaggt aacttaataa aaatgcatgc agcggatttg 1200
   gaaaagccca tggtagaaaa gcaggatcaa tccccttcat taaggactgg agaagaaaaa 1260
   aaagacgtgt ccatttccag actgcgagag gccttttctc ttcgtcacac aacagagaac 1320
   aagcctcaca gcccaaagac tccagaacca agaaggagcc ctctaggaca gaaaaggggt 1380
   atgctgtctt ctagcacttc aggtgccatc tctgacaaag gcgtcctgag acctcagaaa 1440
   gaggcagtga gttccagtca cggacccagt gaccctacgg acagagcgga ggtggagaag 1500
   gactcggggc acggcagcac ttccgtggat tctgaggggt tcagcatccc agacacgggc 1560
   agtcactgca gcagcgagta tgcggccagc tccccagggg acaggggctc gcaggaacat 1620
   gtggactctc aggagaaagc gcctgaaact gacgactctt tttcagatgt ggactgccat 1680
   tcaaaccagg aagataccgg atgtaaattt cgagttttgc ctcagccaac taatctcgca 1740
   accccaaaca caaagcgttt taaaaaagaa gaaattcttt ccagttctga catttgtcaa 1800
   aagttagtaa atactcagga catgtcagcc tctcaggttg atgtagctgt gaaaattaat 1860
   aagaaagttg tgcccctgga cttttctatg agttctttag ctaaacgaat aaagcagtta 1920
   catcatgaag cacagcaaag tgaaggggaa cagaattaca ggaagtttag ggcaaagatt 1980
   tgtcctggag aaaatcaagc agccgaagat gaactaagaa aagagataag taaaacgatg 2040
   tttgcagaaa tggaaatcat tggtcagttt aacctgggat ttataataac caaactgaat 2100
   gaggatatct tcatagtgga ccagcatgcc acggacgaga agtataactt cgagatgctg 2160
   cagcagcaca ccgtgctcca ggggcagagg ctcatagcac ctcagactct caacttaact 2220
   gctgttaatg aagctgttct gatagaaaat ctggaaatat ttagaaagaa tggctttgat 2280
   tttgttatcg atgaaaatgc tccagtcact gaaagggcta aactgatttc cttgccaact 2340
   agtaaaaact ggaccttcgg accccaggac gtcgatgaac tgatcttcat gctgagcgac 2400
   agccctgggg tcatgtgccg gccttcccga gtcaagcaga tgtttgcctc cagagcctgc 2460
   cggaagtcgg tgatgattgg gactgctctt aacacaagcg agatgaagaa actgatcacc 2520
   cacatggggg agatggacca cccctggaac tgtccccatg gaaggccaac catgagacac 2580
   atcgccaacc tgggtgtcat ttctcagaac tgaccgtagt cactgtatgg aataattggt 2640
   tttatcgcag atttttatgt tttgaaagac agagtcttca ctaacctttt ttgttttaaa 2700
   atgaaacctg ctacttaaaa aaaatacaca tcacacccat ttaaaagtga tcttgagaac 2760
   cttttcaaac c                                                      2771
<210> 18
   <211> 932
   <212> PRT
   <213> Homo sapiens
<400> 18
<210> 19
   <211> 3063
   <212> DNA
   <213> Homo sapiens
<400> 19
   ggcacgagtg gctgcttgcg gctagtggat ggtaattgcc tgcctcgcgc tagcagcaag 60
   ctgctctgtt aaaagcgaaa atgaaacaat tgcctgcggc aacagttcga ctcctttcaa 120
   gttctcagat catcacttcg gtggtcagtg ttgtaaaaga gcttattgaa aactccttgg 180
   atgctggtgc cacaagcgta gatgttaaac tggagaacta tggatttgat aaaattgagg 240
   tgcgagataa cggggagggt atcaaggctg ttgatgcacc tgtaatggca atgaagtact 300
   acacctcaaa aataaatagt catgaagatc ttgaaaattt gacaacttac ggttttcgtg 360
   gagaagcctt ggggtcaatt tgttgtatag ctgaggtttt aattacaaca agaacggctg 420
   ctgataattt tagcacccag tatgttttag atggcagtgg ccacatactt tctcagaaac 480
   cttcacatct tggtcaaggt acaactgtaa ctgctttaag attatttaag aatctacctg 540
   taagaaagca gttttactca actgcaaaaa aatgtaaaga tgaaataaaa aagatccaag 600
   atctcctcat gagctttggt atccttaaac ctgacttaag gattgtcttt gtacataaca 660
   aggcagttat ttggcagaaa agcagagtat cagatcacaa gatggctctc atgtcagttc 720
   tggggactgc tgttatgaac aatatggaat cctttcagta ccactctgaa gaatctcaga 780
   tttatctcag tggatttctt ccaaagtgtg atgcagacca ctctttcact agtctttcaa 840
   caccagaaag aagtttcatc ttcataaaca gtcgaccagt acatcaaaaa gatatcttaa 900
   agttaatccg acatcattac aatctgaaat gcctaaagga atctactcgt ttgtatcctg 960
   ttttctttct gaaaatcgat gttcctacag ctgatgttga tgtaaattta acaccagata 1020
   aaagccaagt attattacaa aataaggaat ctgttttaat tgctcttgaa aatctgatga 1080
   cgacttgtta tggaccatta cctagtacaa attcttatga aaataataaa acagatgttt 1140
   ccgcagctga catcgttctt agtaaaacag cagaaacaga tgtgcttttt aataaagtgg 1200
   aatcatctgg aaagaattat tcaaatgttg atacttcagt cattccattc caaaatgata 1260
   tgcataatga tgaatctgga aaaaacactg atgattgttt aaatcaccag ataagtattg 1320
   gtgactttgg ttatggtcat tgtagtagtg aaatttctaa cattgataaa aacactaaga 1380
   atgcatttca ggacatttca atgagtaatg tatcatggga gaactctcag acggaatata 1440
   gtaaaacttg ttttataagt tccgttaagc acacccagtc agaaaatggc aataaagacc 1500
   atatagatga gagtggggaa aatgaggaag aagcaggtct tgaaaactct tcggaaattt 1560
   ctgcagatga gtggagcagg ggaaatatac ttaaaaattc agtgggagag aatattgaac 1620
   ctgtgaaaat tttagtgcct gaaaaaagtt taccatgtaa agtaagtaat aataattatc 1680
   caatccctga acaaatgaat cttaatgaag attcatgtaa caaaaaatca aatgtaatag 1740
   ataataaatc tggaaaagtt acagcttatg atttacttag caatcgagta atcaagaaac 1800
   ccatgtcagc aagtgctctt tttgttcaag atcatcgtcc tcagtttctc atagaaaatc 1860
   ctaagactag tttagaggat gcaacactac aaattgaaga actgtggaag acattgagtg 1920
   aagaggaaaa actgaaatat gaagagaagg ctactaaaga cttggaacga tacaatagtc 1980
   aaatgaagag agccattgaa caggagtcac aaatgtcact aaaagatggc agaaaaaaga 2040
   taaaacccac cagcgcatgg aatttggccc agaagcacaa gttaaaaacc tcattatcta 2100
   atcaaccaaa acttgatgaa ctccttcagt cccaaattga aaaaagaagg agtcaaaata 2160
   ttaaaatggt acagatcccc ttttctatga aaaacttaaa aataaatttt aagaaacaaa 2220
   acaaagttga cttagaagag aaggatgaac cttgcttgat ccacaatctc aggtttcctg 2280
   atgcatggct aatgacatcc aaaacagagg taatgttatt aaatccatat agagtagaag 2340
   aagccctgct atttaaaaga cttcttgaga atcataaact tcctgcagag ccactggaaa 2400
   agccaattat gttaacagag agtcttttta atggatctca ttatttagac gttttatata 2460
   aaatgacagc agatgaccaa agatacagtg gatcaactta cctgtctgat cctcgtctta 2520
   cagcgaatgg tttcaagata aaattgatac caggagtttc aattactgaa aattacttgg 2580
   aaatagaagg aatggctaat tgtctcccat tctatggagt agcagattta aaagaaattc 2640
   ttaatgctat attaaacaga aatgcaaagg aagtttatga atgtagacct cgcaaagtga 2700
   taagttattt agagggagaa gcagtgcgtc tatccagaca attacccatg tacttatcaa 2760
   aagaggacat ccaagacatt atctacagaa tgaagcacca gtttggaaat gaaattaaag 2820
   agtgtgttca tggtcgccca ttttttcatc atttaaccta tcttccagaa actacatgat 2880
   taaatatgtt taagaagatt agttaccatt gaaattggtt ctgtcataaa acagcatgag 2940
   tctggtttta aattatcttt gtattatgtg tcacatggtt attttttaaa tgaggattca 3000
   ctgacttgtt tttatattga aaaaagttcc acgtattgta gaaaacgtaa ataaactaat 3060
   aac                                                               3063
<210> 20
   <211> 934
   <212> PRT
   <213> Homo sapiens
<400> 20
<210> 21
   <211> 3145
   <212> DNA
   <213> Homo sapiens
<400> 21
   ggcgggaaac agcttagtgg gtgtggggtc gcgcattttc ttcaaccagg aggtgaggag 60
   gtttcgacat ggcggtgcag ccgaaggaga cgctgcagtt ggagagcgcg gccgaggtcg 120
   gcttcgtgcg cttctttcag ggcatgccgg agaagccgac caccacagtg cgccttttcg 180
   accggggcga cttctatacg gcgcacggcg aggacgcgct gctggccgcc cgggaggtgt 240
   tcaagaccca gggggtgatc aagtacatgg ggccggcagg agcaaagaat ctgcagagtg 300
   ttgtgcttag taaaatgaat tttgaatctt ttgtaaaaga tcttcttctg gttcgtcagt 360
   atagagttga agtttataag aatagagctg gaaataaggc atccaaggag aatgattggt 420
   atttggcata taaggcttct cctggcaatc tctctcagtt tgaagacatt ctctttggta 480
   acaatgatat gtcagcttcc attggtgttg tgggtgttaa aatgtccgca gttgatggcc 540
   agagacaggt tggagttggg tatgtggatt ccatacagag gaaactagga ctgtgtgaat 600
   tccctgataa tgatcagttc tccaatcttg aggctctcct catccagatt ggaccaaagg 660
   aatgtgtttt acccggagga gagactgctg gagacatggg gaaactgaga cagataattc 720
   aaagaggagg aattctgatc acagaaagaa aaaaagctga cttttccaca aaagacattt 780
   atcaggacct caaccggttg ttgaaaggca aaaagggaga gcagatgaat agtgctgtat 840
   tgccagaaat ggagaatcag gttgcagttt catcactgtc tgcggtaatc aagtttttag 900
   aactcttatc agatgattcc aactttggac agtttgaact gactactttt gacttcagcc 960
   agtatatgaa attggatatt gcagcagtca gagcccttaa cctttttcag ggttctgttg 1020
   aagataccac tggctctcag tctctggctg ccttgctgaa taagtgtaaa acccctcaag 1080
   gacaaagact tgttaaccag tggattaagc agcctctcat ggataagaac agaatagagg 1140
   agagattgaa tttagtggaa gcttttgtag aagatgcaga attgaggcag actttacaag 1200
   aagatttact tcgtcgattc ccagatctta accgacttgc caagaagttt caaagacaag 1260
   cagcaaactt acaagattgt taccgactct atcagggtat aaatcaacta cctaatgtta 1320
   tacaggctct ggaaaaacat gaaggaaaac accagaaatt attgttggca gtttttgtga 1380
   ctcctcttac tgatcttcgt tctgacttct ccaagtttca ggaaatgata gaaacaactt 1440
   tagatatgga tcaggtggaa aaccatgaat tccttgtaaa accttcattt gatcctaatc 1500
   tcagtgaatt aagagaaata atgaatgact tggaaaagaa gatgcagtca acattaataa 1560
   gtgcagccag agatcttggc ttggaccctg gcaaacagat taaactggat tccagtgcac 1620
   agtttggata ttactttcgt gtaacctgta aggaagaaaa agtccttcgt aacaataaaa 1680
   actttagtac tgtagatatc cagaagaatg gtgttaaatt taccaacagc aaattgactt 1740
   ctttaaatga agagtatacc aaaaataaaa cagaatatga agaagcccag gatgccattg 1800
   ttaaagaaat tgtcaatatt tcttcaggct atgtagaacc aatgcagaca ctcaatgatg 1860
   tgttagctca gctagatgct gttgtcagct ttgctcacgt gtcaaatgga gcacctgttc 1920
   catatgtacg accagccatt ttggagaaag gacaaggaag aattatatta aaagcatcca 1980
   ggcatgcttg tgttgaagtt caagatgaaa ttgcatttat tcctaatgac gtatactttg 2040
   aaaaagataa acagatgttc cacatcatta ctggccccaa tatgggaggt aaatcaacat 2100
   atattcgaca aactggggtg atagtactca tggcccaaat tgggtgtttt gtgccatgtg 2160
   agtcagcaga agtgtccatt gtggactgca tcttagcccg agtaggggct ggtgacagtc 2220
   aattgaaagg agtctccacg ttcatggctg aaatgttgga aactgcttct atcctcaggt 2280
   ctgcaaccaa agattcatta ataatcatag atgaattggg aagaggaact tctacctacg 2340
   atggatttgg gttagcatgg gctatatcag aatacattgc aacaaagatt ggtgcttttt 2400
   gcatgtttgc aacccatttt catgaactta ctgccttggc caatcagata ccaactgtta 2460
   ataatctaca tgtcacagca ctcaccactg aagagacctt aactatgctt tatcaggtga 2520
   agaaaggtgt ctgtgatcaa agttttggga ttcatgttgc agagcttgct aatttcccta 2580
   agcatgtaat agagtgtgct aaacagaaag ccctggaact tgaggagttt cagtatattg 2640
   gagaatcgca aggatatgat atcatggaac cagcagcaaa gaagtgctat ctggaaagag 2700
   agcaaggtga aaaaattatt caggagttcc tgtccaaggt gaaacaaatg ccctttactg 2760
   aaatgtcaga agaaaacatc acaataaagt taaaacagct aaaagctgaa gtaatagcaa 2820
   agaataatag ctttgtaaat gaaatcattt cacgaataaa agttactacg tgaaaaatcc 2880
   cagtaatgga atgaaggtaa tattgataag ctattgtctg taatagtttt atattgtttt 2940
   atattaaccc tttttccata gtgttaactg tcagtgccca tgggctatca acttaataag 3000
   atatttagta atattttact ttgaggacat tttcaaagat ttttattttg aaaaatgaga 3060
   gctgtaactg aggactgttt gcaattgaca taggcaataa taagtgatgt gctgaatttt 3120
   ataaataaaa tcatgtagtt tgtgg                                       3145
<210> 22
   <211> 756
   <212> PRT
   <213> Homo sapiens
<400> 22
<210> 23
   <211> 2484
   <212> DNA
   <213> Homo sapiens
<400> 23
   cttggctctt ctggcgccaa aatgtcgttc gtggcagggg ttattcggcg gctggacgag 60
   acagtggtga accgcatcgc ggcgggggaa gttatccagc ggccagctaa tgctatcaaa 120
   gagatgattg agaactgttt agatgcaaaa tccacaagta ttcaagtgat tgttaaagag 180
   ggaggcctga agttgattca gatccaagac aatggcaccg ggatcaggaa agaagatctg 240
   gatattgtat gtgaaaggtt cactactagt aaactgcagt cctttgagga tttagccagt 300
   atttctacct atggctttcg aggtgaggct ttggccagca taagccatgt ggctcatgtt 360
   actattacaa cgaaaacagc tgatggaaag tgtgcataca gagcaagtta ctcagatgga 420
   aaactgaaag cccctcctaa accatgtgct ggcaatcaag ggacccagat cacggtggag 480
   gacctttttt acaacatagc cacgaggaga aaagctttaa aaaatccaag tgaagaatat 540
   gggaaaattt tggaagttgt tggcaggtat tcagtacaca atgcaggcat tagtttctca 600
   gttaaaaaac aaggagagac agtagctgat gttaggacac tacccaatgc ctcaaccgtg 660
   gacaatattc gctccatctt tggaaatgct gttagtcgag aactgataga aattggatgt 720
   gaggataaaa ccctagcctt caaaatgaat ggttacatat ccaatgcaaa ctactcagtg 780
   aagaagtgca tcttcttact cttcatcaac catcgtctgg tagaatcaac ttccttgaga 840
   aaagccatag aaacagtgta tgcagcctat ttgcccaaaa acacacaccc attcctgtac 900
   ctcagtttag aaatcagtcc ccagaatgtg gatgttaatg tgcaccccac aaagcatgaa 960
   gttcacttcc tgcacgagga gagcatcctg gagcgggtgc agcagcacat cgagagcaag 1020
   ctcctgggct ccaattcctc caggatgtac ttcacccaga ctttgctacc aggacttgct 1080
   ggcccctctg gggagatggt taaatccaca acaagtctga cctcgtcttc tacttctgga 1140
   agtagtgata aggtctatgc ccaccagatg gttcgtacag attcccggga acagaagctt 1200
   gatgcatttc tgcagcctct gagcaaaccc ctgtccagtc agccccaggc cattgtcaca 1260
   gaggataaga cagatatttc tagtggcagg gctaggcagc aagatgagga gatgcttgaa 1320
   ctcccagccc ctgctgaagt ggctgccaaa aatcagagct tggaggggga tacaacaaag 1380
   gggacttcag aaatgtcaga gaagagagga cctacttcca gcaaccccag aaagagacat 1440
   cgggaagatt ctgatgtgga aatggtggaa gatgattccc gaaaggaaat gactgcagct 1500
   tgtacccccc ggagaaggat cattaacctc actagtgttt tgagtctcca ggaagaaatt 1560
   aatgagcagg gacatgaggt tctccgggag atgttgcata accactcctt cgtgggctgt 1620
   gtgaatcctc agtgggcctt ggcacagcat caaaccaagt tataccttct caacaccacc 1680
   aagcttagtg aagaactgtt ctaccagata ctcatttatg attttgccaa ttttggtgtt 1740
   ctcaggttat cggagccagc accgctcttt gaccttgcca tgcttgcctt agatagtcca 1800
   gagagtggct ggacagagga agatggtccc aaagaaggac ttgctgaata cattgttgag 1860
   tttctgaaga agaaggctga gatgcttgca gactatttct ctttggaaat tgatgaggaa 1920
   gggaacctga ttggattacc ccttctgatt gacaactatg tgcccccttt ggagggactg 1980
   cctatcttca ttcttcgact agccactgag gtgaattggg acgaagaaaa ggaatgtttt 2040
   gaaagcctca gtaaagaatg cgctatgttc tattccatcc ggaagcagta catatctgag 2100
   gagtcgaccc tctcaggcca gcagagtgaa gtgcctggct ccattccaaa ctcctggaag 2160
   tggactgtgg aacacattgt ctataaagcc ttgcgctcac acattctgcc tcctaaacat 2220
   ttcacagaag atggaaatat cctgcagctt gctaacctgc ctgatctata caaagtcttt 2280
   gagaggtgtt aaatatggtt atttatgcac tgtgggatgt gttcttcttt ctctgtattc 2340
   cgatacaaag tgttgtatca aagtgtgata tacaaagtgt accaacataa gtgttggtag 2400
   cacttaagac ttatacttgc cttctgatag tattccttta tacacagtgg attgattata 2460
   aataaataga tgtgtcttaa cata                                        2484
<210> 24
   <211> 133
   <212> PRT
   <213> Homo sapiens
<400> 24
   Met Lys Gln Leu Pro Ala Ala Thr Val Arg Leu Leu Ser Ser Ser Gln
   1               5                   10                  15
   Ile Ile Thr Ser Val Val Ser Val Val Lys Glu Leu Ile Glu Asn Ser
               20                  25                  30
   Leu Asp Ala Gly Ala Thr Ser Val Asp Val Lys Leu Glu Asn Tyr Gly
           35                  40                  45
   Phe Asp Lys Ile Glu Val Arg Asp Asn Gly Glu Gly Ile Lys Ala Val
   50 55 60
   Asp Ala Pro Val Met Ala Met Lys Tyr Tyr Thr Ser Lys Ile Asn Ser
   65 70 75 80
   His Glu Asp Leu Glu Asn Leu Thr Thr Tyr Gly Phe Arg Gly Glu Ala
   85 90 95
   Leu Gly Ser Ile Cys Cys Ile Ala Glu Val Leu Ile Thr Thr Arg Thr
   100 105 110
   Ala Ala Asp Asn Phe Ser Thr Gln Tyr Val Leu Asp Gly Ser Gly His
   115 120 125
   Ile Leu Ser Gln Lys
   130
<210> 25
   <211> 426
   <212> DNA
   <213> Homo sapiens
<400> 25
   cgaggcggat cgggtgttgc atccatggag cgagctgaga gctcgagtac agaacctgct 60
   aaggccatca aacctattga tcggaagtca gtccatcaga tttgctctgg gcaggtggta 120
   ctgagtctaa gcactgcggt aaaggagtta gtagaaaaca gtctggatgc tggtgccact 180
   aatattgatc taaagcttaa ggactatgga gtggatctta ttgaagtttc agacaatgga 240
   tgtggggtag aagaagaaaa cttcgaaggc ttaactctga aacatcacac atctaagatt 300
   caagagtttg ccgacctaac tcaggttgaa acttttggct ttcgggggga agctctgagc 360
   tcactttgtg cactgagcga tgtcaccatt tctacctgcc acgcatcggc gaaggttgga 420
   acttga                                                            426
<210> 26
   <211> 1360
   <212> PRT
   <213> Homo sapiens
<400> 26
<210> 27
   <211> 4244
   <212> DNA
   <213> Homo sapiens
<400> 27
   gccgcgcggt agatgcggtg cttttaggag ctccgtccga cagaacggtt gggccttgcc 60
   ggctgtcggt atgtcgcgac agagcaccct gtacagcttc ttccccaagt ctccggcgct 120
   gagtgatgcc aacaaggcct cggccagggc ctcacgcgaa ggcggccgtg ccgccgctgc 180
   ccccggggcc tctccttccc caggcgggga tgcggcctgg agcgaggctg ggcctgggcc 240
   caggcccttg gcgcgctccg cgtcaccgcc caaggcgaag aacctcaacg gagggctgcg 300
   gagatcggta gcgcctgctg cccccaccag ttgtgacttc tcaccaggag atttggtttg 360
   ggccaagatg gagggttacc cctggtggcc ttgtctggtt tacaaccacc cctttgatgg 420
   aacattcatc cgcgagaaag ggaaatcagt ccgtgttcat gtacagtttt ttgatgacag 480
   cccaacaagg ggctgggtta gcaaaaggct tttaaagcca tatacaggtt caaaatcaaa 540
   ggaagcccag aagggaggtc atttttacag tgcaaagcct gaaatactga gagcaatgca 600
   acgtgcagat gaagccttaa ataaagacaa gattaagagg cttgaattgg cagtttgtga 660
   tgagccctca gagccagaag aggaagaaga gatggaggta ggcacaactt acgtaacaga 720
   taagagtgaa gaagataatg aaattgagag tgaagaggaa gtacagccta agacacaagg 780
   atctaggcga agtagccgcc aaataaaaaa acgaagggtc atatcagatt ctgagagtga 840
   cattggtggc tctgatgtgg aatttaagcc agacactaag gaggaaggaa gcagtgatga 900
   aataagcagt ggagtggggg atagtgagag tgaaggcctg aacagccctg tcaaagttgc 960
   tcgaaagcgg aagagaatgg tgactggaaa tggctctctt aaaaggaaaa gctctaggaa 1020
   ggaaacgccc tcagccacca aacaagcaac tagcatttca tcagaaacca agaatacttt 1080
   gagagctttc tctgcccctc aaaattctga atcccaagcc cacgttagtg gaggtggtga 1140
   tgacagtagt cgccctactg tttggtatca tgaaacttta gaatggctta aggaggaaaa 1200
   gagaagagat gagcacagga ggaggcctga tcaccccgat tttgatgcat ctacactcta 1260
   tgtgcctgag gatttcctca attcttgtac tcctgggatg aggaagtggt ggcagattaa 1320
   gtctcagaac tttgatcttg tcatctgtta caaggtgggg aaattttatg agctgtacca 1380
   catggatgct cttattggag tcagtgaact ggggctggta ttcatgaaag gcaactgggc 1440
   ccattctggc tttcctgaaa ttgcatttgg ccgttattca gattccctgg tgcagaaggg 1500
   ctataaagta gcacgagtgg aacagactga gactccagaa atgatggagg cacgatgtag 1560
   aaagatggca catatatcca agtatgatag agtggtgagg agggagatct gtaggatcat 1620
   taccaagggt acacagactt acagtgtgct ggaaggtgat ccctctgaga actacagtaa 1680
   gtatcttctt agcctcaaag aaaaagagga agattcttct ggccatactc gtgcatatgg 1740
   tgtgtgcttt gttgatactt cactgggaaa gtttttcata ggtcagtttt cagatgatcg 1800
   ccattgttcg agatttagga ctctagtggc acactatccc ccagtacaag ttttatttga 1860
   aaaaggaaat ctctcaaagg aaactaaaac aattctaaag agttcattgt cctgttctct 1920
   tcaggaaggt ctgatacccg gctcccagtt ttgggatgca tccaaaactt tgagaactct 1980
   ccttgaggaa gaatatttta gggaaaagct aagtgatggc attggggtga tgttacccca 2040
   ggtgcttaaa ggtatgactt cagagtctga ttccattggg ttgacaccag gagagaaaag 2100
   tgaattggcc ctctctgctc taggtggttg tgtcttctac ctcaaaaaat gccttattga 2160
   tcaggagctt ttatcaatgg ctaattttga agaatatatt cccttggatt ctgacacagt 2220
   cagcactaca agatctggtg ctatcttcac caaagcctat caacgaatgg tgctagatgc 2280
   agtgacatta aacaacttgg agatttttct gaatggaaca aatggttcta ctgaaggaac 2340
   cctactagag agggttgata cttgccatac tccttttggt aagcggctcc taaagcaatg 2400
   gctttgtgcc ccactctgta accattatgc tattaatgat cgtctagatg ccatagaaga 2460
   cctcatggtt gtgcctgaca aaatctccga agttgtagag cttctaaaga agcttccaga 2520
   tcttgagagg ctactcagta aaattcataa tgttgggtct cccctgaaga gtcagaacca 2580
   cccagacagc agggctataa tgtatgaaga aactacatac agcaagaaga agattattga 2640
   ttttctttct gctctggaag gattcaaagt aatgtgtaaa attataggga tcatggaaga 2700
   agttgctgat ggttttaagt ctaaaatcct taagcaggtc atctctctgc agacaaaaaa 2760
   tcctgaaggt cgttttcctg atttgactgt agaattgaac cgatgggata cagcctttga 2820
   ccatgaaaag gctcgaaaga ctggacttat tactcccaaa gcaggctttg actctgatta 2880
   tgaccaagct cttgctgaca taagagaaaa tgaacagagc ctcctggaat acctagagaa 2940
   acagcgcaac agaattggct gtaggaccat agtctattgg gggattggta ggaaccgtta 3000
   ccagctggaa attcctgaga atttcaccac tcgcaatttg ccagaagaat acgagttgaa 3060
   atctaccaag aagggctgta aacgatactg gaccaaaact attgaaaaga agttggctaa 3120
   tctcataaat gctgaagaac ggagggatgt atcattgaag gactgcatgc ggcgactgtt 3180
   ctataacttt gataaaaatt acaaggactg gcagtctgct gtagagtgta tcgcagtgtt 3240
   ggatgtttta ctgtgcctgg ctaactatag tcgagggggt gatggtccta tgtgtcgccc 3300
   agtaattctg ttgccggaag ataccccccc cttcttagag cttaaaggat cacgccatcc 3360
   ttgcattacg aagacttttt ttggagatga ttttattcct aatgacattc taataggctg 3420
   tgaggaagag gagcaggaaa atggcaaagc ctattgtgtg cttgttactg gaccaaatat 3480
   ggggggcaag tctacgctta tgagacaggc tggcttatta gctgtaatgg cccagatggg 3540
   ttgttacgtc cctgctgaag tgtgcaggct cacaccaatt gatagagtgt ttactagact 3600
   tggtgcctca gacagaataa tgtcaggtga aagtacattt tttgttgaat taagtgaaac 3660
   tgccagcata ctcatgcatg caacagcaca ttctctggtg cttgtggatg aattaggaag 3720
   aggtactgca acatttgatg ggacggcaat agcaaatgca gttgttaaag aacttgctga 3780
   gactataaaa tgtcgtacat tattttcaac tcactaccat tcattagtag aagattattc 3840
   tcaaaatgtt gctgtgcgcc taggacatat ggcatgcatg gtagaaaatg aatgtgaaga 3900
   ccccagccag gagactatta cgttcctcta taaattcatt aagggagctt gtcctaaaag 3960
   ctatggcttt aatgcagcaa ggcttgctaa tctcccagag gaagttattc aaaagggaca 4020
   tagaaaagca agagaatttg agaagatgaa tcagtcacta cgattatttc gggaagtttg 4080
   cctggctagt gaaaggtcaa ctgtagatgc tgaagctgtc cataaattgc tgactttgat 4140
   taaggaatta tagactgact acattggaag ctttgagttg acttctgaca aaggtggtaa 4200
   attcagacaa cattatgatc taataaactt tattttttaa aaat                4244
<210> 28
   <211> 1128
   <212> PRT
   <213> Homo sapiens
<400> 28
   Met Ser Arg Arg Lys Pro Ala Ser Gly Gly Leu Ala Ala Ser Ser Ser
    1               5                  10                  15
   Ala Pro Ala Arg Gln Ala Val Leu Ser Arg Phe Phe Gln Ser Thr Gly
               20                  25                  30
   Ser Leu Lys Ser Thr Ser Ser Ser Thr Gly Ala Ala Asp Gln Val Asp
           35                  40                  45
   Pro Gly Ala Ala Ala Ala Ala Ala Pro Pro Ala Pro Ala Phe Pro Pro
       50                  55                  60
   Gln Leu Pro Pro His Val Ala Thr Glu Ile Asp Arg Arg Lys Lys Arg
   65                  70                  75                  80
   Pro Leu Glu Asn Asp Gly Pro Val Lys Lys Lys Val Lys Lys Val Gln
                   85                  90                  95
   Gln Lys Glu Gly Gly Ser Asp Leu Gly Met Ser Gly Asn Ser Glu Pro
               100                 105                 110
   Lys Lys Cys Leu Arg Thr Arg Asn Val Ser Lys Ser Leu Glu Lys Leu
           115                 120                 125
   Lys Glu Phe Cys Cys Asp Ser Ala Leu Pro Gln Ser Arg Val Gln Thr
       130                 135                 140
   Glu Ser Leu Gln Glu Arg Phe Ala Val Leu Pro Lys Cys Thr Asp Phe
   145                 150                 155                 160
   Asp Asp Ile Ser Leu Leu His Ala Lys Asn Ala Val Ser Ser Glu Asp
                   165                 170                 175
   Ser Lys Arg Gln Ile Asn Gln Lys Asp Thr Thr Leu Phe Asp Leu Ser
               180                 185                 190
   Gln Phe Gly Ser Ser Asn Thr Ser His Glu Asn Leu Gln Lys Thr Ala
           195                 200                 205
   Ser Lys Ser Ala Asn Lys Arg Ser Lys Ser Ile Tyr Thr Pro Leu Glu
       210                 215                 220
   Leu Gln Tyr Ile Glu Met Lys Gln Gln His Lys Asp Ala Val Leu Cys
   225                 230                 235                 240
   Val Glu Cys Gly Tyr Lys Tyr Arg Phe Phe Gly Glu Asp Ala Glu Ile
                   245                 250                 255
   Ala Ala Arg Glu Leu Asn Ile Tyr Cys His Leu Asp His Asn Phe Met
   260 265 270
   Thr Ala Ser Ile Pro Thr His Arg Leu Phe Val His Val Arg Arg Leu
   275 280 285
   Val Ala Lys Gly Tyr Lys Val Gly Val Val Lys Gln Thr Glu Thr Ala
   290 295 300
   Ala Leu Lys Ala Ile Gly Asp Asn Arg Ser Ser Leu Phe Ser Arg Lys
   305 310 315 320
   Leu Thr Ala Leu Tyr Thr Lys Ser Thr Leu Ile Gly Glu Asp Val Asn
   325 330 335
   Pro Leu Ile Lys Leu Asp Asp Ala Val Asn Val Asp Glu Ile Met Thr
   340 345 350
   Asp Thr Ser Thr Ser Tyr Leu Leu Cys Ile Ser Glu Asn Lys Glu Asn
   355 360 365
   Val Arg Asp Lys Lys Lys Gly Asn Ile Phe Ile Gly Ile Val Gly Val
   370 375 380
   Gln Pro Ala Thr Gly Glu Val Val Phe Asp Ser Phe Gln Asp Ser Ala
   385 390 395 400
   Ser Arg Ser Glu Leu Glu Thr Arg Met Ser Ser Leu Gln Pro Val Glu
   405 410 415
   Leu Leu Leu Pro Ser Ala Leu Ser Glu Gln Thr Glu Ala Leu Ile His
   420 425 430
   Arg Ala Thr Ser Val Ser Val Gln Asp Asp Arg Ile Arg Val Glu Arg
   435 440 445
   Met Asp Asn Ile Tyr Phe Glu Tyr Ser His Ala Phe Gln Ala Val Thr
   450 455 460
   Glu Phe Tyr Ala Lys Asp Thr Val Asp Ile Lys Gly Ser Gln Ile Ile
   465 470 475 480
   Ser Gly Ile Val Asn Leu Glu Lys Pro Val Ile Cys Ser Leu Ala Ala
   485 490 495
   Ile Ile Lys Tyr Leu Lys Glu Phe Asn Leu Glu Lys Met Leu Ser Lys
   500 505 510
   Pro Glu Asn Phe Lys Gln Leu Ser Ser Lys Met Glu Phe Met Thr Ile
   515 520 525
   Asn Gly Thr Thr Leu Arg Asn Leu Glu Ile Leu Gln Asn Gln Thr Asp
   530 535 540
   Met Lys Thr Lys Gly Ser Leu Leu Trp Val Leu Asp His Thr Lys Thr
   545 550 555 560
   Ser Phe Gly Arg Arg Lys Leu Lys Lys Trp Val Thr Gln Pro Leu Leu
   565 570 575
   Lys Leu Arg Glu Ile Asn Ala Arg Leu Asp Ala Val Ser Glu Val Leu
   580 585 590
   His Ser Glu Ser Ser Val Phe Gly Gln Ile Glu Asn His Leu Arg Lys
   595 600 605
   Leu Pro Asp Ile Glu Arg Gly Leu Cys Ser Ile Tyr His Lys Lys Cys
   610 615 620
   Ser Thr Gln Glu Phe Phe Leu Ile Val Lys Thr Leu Tyr His Leu Lys
   625 630 635 640
   Ser Glu Phe Gln Ala Ile Ile Pro Ala Val Asn Ser His Ile Gln Ser
   645 650 655
   Asp Leu Leu Arg Thr Val Ile Leu Glu Ile Pro Glu Leu Leu Ser Pro
   660 665 670
   Val Glu His Tyr Leu Lys Ile Leu Asn Glu Gln Ala Ala Lys Val Gly
   675 680 685
   Asp Lys Thr Glu Leu Phe Lys Asp Leu Ser Asp Phe Pro Leu Ile Lys
   690 695 700
   Lys Arg Lys Asp Glu Ile Gln Gly Val Ile Asp Glu Ile Arg Met His
   705 710 715 720
   Leu Gln Glu Ile Arg Lys Ile Leu Lys Asn Pro Ser Ala Gln Tyr Val
   725 730 735
   Thr Val Ser Gly Gln Glu Phe Met Ile Glu Ile Lys Asn Ser Ala Val
   740 745 750
   Ser Cys Ile Pro Thr Asp Trp Val Lys Val Gly Ser Thr Lys Ala Val
   755 760 765
   Ser Arg Phe His Ser Pro Phe Ile Val Glu Asn Tyr Arg His Leu Asn
       770                 775                 780
   Gln Leu Arg Glu Gln Leu Val Leu Asp Cys Ser Ala Glu Trp Leu Asp
   785                 790                 795                 800
   Phe Leu Glu Lys Phe Ser Glu His Tyr His Ser Leu Cys Lys Ala Val
                   805                 810                 815
   His His Leu Ala Thr Val Asp Cys Ile Phe Ser Leu Ala Lys Val Ala
               820                 825                 830
   Lys Gln Gly Asp Tyr Cys Arg Pro Thr Val Gln Glu Glu Arg Lys Ile
           835                 840                 845
   Val Ile Lys Asn Gly Arg His Pro Val Ile Asp Val Leu Leu Gly Glu
       850                 855                 860
   Gln Asp Gln Tyr Val Pro Asn Asn Thr Asp Leu Ser Glu Asp Ser Glu
   865                 870                 875                 880
   Arg Val Met Ile Ile Thr Gly Pro Asn Met Gly Gly Lys Ser Ser Tyr
                   885                 890                 895
   Ile Lys Gln Val Ala Leu Ile Thr Ile Met Ala Gln Ile Gly Ser Tyr
               900                 905                 910
   Val Pro Ala Glu Glu Ala Thr Ile Gly Ile Val Asp Gly Ile Phe Thr
           915                 920                 925
   Arg Met Gly Ala Ala Asp Asn Ile Tyr Lys Gly Arg Ser Thr Phe Met
       930                 935                 940
   Glu Glu Leu Thr Asp Thr Ala Glu Ile Ile Arg Lys Ala Thr Ser Gln
   945                 950                 955                 960
   Ser Leu Val Ile Leu Asp Glu Leu Gly Arg Gly Thr Ser Thr His Asp
                   965                 970                 975
   Gly Ile Ala Ile Ala Tyr Ala Thr Leu Glu Tyr Phe Ile Arg Asp Val
               980                 985                 990
   Lys Ser Leu Thr Leu Phe Val Thr His Tyr Pro Pro Val Cys Glu Leu
           995                 1000                1005
   Glu Lys Asn Tyr Ser His Gln Val Gly Asn Tyr His Met Gly Phe Leu
       1010                1015                1020
   Val Ser Glu Asp Glu Ser Lys Leu Asp Pro Gly Ala Ala Glu Gln Val
   1025 1030 1035 1040
   Pro Asp Phe Val Thr Phe Leu Tyr Gln Ile Thr Arg Gly Ile Ala Ala
   1045 1050 1055
   Arg Ser Tyr Gly Leu Asn Val Ala Lys Leu Ala Asp Val Pro Gly Glu
   1060 1065 1070
   Ile Leu Lys Lys Ala Ala His Lys Ser Lys Glu Leu Glu Gly Leu Ile
   1075 1080 1085
   Asn Thr Lys Arg Lys Arg Leu Lys Tyr Phe Ala Lys Leu Trp Thr Met
   1090 1095 1100
   His Asn Ala Gln Asp Leu Gln Lys Trp Thr Glu Glu Phe Asn Met Glu
   1105 1110 1115 1120
   Glu Thr Gln Thr Ser Leu Leu His
   1125
<210> 29
   <211> 4374
   <212> DNA
   <213> Homo sapiens
<400> 29
   gggcacgagc cctgccatgt ctcgccggaa gcctgcgtcg ggcggcctcg ctgcctccag 60
   ctcagcccct gcgaggcaag cggttttgag ccgattcttc cagtctacgg gaagcctgaa 120
   atccacctcc tcctccacag gtgcagccga ccaggtggac cctggcgctg cagcggccgc 180
   agcgccccca gcgcccgcct tcccgcccca gctgccgccg cacgtagcta cagaaattga 240
   cagaagaaag aagagaccat tggaaaatga tgggcctgtt aaaaagaaag taaagaaagt 300
   ccaacaaaag gaaggaggaa gtgatctggg aatgtctggc aactctgagc caaagaaatg 360
   tctgaggacc aggaatgttt caaagtctct ggaaaaattg aaagaattct gctgcgattc 420
   tgcccttcct caaagtagag tccagacaga atctctgcag gagagatttg cagttctgcc 480
   aaaatgtact gattttgatg atatcagtct tctacacgca aagaatgcag tttcttctga 540
   agattcgaaa cgtcaaatta atcaaaagga cacaacactt tttgatctca gtcagtttgg 600
   atcatcaaat acaagtcatg aaaatttaca gaaaactgct tccaaatcag ctaacaaacg 660
   gtccaaaagc atctatacgc cgctagaatt acaatacata gaaatgaagc agcagcacaa 720
   agatgcagtt ttgtgtgtgg aatgtggata taagtataga ttctttgggg aagatgcaga 780
   gattgcagcc cgagagctca atatttattg ccatttagat cacaacttta tgacagcaag 840
   tatacctact cacagactgt ttgttcatgt acgccgcctg gtggcaaaag gatataaggt 900
   gggagttgtg aagcaaactg aaactgcagc attaaaggcc attggagaca acagaagttc 960
   actcttttcc cggaaattga ctgcccttta tacaaaatct acacttattg gagaagatgt 1020
   gaatccccta atcaagctgg atgatgctgt aaatgttgat gagataatga ctgatacttc 1080
   taccagctat cttctgtgca tctctgaaaa taaggaaaat gttagggaca aaaaaaaggg 1140
   caacattttt attggcattg tgggagtgca gcctgccaca ggcgaggttg tgtttgatag 1200
   tttccaggac tctgcttctc gttcagagct agaaacccgg atgtcaagcc tgcagccagt 1260
   agagctgctg cttccttcgg ccttgtccga gcaaacagag gcgctcatcc acagagccac 1320
   atctgttagt gtgcaggatg acagaattcg agtcgaaagg atggataaca tttattttga 1380
   atacagccat gctttccagg cagttacaga gttttatgca aaagatacag ttgacatcaa 1440
   aggttctcaa attatttctg gcattgttaa cttagagaag cctgtgattt gctctttggc 1500
   tgccatcata aaatacctca aagaattcaa cttggaaaag atgctctcca aacctgagaa 1560
   ttttaaacag ctatcaagta aaatggaatt tatgacaatt aatggaacaa cattaaggaa 1620
   tctggaaatc ctacagaatc agactgatat gaaaaccaaa ggaagtttgc tgtgggtttt 1680
   agaccacact aaaacttcat ttgggagacg gaagttaaag aagtgggtga cccagccact 1740
   ccttaaatta agggaaataa atgcccggct tgatgctgta tcggaagttc tccattcaga 1800
   atctagtgtg tttggtcaga tagaaaatca tctacgtaaa ttgcccgaca tagagagggg 1860
   actctgtagc atttatcaca aaaaatgttc tacccaagag ttcttcttga ttgtcaaaac 1920
   tttatatcac ctaaagtcag aatttcaagc aataatacct gctgttaatt cccacattca 1980
   gtcagacttg ctccggaccg ttattttaga aattcctgaa ctcctcagtc cagtggagca 2040
   ttacttaaag atactcaatg aacaagctgc caaagttggg gataaaactg aattatttaa 2100
   agacctttct gacttccctt taataaaaaa gaggaaggat gaaattcaag gtgttattga 2160
   cgagatccga atgcatttgc aagaaatacg aaaaatacta aaaaatcctt ctgcacaata 2220
   tgtgacagta tcaggacagg agtttatgat agaaataaag aactctgctg tatcttgtat 2280
   accaactgat tgggtaaagg ttggaagcac aaaagctgtg agccgctttc actctccttt 2340
   tattgtagaa aattacagac atctgaatca gctccgggag cagctagtcc ttgactgcag 2400
   tgctgaatgg cttgattttc tagagaaatt cagtgaacat tatcactcct tgtgtaaagc 2460
   agtgcatcac ctagcaactg ttgactgcat tttctccctg gccaaggtcg ctaagcaagg 2520
   agattactgc agaccaactg tacaagaaga aagaaaaatt gtaataaaaa atggaaggca 2580
   ccctgtgatt gatgtgttgc tgggagaaca ggatcaatat gtcccaaata atacagattt 2640
   atcagaggac tcagagagag taatgataat taccggacca aacatgggtg gaaagagctc 2700
   ctacataaaa caagttgcat tgattaccat catggctcag attggctcct atgttcctgc 2760
   agaagaagcg acaattggga ttgtggatgg cattttcaca aggatgggtg ctgcagacaa 2820
   tatatataaa ggacggagta catttatgga agaactgact gacacagcag aaataatcag 2880
   aaaagcaaca tcacagtcct tggttatctt ggatgaacta ggaagaggga cgagcactca 2940
   tgatggaatt gccattgcct atgctacact tgagtatttc atcagagatg tgaaatcctt 3000
   aaccctgttt gtcacccatt atccgccagt ttgtgaacta gaaaaaaatt actcacacca 3060
   ggtggggaat taccacatgg gattcttggt cagtgaggat gaaagcaaac tggatccagg 3120
   cgcagcagaa caagtccctg attttgtcac cttcctttac caaataacta gaggaattgc 3180
   agcaaggagt tatggattaa atgtggctaa actagcagat gttcctggag aaattttgaa 3240
   gaaagcagct cacaagtcaa aagagctgga aggattaata aatacgaaaa gaaagagact 3300
   caagtatttt gcaaagttat ggacgatgca taatgcacaa gacctgcaga agtggacaga 3360
   ggagttcaac atggaagaaa cacagacttc tcttcttcat taaaatgaag actacatttg 3420
   tgaacaaaaa atggagaatt aaaaatacca actgtacaaa ataactctcc agtaacagcc 3480
   tatctttgtg tgacatgtga gcataaaatt atgaccatgg tatattccta ttggaaacag 3540
   agaggttttt ctgaagacag tctttttcaa gtttctgtct tcctaacttt tctacgtata 3600
   aacactcttg aatagacttc cactttgtaa ttagaaaatt ttatggacag taagtccagt 3660
   aaagccttaa gtggcagaat ataattccca agcttttgga gggtgatata aaaatttact 3720
   tgatattttt atttgtttca gttcagataa ttggcaactg ggtgaatctg gcaggaatct 3780
   atccattgaa ctaaaataat tttattatgc aaccagttta tccaccaaga acataagaat 3840
   tttttataag tagaaagaat tggccaggca tggtggctca tgcctgtaat cccagcactt 3900
   tgggaggcca aggtaggcag atcacctgag gtcaggagtt caagaccagc ctggccaaca 3960
   tggcaaaacc ccatctttac taaaaatata aagtacatct ctactaaaaa tacgaaaaaa 4020
   ttagctgggc atggtggcgc acacctgtag tcccagctac tccggaggct gaggcaggag 4080
   aatctcttga acctgggagg cggaggttgc aatgagccga gatcacgtca ctgcactcca 4140
   gcttgggcaa cagagcaaga ctccatctca aaaaagaaaa aagaaaagaa atagaattat 4200
   caagctttta aaaactagag cacagaagga ataaggtcat gaaatttaaa aggttaaata 4260
   ttgtcatagg attaagcagt ttaaagattg ttggatgaaa ttatttgtca ttcattcaag 4320
   taataaatat ttaatgaata cttgctataa aaaaaaaaaa aaaaaaaaaa aaaa       4374
<210> 30
   <211> 20
   <212> DNA
   <213> Artificial Sequence
<220>
   <223> Description of Artificial Sequence:oligonucleotide primer
<400> 30
   gatatctcca ctgacgtaag 20
<210> 31
   <211> 19
   <212> DNA
   <213> Artificial Sequence
<220>
   <223> Description of Artificial Sequence: oligonucleotide primer
<400> 31
   tgttgccggt cttgcgatg 19
<210> 32
   <211> 21
   <212> DNA
   <213> Artificial Sequence
<220>
   <223> Description of Artificial Sequence: oligonucleotide primer
<400> 32
   cccgatctag taacatagat g 21
<210> 33
   <211> 21
   <212> DNA
   <213> Artificial Sequence
<220>
   <223> Description of Artificial Sequence:oligonucleotide primer
<400> 33
   cagtctggat cgcgaaaact g 21
<210> 34
   <211> 20
   <212> DNA
   <213> Artificial Sequence
<220>
   <223> Description of Artificial Sequence:oligonucleotide primer
   <400> 34
   ggtgattacc gacgaaaacg 20
<210> 35
   <211> 20
   <212> DNA
   <213> Artificial Sequence
<220>
   <223> Description of Artificial Sequence:oligonucleotide primer
<400> 35
   agtgaagggc gaacagttcc 20
<210> 36
   <211> 18
   <212> DNA
   <213> Artificial Sequence
<220>
   <223> Description of Artificial Sequence:oligonucleotide primer
<400> 36
   gagtattgcc aacgaacc 18
<210> 37
   <211> 20
   <212> DNA
   <213> Artificial Sequence
<220>
   <223> Description of Artificial Sequence:oligonucleotide primer
<400> 37
   gtatcaccgc gtctttgatc 20
<210> 38
   <211> 19
   <212> DNA
   <213> Artificial Sequence
<220> ..
   <223> Description of Artificial Sequence:oligonucleotide primer
<400> 38
   cgaaacgcag cacgatacg 19
<210> 39
   <211> 19
   <212> DNA
   <213> Artificial Sequence
<220>
   <223> Description of Artificial Sequence:oligonucleotide primer
<400> 39
   gttcaacgct gacatcacc 19
<210> 40
   <211> 20
   <212> DNA
   <213> Artificial Sequence
<220>
   <223> Description of Artificial Sequence: oligonucleotide primer
<400> 40
   catgttcatc tgcccagtcg 20
<210> 41
   <211> 18
   <212> DNA
   <213> Artificial Sequence
<220> <223> Description of Artificial Sequence:oligonucleotide primer
<400> 41
   gctttggaca taccatcc 18
<210> 42
   <211> 18
   <212> DNA
   <213> Artificial Sequence
<220>
   <223> Description of Artificial Sequence:oligonucleotide primer
<400> 42
   caccgaagtt catgccag 18
<210> 43
   <211> 21
   <212> DNA
   <213> Artificial Sequence
<220>
   <223> Description of Artificial Sequence:oligonucleotide primer
<400> 43
   tgactacttt tgacttcagc c 21
<210> 44
   <211> 22
   <212> DNA
   <213> Artificial Sequence
<220>
   <223> Description of Artificial Sequence:oligonucleotide primer
<400> 44
   aaccattcaa catttttaac cc 22